(19) Europäisches Patentamt
European Patent Office
Office européen des brevets

(11) EP 3 300 715 A1

(12) **EUROPÄISCHE PATENTANMELDUNG**

(43) Veröffentlichungstag:
**04.04.2018 Patentblatt 2018/14**

(51) Int Cl.:
***A61K 8/37*** *(2006.01)* ***C07C 69/96*** *(2006.01)*

(21) Anmeldenummer: **16191899.0**

(22) Anmeldetag: **30.09.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **BASF SE**
**67056 Ludwigshafen (DE)**

(72) Erfinder:
- **DIERKER, Markus**
  **40589 Düsseldorf-Holthausen (DE)**
- **ALBERS, Thomas**
  **40589 Düsseldorf-Holthausen (DE)**
- **BAEK, Yeah-Young**
  **40789 Monheim (DE)**
- **BEUCHE, Marc**
  **92593 Levallois-Perret Cedex (FR)**
- **GUELDNER, Marco**
  **40789 Monheim (DE)**
- **ISSBERNER, Ulrich**
  **40789 Monheim (DE)**
- **JUNG, Robert**
  **40589 Düsseldorf-Holthausen (DE)**
- **KAVARNOU-SEILER, Asimina**
  **40789 Monheim (DE)**
- **LACOUTIÈRE, Stéphane**
  **91190 Gif Sur Yvette (FR)**
- **MARQUIS, Cecile**
  **68331 Huningue Cedex (FR)**
- **MEHLING, Annette**
  **40589 Düsseldorf-Holthausen (DE)**
- **PRINZ, Daniela**
  **40589 Düsseldorf-Holthausen (DE)**
- **RIEDEL, Heidi**
  **40589 Düsseldorf-Holthausen (DE)**

(74) Vertreter: **BASF IP Association**
**BASF SE**
**G-FLP-C006**
**67056 Ludwigshafen (DE)**

# (54) VERWENDUNG VON DIALKYLCARBONATEN VON VERZWEIGTEN ALKOHOLEN ALS DISPERGIERMITTEL

(57) Die Erfindung betrifft die Verwendung von Dialkylcarbonaten von verzweigten Alkoholen als Dispergiermittel. Diese Dialkylcarbonate sind bevorzugt in kosmetischen und/oder pharmazeutischen Zubereitungen zu verwenden.

EP 3 300 715 A1

Printed by Jouve, 75001 PARIS (FR)

## Beschreibung

Gebiet der Erfindung

Die Erfindung betrifft die Verwendung von Dialkylcarbonaten von verzweigten Alkoholen als Dispergiermittel, beispielsweise in kosmetischen und/oder pharmazeutischen Zubereitungen.

Stand der Technik

Im Bereich kosmetischer Zubereitungen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser, sowie unlösliche Substanzen, wie z.B. Pigmente. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit hinsichtlich glatter Texturen zu erfüllen, werden kontinuierlich neue Dispergiermittel entwickelt und getestet.

Dialkylcarbonate von linearen Alkoholen und ihre kosmetische Verwendung sind aus dem Stand der Technik bekannt, so beschreibt WO 97/47282 die kosmetische Verwendung von Octyl-methyl-carbonat, EP 1 510 199 die kosmetische Verwendung von Distearylcarbonat. Din-octylcarbonat ist als kosmetischer Rohstoff unter dem Handelsnamen Cetiol® CC (Cognis Deutschland GmbH & Co. KG) erhältlich.

Die Herstellung von Dialkylcarbonaten ist in WO 97/47583 beschrieben. Die nach dem dort beschriebenen Verfahren erhältlichen symmetrischen Dialkylcarbonate enthalten als Nebenprodukte z.B. 2-Ethyl-1-hexyl-methylcarbonat oder 2-Butyl-1-Octyl-methylcarbonat. Eine kosmetische Verwendung dieser Verbindungen wird nicht erwähnt. EP 1 083 247 beschreibt verschiedene kurzkettige verzweigte Dialkylcarbonate und ihre Eignung zum Waschen von Metalloberflächen.

WO 2008/067946 beschreibt die Verwendung von verzweigten Dialkylcarbonaten in der Kosmetik.

Aufgabe der vorliegenden Erfindung war es, Dispergiermittel beispielsweise für kosmetische Applikationen zur Verfügung zu stellen, die eine ausgezeichnete Verteilung von Feststoffen in kosmetischen und Zusammensetzungen bewirken, wobei die sensorischen Eigenschaften (Leichtigkeit, "nicht-fettendes Hautgefühl", Weichheit, Spreitvermögen, Absorption, Verteilbarkeit, Öligkeit) der Zusammensetzung erhalten bleiben. Die Dispergiermittel sollten sowohl in W/O als auch in O/W Formulierungen einarbeitbar sein und insbesondere mit kristallinen UV-Filtern, Pigmenten, Antiperspirantien Salzen sowie Silikonen kompatibel sein.

Überraschenderweise wurde gefunden, dass die Dialkylcarbonate der vorliegenden Erfindung diese Aufgabe erfüllen und insbesondere zu glatten und homogenen Produkten führen.

Beschreibung der Erfindung

Dialkylcarbonate sind Ester der Kohlensäure der allgemeinen Formel $O=C(OR)_2$, wobei R einen Alkyl-Rest bedeutet. Die Bezeichnung der Dialkylcarbonate erfolgt entweder, indem die Alkylsubstituenten dem Begriff "Carbonat" voran stellt oder als Kohlensäure"alkyl"ester. So z.B. $O=C(OC_8H_{17})_2$ als Dioctylcarbonat oder Kohlensäuredioctylester oder z.B. $O=C(OC_2H_5)(OC_8H_{17})$ als Ethyl-octyl-carbonate oder als Kohlensäure-Ethyl-Octylester. Im Folgenden wird für die Dialkylcarbonate die Formel R-O-CO-O-R verwendet.

Gegenstand der Erfindung sind Dialkylcarbonate der Formel (I)

$$R'O\text{-}CO\text{-}OR^2 \quad (I),$$

wobei $R^1$ ein 2-Propyl-1-heptyl-Rest oder ein 2-Ethyl-1-Butyl Rest ist, und $R^2$ für lineare oder verzweigte oder cyclische Kohlenwasserstoffreste mit 1 bis 22 Kohlenstoffatomen und 0 oder 1 bis 3 Doppelbindungen steht.

In einer bevorzugten Ausführungsform der Erfindung ist $R^1$ identisch zu $R^2$: Diese Ausführungsform der Erfindung betrifft somit Di-(2- Propyl-1-heptyl)carbonat bzw. Di-(2-Ethyl-1-butyl)carbonat. Di-(2- Propyl-1-heptyl)carbonat ist insbesondere bevorzugt.

In einer besonders bevorzugten Ausführungsform der Erfindung werden Dialkylcarbonate der Formel (I) verwendet, welche eine Gesamtkohlenstoffzahl von größer 12 enthalten.

$R^2$ kann ein linearer oder verzweigter oder cyclischer Kohlenwasserstoffrest mit 1 bis 22 Kohlenstoffatomen und 0 oder 1 bis 3 Doppelbindungen sein.

Beispielsweise seien als lineare Alkylreste genannt: Methyl-, Ethyl-, Butyl-, Pentyl-, Hexyl-, Heptyl-, Octyl-, Nonyl-, Decyl-, Undecyl-, Undecenyl-, Dodecyl- , Tridecyl-, Tetradecyl-, Pentadecyl-, Hexadecyl-, Heptadecyl-, Octadecyl-, Octadecenyl-, Nonadecyl-, Eicosanyl (=C20)-, Docosanyl (= C22)- Reste genannt.

Beispielsweise seien als verzweigte Alkylreste genannt: 2-Methylpropyl-, iso-Butyl-, iso-Pentyl, wie z.B. 2,2,-Dimethylpropyl (=Neopentyl), 3-Methylbutyl- (=iso-Pentyl-), iso-Hexyl-, i-Octyl, wie z.B. 2-Ethyl-hexyl- oder 3-Ethyl-hexyl oder 4-Ethylhexyl- oder 5-Ethylhexyl Reste, i-Decyl-Reste wie z.B. Trimethylheptyl-Rest (= Neodecyl-Rest), Isostearyl-, Isooctyl-, Isononyl-, Isodecyl-, Isotridecyl-, 2-Ethylbutyl-, 2-Ethylhexyl-, 2-Propylheptyl, 2-Butyloctyl, 2-Butyldecyl-, 2-Hexyloctyl-, 2-Hexyldecyl-, 2-Hexyldodecyl-, 2-Octyldecyl-Rest.

Als cyclische Alkyreste seinen genannt der Borneyl- und Isoborneyl-Rest sowie Cyclohexyl-Rest.

Beispiele für ungesättigte Reste sind Oleyl, Linoleyl, Vinyl und Propargyl.

Die Dialkylcarbonate als Dispergiermittel sind in der Lage, Feststoffe insbesondere in kosmetischen oder pharmazeutischen Zusammensetzungen zu dispergieren, um eine homogene Verteilung dieser Feststoffe zu bewirken. Es bilden sich glatte Texturen, die sich ohne weiteres auf die Haut bzw. Haare auftragen lassen. Vorzugsweise sind diese Feststoffe Pigmente, wie organische und/oder anorganische Pigmente. Anorganische LichtfilterPigmente, z.B. Titandioxid und/oder mindestens ein Eisenoxidpigment und/oder Zinkoxidpigment, sind insbesondere bevorzugt. Weitere bevorzugte Pigmente sind Puderrohstoffe, Füllstoffe und Feststoffe zur Modifikation der Sensorik.

In einer weiteren bevorzugten Ausführungsform der Erfindung ist das Pigment ein organischer pigmentärer Lichtfilter, vorzugsweise Methylen-bis-benzotriazolyltetramethylbutylphenol.

Überraschenderweise wurde festgestellt, dass sich die Dialkylcarbonate besonders eignen zur Herstellung von kosmetischen Zubereitungen, d. h., sie eignen sich als Dispergiermittel für Feststoffe in kosmetischen Zubereitungen. Die Dialkylcarbonate eignen sich weiterhin zur Herstellung von pharmazeutischen Zubereitungen, wobei die Dialkylcarbonate als technische Hilfsstoffe, wie Dispergiermittel eingesetzt werden. Die Dialkylcarbonate können zur Herstellung von kosmetischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

Die Erfindung betrifft außerdem eine kosmetische und/oder pharmazeutische Zusammensetzung, die 0,1 bis 95 Gew.-% mindestens ein Dialkylcarbonat der allgemeinen Formel (I) als Dispergiermittel enthält.

In einer bevorzugten Ausführungsform enthält die kosmetische und/oder pharmazeutische Zusammensetzung Di-(2- Propyl-1-heptyl)carbonat als Dispergiermittel.

Die kosmetische und/oder pharmazeutische Zusammensetzung enthält in einer weiteren Ausführungsform mindestens ein Pigment. Das mindestens eine Pigment ist aus der Gruppe organisches und anorganisches Pigment gewählt. Anorganische Lichtfilterpigmente sind bevorzugt, wobei Titandioxid und/oder ein oder mehrere Eisenoxid oder Zinkoxid-Pigmente, besonders bevorzugt sind. Weitere bevorzugte Pigmente sind Puderrohstoffe, Füllstoffe und Feststoffe zur Modifikation der Sensorik enthalten.

In einer weiteren bevorzugten Ausführungs ist das Pigment ein organischer pigmentärer Lichtfilter, vorzugsweise Methylen-bis-benzotriazolyltetramethylbutylphenol.

In einer weiteren Ausführungsform der Erfindung enthält die kosmetische und/oder pharmazeutische Zusammensetzung ein oder mehrere Aluminiumsalze, wie beispielsweise Aluminiumchlorhydrat.

Es hat sich überraschend gezeigt, dass die erfindungsgemäß verwendeten Dialkylcarbonate gegenüber anderen Medien hervorragende Dispersionen mit Feststoffen bilden. Hierzu wird auf die Abbildung verwiesen, in der der Graph deutlich zeigt, dass die höchsten Pigmentkonzentrationen in Di-(2- Propyl-1-heptyl)carbonat als Dispergiermittel erhalten werden.

Das Experiment wurde wie folgt durchgeführt. Das Medium wurde tropfenweise zu 10 g Pigment (grau = Eisenoxid, dunkelgrau = Titanoxid), bis sich eine Paste gebildet hatte (kein Medium im Überschuss). Die folgende Rechnung entspricht der maximalen Menge Pigment in prozent, die in das jeweilige Medium gegeben werden kann:

$$\% \text{ Pigment} = m(\text{Pigment})/m(\text{Pigment}) + m(\text{Medium}) \times 100$$

<u>Herstellung</u>

Die Herstellung der Dialkylcarbonate erfolgt nach dem Fachmann bekannten Verfahren beispielsweise durch Umesterung niederer Dialkylcarbonate, wie beispielsweise Dimethylcarbonat, Diethylcarbonat, Dipropylcarbonat oder

Dibutylcarbonat mit den entsprechenden Alkoholen oder Alkoholgemischen in Gegenwart eines Katalysators, wie beispielsweise in WO 97/47583 beschrieben.

Andere Herstellverfahren sind beschrieben in M. Dierker: Lipid Technology, 2004, 16(6), S. 130-134.

Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkoniumtetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt.

Die erfindungsgemäßen Zubereitungen können die Antiperspirantien in Mengen von 1 bis 50, vorzugsweise 5 bis 30 und insbesondere 8 bis 25 Gew.-% - bezogen auf das Gesamtgewicht der kosmetischen und/oder pharmazeutischen Zubereitung - enthalten.

Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Trüsopropylcitrat, Tributylcitrat und insbesondere Triethylcitrat (Hydagen® CAT, BASF Personal Care and Nutrition GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder-phosphate, wie beispielsweise Lanosterin-, Cholesterin-, Campesterin-, Stigmasterin- und Sitosterinsulfat bzw-phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäure-diethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf das Gesamtgewicht der kosmetischen und/oder pharmazeutischen Zubereitung - enthalten. Desodorierende Wirkstoffe wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Daneben kommen Antitranspirantien zum Einsatz. Die in Antitranspirants am häufigsten vorkommenden Hauptkomponenten sind Aluminiumverbindungen (ein Komplex aus Glycin und Aluminium-Zirkonium-Tetrachlorhydrat). Daneben finden sich Stoffe wie Aluminiumchlorid und Propanthelinbromid. Diese Stoffe verringern die Schweißsekretion durch eine temporäre Verengung oder Verstopfung der Ausführungsgänge der Schweißdrüsen. Häufig eingesetzte Antitranspirant-Wirkstoffe sind Aluminiumchlorohydrat (ACH) und der Aluminium-Zirkoniumtetrachloro-Glycin-Komplex (ZAG).

Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Die erfindungsgemäßen Zubereitungen können die bakterizide bzw. bakteriostatische Wirkstoffe in Mengen von 0,01 bis 5 und vorzugsweise 0,1 bis 2 Gew.-% - bezogen auf das Gesamtgewicht der kosmetischen und/oder pharmazeutischen Zubereitung - enthalten.

Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf das Gesamtgewicht der kosmetischen und/oder pharmazeutischen Zubereitung - enthalten.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide neben Vaseline und Paraffinen und Paraffinwachsen verwendet werden, wobei Fettsäurealkanolamide gleichzeitig als Schaumstabilisatoren dienen.

UV-Filter

Durch Verwendung der Dialkylcarbonate werden sensorisch leichte, kosmetische und/oder pharmazeutische Zubereitungen erhalten, dies ist insbesondere dann der Fall, wenn die Dialkylcarbonate zusammen mit UV-Lichtschutzfiltern eingesetzt werden.

Erfindungsgemäß sind als UV-Lichtschutzfilter bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:

- 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
- 3-(4'-Trimethylammonium) benzyliden- bornan-2-on-methylsulfat (Mexoryl SO)
- 3,3'-(1,4-Phenylendimethin)-bis (7,7-dimethyl-2-oxobicyclo-[2.2.1] heptan-1-methansulfonsäure) and salts (Mexoryl SX)
- 3-(4'-Sulfo)-benzyliden-bornan-2-on and salts (Mexoryl SL)
- Polymer von N-{(2und 4)-[2-oxoborn-3-yliden)methyl}benzyl]acrylamid (Mexoryl SW)
- 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb® HEB);
- 2,2(-Methylen-bis(6-(2H-benzotriazol-2-yl)-4- (1,1,3,3-tetramethyl-butyl)phenol) (Tinosorb M);
- 2,4-Bis[4-(2-ethylhexyloxy)-2- hydroxyphenyl]-6-(4- methoxyphenyl)-1,3,5- triazin (Tinosorb S);
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der EP 0694521 B1 beschrieben;
- Dimethicodiethylbenzalmalonate (Parsol SLX).

Als wasserlösliche UV - Filter kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- 2,2(-(1,4-Phenylen)bis(1H-benzimidazol- 4,6-disulfon-säure, Mononatriumsalz) (Neo Heliopan AP)
- Sulfonsäurederivate von Benzophenonen, vorzugsweise 2-Hydroxy-4-methoxybenzo-phenon-5-sulfonsäure und ihre Salze;
- Sulfonsäurederivate des 3-Benzylidencamphers, wie z.B. 4-(2-Oxo-3-bornylidenmethyl)-benzolsulfonsäure und

2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).
Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der Fassung Commission Directive 2005/9/EC of 28 January 2005 amending Council Directive 76/768/EEC, concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pig-mente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 8/1996, S. 543-548 sowie Parf.Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. - Carotin, -Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, - Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.
Eine bevorzugte Ausführungsform der Erfindung betrifft kosmetischen und/oder pharmazeutische Zubereitungen, enthaltend 0,1 bis 80 Gew.% Dipropylheptylcarbonat und mindestens einen UV-Lichtschutzfilter ausgewählt aus der Gruppe

bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden-bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl} benzyl]acrylamid, 2-(2H-Benzotriazol-2-yl)-4-methyl-6-(2-methyl-3-(1,3,3,3-tetramethyl-1-(trimethylsilyloxy) disiloxanyl)propyl) phenol, Dimethicodiethyl-benzalmalonate und ihren Mischungen.

Diese UV-Lichtschutzfilter sind beispielsweise unter den folgenden Handelsnamen kommerziell erhältlich:

NeoHeliopan®MBC (INCI: 4-Methylbenzylidene Camphor; Hersteller: Symrise); NeoHeliopan® BB (INCI: Benzophenone-3, Hersteller: Symrise); Parsol®1789 (INCI: Butyl Methoxydibenzoylmethane, Hersteller: Hoffmann-La Roche (Givaudan); Tinosorb®S (INCI: Bis-Ethylhexyloxyphenol Methoxyphenyl Triazine); Tinosorb®M (INCI: Methylene Bis-Benzotriazolyl Tetramethylbutylphenol): Herstelller: BASF; Uvasorb®HEB (INCI: Diethylhexyl Butamido Triazone, Hersteller: 3V Inc.), Uvinul®T 150 (INCI: Ethylhexyl Triazone, Hersteller: BASF AG); Uvinul® A plus (INCI: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCI: Camphor Benzalkonium Methosulfate; Mexoryl®SX: 3,3'-(1,4-Phenylendimethin)-bis (7,7- dimethyl-2-oxobicyclo-[2.2.1] heptan-1-methansulfonsäure), CTFA: INCI Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCI Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy) disiloxanyl)propyl) phenol; INCI: DROMETRIZOLE TRISILOXANE; Parsol® SLX: Dimethicodiethylbenzalmalonate, INCI Polysilicone-15.

Weitere Beispiele sind pigmentäre Lichtfilter, wie beispielsweise anorganische pigmentäre Lichtfilter, wie Titandioxid und Zinkoxid und/oder organische pigmentäre Lichtfilter, wie Methylen-bis-benzotriazolyltetramethylbutylphenol (Tinosorb M).

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf das Gesamtgewicht der kosmetischen und/oder pharmazeutischen Zubereitung - enthalten.

Selbstbräuner

Als Selbstbräuner sind Substanzen zu verstehen, welche eine Bräunung der Haut verursachen. Beispielsweise seien Dihydroxyaceton, Erythrulose sowie alpha, betaungestättige Aldehyde genannt, welche mit den Aminosäuren der Haut im Sinne einer Maillard Reaktion zu gefärbten Verbindungen abreagieren. Als Wirkstoffe für Selbstbräuner kommen weiterhin in Frage natürliche oder synthetische Ketole oder Aldole. Als geeignete Wirkstoffe seien exemplarisch genannt Dihydroxyaceton, Erythrulose, Glycerolaldehyd, Alloxan, Hydroxymethylglyoxal, gamma-Dialdehyd, 6-Aldo-D-Fructose, Ninhydrin und meso-Weinsäuredialdehyd. Als Selbstbräuner eignen sich insbesondere Dihydroxyaceton und/oder Erythrulose.

Als besonders vorteilhaft haben sich Mischungen der o. g. Wirkstoffe untereinander oder mit Mucondialdehyd oder/und Naphthochinone wie z. B. 5-Hydroxy-1,4-naphthochinon (Juglon) und 2-Hydroxy-1,4-Naphthochinon erwiesen.

Die Selbstbräuner sind üblicherweise in Konzentrationen von 1 bis 10, insbesondere von 2 bis 5 Gew.-% -bezogen auf das Gesamtgewicht der kosmetischen und/oder pharmazeutischen Zubereitung, enthalten.

Die erfindungsgemäßen kosmetischen und/oder pharmazeutischen Zubereitungen können beispielsweise als O/W oder W/O Pflegeemulsionen, Sonnenschutzformulierung, AP/Deo Konzepte, Formulierungen für die dekorative Kosmetik, ölige Pflegezubereitungen, Tränkflüssigkeiten für Substrate, wie beispielsweise Papier- und Vliessprodukte vorliegen. Exemplarisch seien genannt Wet Wipes, Taschentücher, Windeln oder Hygieneprodukte.

Die erfindungsgemäßen kosmetischen und/oder pharmazeutischen Zubereitungen, eignen sich insbesondere auch für leichte, sprühbare Anwendungen und/oder als Bestandteile von Pflegeemulsionen für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung. Sie lassen sich auf Tissues, Papiere, Wipes, Vlies-Produkte, Schwämme, Puffs, Pflaster und Bandagen applizieren, die ihren Einsatz im Bereich der Reinigung, Hygiene und/oder Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes, Toilettenpapier, Erfrischungstücher, After-shave Tücher). Sie lassen sich u.a. auch in Zubereitungen zur Haarpflege, Haarreinigung oder Haarfärbung einsetzen. Die erfindungsgemäßen kosmetischen und/oder

pharmazeutischen Zubereitungen zeichnen sich durch positives sensorisches Verhalten bei Applikation aus.

Die erfindungsgemäßen kosmetischen und/oder pharmazeutischen Zubereitungen eignen sich als Zubereitungen der dekorativen Kosmetik, wie beispielsweise Lippenstifte, Augen-Make up, wie beispielsweise Lidschatten, Mascara, Lidstifte, Kajal, Nagellack, etc. sowie Make-up Formulierungen.

Pigmente

Der Begriff Pigment umfasst Partikel jeder Form, die weiß oder gefärbt, organisch oder anorganisch sind, in den Zubereitungen nicht löslich sind, und dem Zweck dienen die Zubereitung zu färben. In einer bevorzugten Ausführungsform werden anorganische Pigmente verwendet, besonders bevorzugt sind Metalloxide.

Als anorganische Pigmente seien exemplarisch genannt: Titandioxid, optional oberflächenbeschichtet, Zirkonium oder Ceriumoxide und Zink-, Eisen- (Schwarz, Gelb oder Rot) und Chrom-oxide, Manganviolett, Ultramarine Blau, Chromhydroate und Eisen(III)blau, Metallpulver wie Aluminiumpulver oder Kupferpulver.

In einer bevorzugten Ausführungsform der Erfindung ist das Pigment ausgewählt aus den anorganischen Pigmenten, vorzugweise aus den Metalloxiden. In einer bevorzugten Ausführungsform ist das Pigment ausgewählt aus der Gruppe bestehend aus Titandioxid, Zinkoxid, Eisenoxid und Mischungen daraus. Die anorganische Pigmente können farbgebend sein. Besonders bevorzugte, anorganische, farbgebende Pigmnente sind Metalloxide und insbesondere das Eisenoxid.

Die Pigmente können sowohl einzeln als auch in Mischungen vorliegen.

Bevorzugt im Sinne der vorliegenden Erfindung sind Pigmentmischungen aus Weiss-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen koennen. Unter den Farbpigmenten sind Eisenoxide besonders bevorzugt.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäss vorteilhaft zusätzlich zu einem oder mehreren Weiss- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenfoermiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenfoermige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phaenomen der Interferenz (Interferenzpigmente) (DIN 55944: 2003-11).

Beispiele für erfindungsgemäss bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).

Folgende Pigmente können ebenfalls erfindungsgemäß verwendet werden.

Pigmente auf Glimmerbasis:

Cellini®, Cloisonné®, Duocrome®, Flamenco®, Gemtone®, Timica®, MultiReflections™

Das Substrat Glimmer wird mit Metalloxiden - Titandioxid ($TiO_2$)und/oder Eisenoxid ($Fe_2O_3$), um weiße, interferierende und metallische Effekte zu erzeugen. Die reflektierende Farbe hängt von der Dicke der Metalloxidbeschichtung ab. Zur Erzeugung von absorption und komplexer Farbwanderungseffekte, werden ebenfalls zusätzliche Pigmente verwendet. Diese anorganischen und organischen Pigmente sind Chromoxid, Eisenoxide, Eisenferrocyanid (Eisenblau), Carmin und D&C/FD&C-Farben.

Pigmente auf Basis Calciumnatriumborsilicat:

Plättchen aus Reflecks™, Reflecks™ Dimensions, und Reflecks™ MultiDimensions oder anderen Farbstoffen werden mit Metalloxiden und/oder anderen Farbmitteln beschichtet, um Effektpigmente mit verstärkter Farbreinheit, Helligkeit, Transparenz und Reflexionsvermögen zu erzeugen. Reflecks™ MultiDimensions besteht aus Plättchen aus Calciumnatriumborsilicat, die mit Titandioxid und Siliciumdioxid ($SiO_2$) beschichtet sind. Diese Pigmente ermöglichen unverwechselbare Farbwanderungseffekte.

Pigmente auf Basis von synthetischem Glimmer:

Chione™
Metalloxide sind auf Substrate aus synthetischem Glimmer aufgetragen. Auf grund der sauberen Hauptfarbe des Substrats ist das daraus entstandene Aussehen durch sehr helle Farben mit verstärkter Chroma charakterisiert. Diese Pigmente auf Basis von synthetischem Glimmer sind für die Verwendung in solchen Produkten ideal, wo breite Perlglanzeffekte und irisierende Effekte gewünscht sind.

Pigmente auf Basis von Wismuthoxichlorid:

Biju®, Mearlite®, Pearl-Glo®, Chroma-Lite®
Plättchen aus Wismuthoxichloridkristallen warden aus einer Lösung gefällt. Jeder Grad hat seine eigene Größe, Form, Dicke und Schimmer. Möglich sind auch Pigmente aus dem Bereich Chroma-Lite, wo Wismuthoxichlorid mit Glimmer und zusätzlichen Farbmitteln kombiniert sind.

Specialty Performance Minerals:

Mearlmica®, Chione™ M-SVA, Bi-Lite®,
Specialty performance minerals sind eine Familie aus Mineralprodukten, die eine Balance und Konsistenz in gewisse Typen von kosmetischen Zusammensetzungen abgeben, während diese optisch neutral bleiben. Sie bewirken ebenfalls eine verbesserte Textur, insbesondere in Pulveranwendungen.

Ferner können die erfindungsgemässen Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weisspigmente sind ohne praktische Bedeutung.
Die Pigmente können vorteilhaft im Sinne der vorliegenden Erfindung auch in Form kommerziell erhältlicher öliger oder wässriger Vordispersionen zur Anwendung kommen.
Die erfindungsgemäßen Zubereitungen enthalten üblicherweise 0,1 bis 40 Gew.-% Pigmente - bezogen auf das Gesamtgewicht der kosmetischen und/oder pharmazeutischen Zubereitung.
Es ist ferner vorteilhaft im Sinne der vorliegenden Erfindung, wenn die erfindungsgemässe Zubereitung einen oder mehrere Farbstoffe enthält.
Die Farbstoffe können sowohl synthetischen als auch natürlichen Ursprungs sein. Eine Liste von geeigneten Farbstoffen findet sich in EP 1 371 359 A2, S.8, Z. 25-57, S.9 und S.10 sowie S.11, Z. 1 bis 54, auf welche hiermit explizit Bezug genommen wird.
Die erfindungsgemäßen Zubereitungen enthalten üblicherweise 0,01 bis 5, vorzugsweise 0,1 bis 1,0 Gew.-% Farbstoffe- bezogen auf das Gesamtgewicht der kosmetischen und/oder pharmazeutischen Zubereitung. Die erfindungsgemäßen Zubereitungen enthalten üblicherweise eine Gesamtmenge an Farbstoffen und Pigmenten im Bereich von 0,01 bis 30 Gew.-%, insbesonderen 0,1 bis 15 Gew.-%, vorzugsweise 1 bis 10 Gew.-%, bezogen auf das Gesamtgewicht der kosmetischen und/oder pharmazeutischen Zubereitung.
Als Farbstoffe und Pigmente eignen sich insbesondere die gemäß Annex IV der Kommissions Direktive zugelassenen Farbstoffe und Pigmente (in der Fassung: Commission Directive 2007/22/EC of 17 April 2007 amending Council Directive 76/768/EEC, concerning cosmetic products, for the purposes of adapting Annexes IV and VI thereto to technical progress) zugelassen Stoffe, auf die hiermit explizit Bezug genommen wird.

Puderrohstoffe / Füllstoffe / Sensorikmodifikatoren

Geeignete Füllstoffe können Talk, Siliciumoxid, Zinkstearat, Glimmer, Kaolin, Nylon (insbesondere Orgasol)pulver, Polyethylenpulver, Polypropylenpulver, Acrylatpulver, Teflon, Stärke, Bornitrid, Copolymermikrokugeln, wie Expancel (Nobel Industrie), Polytrap (Dow Coming) und Siliconharzmikroperlen (Tospearl from Toshiba).
Andere Füllstoffe, die in den Zusammensetzungen der Erfindung verwendet werden können, umfassen andere anorganische Pulver wie Kalk, hochdisperse Kieselsäure, Calciumoxid, Calciumcarbonat, Magnesiumoxid, Magnesiumcarbonat, Fuller's Erde, Attapulgit, Bentonit, Muscovit, Phlogopit, synthetischer Glimmer, Lepidolit, Hectorit, Biotit, Lithionglimmer, Vermiculit, Aluminumsilicat, Aluminummagnesiumsilicat, Diatomeenerde, Stärke, Alkyl- und/oder Trialkylarylammoniumsmectite, chemisch modifiziertes Magnesiumaluminumsilicat, organisch modifizierter Montmorillonitton, hydratisiertes Aluminumsilicat, hydratisierte Kieselsäure, hochdisperses Aluminumstärke-Octenyl succinatbariumsilicat, Calciumsilicat, Magnesiumsilicat, Strontiumsilicat, Metallwolframat, Magnesium, Siliciumoxid-Aluminiumoxid, Zeolit, Bariumsulfat, calciniertes Calciumsulfat (calcinierter Gips), Calciumphosphat, Fluorapatit, Hydroxyapatit, Keramikpulver, Metallseife (Zinkstearat, Magnesiumstearat, Zinkmyristat, Calciumpalmitat and Aluminumstearat), colloidales Siliciumdioxid; organisches Pulver, Cyclodextrin, Methylpolymethacrylatpulver, Copolymerpulver aus Styrol und Acrylsäure, Benzoguanaminharzpulver und Poly(ethylentetrafluorid) pulver; Siliciumoxiddimethylsilylat, Methylmethacrylat-Kreuzpolymer, Silsesquioxan, Vinyldimethicon/Methiconsilsesquioxan-Kreuzpolymer, Lauroyllysin.

Die kosmetischen und/oder pharmazeutischen Zubereitungen können Formulierungen zur Körperpflege sein, z. B. eine Körpermilch, Cremes, Lotionen, sprühbare Emulsionen, Produkte zur Eliminierung des Körpergeruchs etc. Dipropylheptylcarbonat lässt sich auch in tensidhaltigen Formulierungen wie z. B. Schaum- und Duschbädern, Haarshampoos und Pflegespülungen einsetzen. Je nach Applikationszweck enthalten die kosmetischen und/oder pharmazeutischen Zubereitungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Die kosmetischen und/oder pharmazeutischen Zubereitungen können 0,1 bis 80 Gew.-% Dipropylheptylcarbonat und mindestens einen Emulgator und/oder ein Tensid und/oder eine Wachskomponente und/oder ein Polymer und/oder einen weiteren Ölkörper enthalten.

## Emulgator

In einer Ausführungsform der Erfindung enthalten die erfindungsgemäßen Zubereitungen mindestens einen Emulgator.

Die erfindungsgemäßen Zubereitungen enthalten den/die Emulgator(en) üblicherweise in einer Menge von 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zubereitung.

Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Grösse und Stärke der hydrophilen und der lipophilen Gruppen eines Emulgators aus. Der HLB-Wert eines Emulgators lässt sich auch aus Inkrementen errechnen, wobei die HLB-Inkremente für die verschiedenen hydrophilen und hydrophoben Gruppen, aus denen sich ein Molekül zusammensetzt. In der Regel kann er Tabellenwerken (z. B. H. P. Fiedler, Lexikon der Hilfsstoffe für Pharmazie, Kosmetik und angrenzende Gebiete, Editio Cantor Verlag, Aulendorf, 4. Aufl. 1996) oder den Herstellerangaben entnommen werden. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Öiphase entsteht unter sonst gleichen Herstellungsbedingungen eine W/O-Emulsion.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

## Nicht-ionische Emulgatoren

Zur Gruppe der nicht-ionischen Emulgatoren gehören beispielsweise:

(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.

(2) $C_{12}$-$C_{18}$-Fettsäuremono- und -diester von Anlagerungsprodukten von 1 bis 50 Mol Ethylenoxid an Glycerin.

(3) Sorbitanmono- und -diester von gesättigten und ungesättigten Fettsäuren mit 6 bis 22 Kohlenstoffatomen und deren Ethylenoxidanlagerungsprodukte.

(4) Alkylmono- und -oligoglycoside mit 8 bis 22 Kohlenstoffatomen im Alkylrest und deren ethoxylierte Analoga.

(5) Anlagerungsprodukte von 7 bis 60 Mol Ethylenoxid an Ricinusöl und/oder gehärtetes Ricinusöl.

(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.

(7) Anlagerungsprodukte von 2 bis 15 Mol Ethylenoxid an Ricinusöl und/oder gehärtetes Ricinusöl.

(8) Partialester auf Basis linearer, verzweigter, ungesättigter bzw. gesättigter $C_6$-$C_{22}$-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischesterwie z. B. Glycerylstearatcitrat und Glycerylstearatlactat.

(9) Polysiloxan-Polyalkyl-Polyether-Copolymere bzw. entsprechende Derivate.

(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. $C_{12/18}$-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls® PGPH" (W/O-Emulgator) oder "Eumulgin® VL 75" (Abmischung mit Lauryl Glucosides im Gewichtsverhältnis 1:1, O/W-Emulgator) oder Dehymuls® SBL (W/O-Emulgator) von der BASF Personal Care and Nutrition Deutschland GmbH vertrieben werden. In diesem Zusammenhang sei insbesondere auf das Europäische Patent EP 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Besonders bevorzugte Emulgatoren sind z. B. Cetyl Dimethicone Copolyol (z.B. Abil EM-90), Polyglyceryl-2 Dipolyhydroxystearate (z.B. Dehymuls PGPH), Polyglycerin-3-Diisostearate (z.B. Lameform TGI), Polyglyceryl-4 Isostearate (z.B. Isolan GI 34), Polyglyceryl-3 Oleate (z.B. Isolan GO 33), Diisostearoyl Polyglyceryl-3 Diisostearat (z.B. Isolan PDI), Polyglyceryl-3 Methylglucose Distearate (z.B. Tego Care 450), Polyglyceryl-3 Beeswax (z.B. Cera Bellina), Polyglyceryl-4 Caprate (z.B. Polyglycerol Caprate T2010/90), Polyglyceryl-3 Cetyl Ether (z.B. Chimexane NL), Polyglyceryl-3 Distearate (z. B. Cremophor GS 32) und Polyglyceryl Polyricinoleate (z.B. Admul WOL 1403), Glyceryl Oleate (z.B. Monomuls 90-O 18), Alkyl Glucoside (z.B. Plantacare 1200, Emulgade PL 68/50, Montanov 68, Tego Care CG 90, Tego Glucosid L 55), Methyl Glucose Isostearate (z.B. Tego Care IS), Methyl Glucose Sesquistearate (Tego Care PS), Sodium Cocoyl Hydrolyzed Wheat Protein (z.B. Gluadin WK), Potassium Cetyl Phosphate (z.B. Amphisol K, Crodafos CKP), Sodium Alkylsulfate (z.B. Lanette E), Sucrose Ester (z.B. Crodesta F-10, F-20, F-50, F-70, F-110, F-160, SL-40, Emulgade® Sucro), ethoxylierte und/oder propoxylierte Fettalkohole Fettsäuren, Rizinusöle bzw. hydrierte Rizinusöle (z.B. Eumulgin B2, B2, B3, L, HRE 40, HRE 60, RO 40, Cremophor HRE 40, HRE 60, L, WO 7, Dehymuls HRE 7, Arlacel 989), PEG-30 Dipolyhydroxystearate (z.B. Arlacel P 135, Dehymuls LE), Sorbitan Ester, Sorbitan Ester ethoxyliert und/oder propoxyliert sowie deren Gemische. Ein besonders effektives Gemisch besteht aus Polyglyceryl-2 Dipolyhydroxystearate und Lauryl Glucoside und Glycerin (z.B. Eumulgin VL 75). Geeignet sind weiterhin Polyglyceryl-4 Diisostearat/ Polyhydroxystearate/Sebacate (Isolan® GPS), Düsostearoyl Polyglyceryl-3 Diisostearate (z. B. Isolan PDI), Alkalisalze Acylglutamate (z.B. Eumulgin SG).

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer,"Encyclopedia of Chemical Technology", 3. Aufl., 1979, Band 8, Seite 913, aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von $C_4$-$C_6$-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitan-dihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitan-trimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nichtionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 -40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin® HRE 40 (INCI: PEG-40 Hydrogenated Castor Oil), Eumulgin® HRE 60 (INCI: PEG-60 Hydrogenated Castor Oil), Eumulgin® L (INCI: PPG-1-PEG-9 Laurylglycolether), sowie Eumulgin® SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. $C_8$-$C_{22}$-Alkylmono- und - oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare® zur Verfügung stehen, enthalten eine glucosidisch gebundene $C_8$-$C_{16}$-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade® PL 68/50 von der BASF Personal Care and Nutrition Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin® VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur:

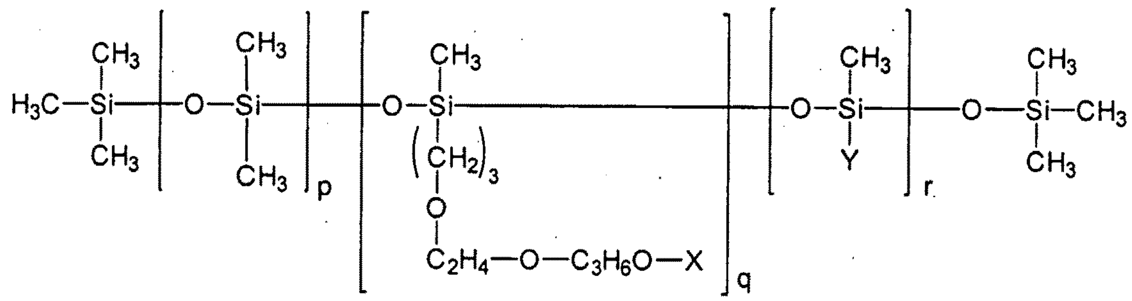

bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von Evonik Evonik unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden. Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikEvonik unter der Warenbezeichnung ABIL® EM 90 verkauft wird. Ein weiteres Beispiel für besonders vorteilhaft im Sinne der vorliegenden Erfindung zu verwendende grenzflächenaktiven Substanzen ist das Cyclomethicon Dimethiconcopolyol, welches von Evonik Evonik unter der Warenbezeichnung ABIL®EM 97 und ABIL®WE 09 verkauft wird. Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist. Ein weiterer vorteilhafter Silikonemulgator ist Octyl Dimethicon Ethoxy Glucosid der Firma Wacker.

Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z. B. ABIL® EM 90 Evonik Evonik), DC5200 Formulation Aid (Dow Corning)] sowie beliebige Mischungen aus beiden Emulgatoren.

Tenside

In einer Ausführungsform der Erfindung enthalten die erfindungsgemäßen Zubereitungen mindestens ein Tensid.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekuelteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Als Tenside werden üblicherweise oberflächen aktive Substanzen verstanden, die einen HLB-Wert von größer 20 aufweisen.

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Die erfindungsgemäßen Zubereitungen enthalten den/die Tenside üblicherweise in einer Menge von 0 bis 40 Gew.-%., vorzugsweise 0,05 bis 30 Gew.-% insbesondere 0,05 bis 20 Gew.-%, vorzugsweise 0,1 bis 15 Gew.-% und insbesondere 0,1 bis 10 Gew.-% bezogen auf das Gesamtgewicht der Zubereitung.

Typische Beispiele für nichtionische Tenside sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfett¬säureester, Polyglycerinfettsäurester, Zuckerester, Sorbi¬tan¬ester, Poly¬sor¬bate und Aminoxide, Polyethermodifizierte Polysiloxane, Polyethermodifizierte Siloxanelastomere.. Sofern die nichtionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine -$COO^{(-)}$- oder-$SO_3^{(-)}$ -Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine-COOH-oder-$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, $\alpha$-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

Anionische Tenside sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für anionische Tenside sind Seifen, Alkali (z.B. Natrium und Kalium)-Salze, Ammonium- und Alkylammoniumsalze, Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Alkyllactate, Alkyltartrate, Alkylcitrate, Alkylphosphate, N-Acylaminosäuren, wie beispielsweise Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

Wachskomponente

In einer Ausführungsform der Erfindung enthalten die erfindungsgemäßen Zubereitungen mindestens eine Wachskomponente.

Die erfindungsgemäßen Zubereitungen enthalten den/die Wachskomponente(n) üblicherweise in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zubereitung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten TriglyceridGemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der BASF Personal Care and Nutrition Deutschland GmbH & Co. KG vermarkteten Produkte Novata AB und Novata B (Gemisch aus C12-C18-Mono-, Di- und Triglyceriden), sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen.

Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der BASF Personal Care and Nutrition Deutschland GmbH unter der Bezeichnung Lanette 18 (C18-Alkohol), Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oder -stearat kommen hierfür in Frage.

Als Wachse eignen sich weiterhin Perlglanzwachse. Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Polymere

In einer Ausführungsform der Erfindung enthalten die erfindungsgemäßen Zubereitungen mindestens ein Polymer.

Die erfindungsgemäßen Zubereitungen enthalten das/die Polymere(n) üblicherweise in einer Menge von 0 bis 20 Gew.-%, vorzugsweise 0,1 bis 15 Gew.-% und insbesondere 0,1 bis 10 Gew.-% bezogen auf das Gesamtgewicht der Zubereitung.

Geeignete kationische Polymere sind beispielsweise kationische Cellulosederivate, wie z. B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie

beispielsweise Lauryldimonium hydroxypropyl hydrolyzed collagen (Lamequat®L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen sowie beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone® Gel VS-5PC (Elementis).

Ebenso geeignet sind sogenannte quaternäre Polymere, z.B. mit der INCI - Bezeichnung Polyquaternium-37, die der folgenden allgemeinen Formel entsprechen:

Alternativ können auch andere Dialkylaminoalkyl (meth)acrylate sowie deren durch Alkylierung oder Protonierung erhältliche Ammoniumsalze oder Dialkylaminoalkyl (meth)acrylamide sowie deren durch Alkylierung oder Protonierung erhältliche Ammoniumsalze eingesetzt werden. Besonders bevorzugt sind Polymere enthaltend MAPTAC, APTAC, MADAME, ADAME, DMAEMA und TMAEMAC. Darüberhinaus können auch Co-polymere mit anionischen, weiteren kationischen oder ungeladenen Monomeren erfindungsgemäß eingesetzt werden, insbesondere solche, die neben den genannten

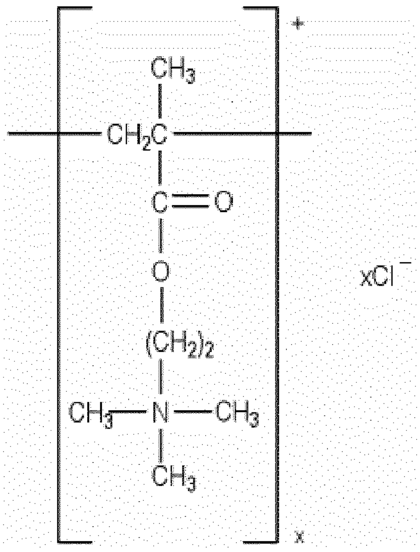

Alkylaminoalkyl (meth)acrylat oder-(meth)acrylamid Monomeren zusätzlich (Meth)acrylsäure und/oder 2-Acrylamido-2-methyl-propansulfonsäure und/oder Acrylamid und/oder Vinylpyrrolidon und/oder Alkyl(meth)acrylate enthalten. Beispielhaft seien solche Polymere mit der INCI Bezeichnung Polyquaternium-11, Polyquaternium-13, Polyquaternium-14, Polyquaternium-15, Polyquaternium-28, Polyquaternium-32, Polyquaternium-43, Polyquaternium-47 genannt.

Ölkörper

In einer Ausführungsform der Erfindung enthalten die erfindungsgemäßen Zubereitungen mindestens einen Ölkörper. Üblicherweise enthalten die erfindungsgemäßen Zubereitungen Dipropylheptylcarbonat als Ölkörper. In der hier als bevorzugte genannten Ausführungsform enthalten die Zubereitungen somit einen von dem erfindungsgemäßen Kohlenstoffen verschiedenen Ölkörper, auch als "weiterer Ölkörper" bezeichnet.

Ein weiterer Gegenstand der Erfindung betrifft daher kosmetische und/oder pharmazeutische Zubereitungen, enthaltend 1 bis 80 Gew.-% Dipropylheptylcarbonat mindestens einen (weiteren) Ölkörper.

Die Ölkörper (Dipropylheptylcarbonat plus weitere Ölkörper) sind üblicherweise in einer Gesamtmenge von 0,1 - 90, insbesondere 0,1 - 80, insbesondere 0,5 bis 70, bevorzugt 1 bis 60, insbesondere 1 bis 50 Gew.-%, insbesondere 1 bis 40 Gew.-%, vorzugsweise 5-25 Gew.-% und insbesondere 5 - 15 Gew.-% enthalten. Die weiteren Ölkörper sind üblicherweise in einer Menge von 0,1 bis 40 Gew.-% bezogen auf das Gesamtgewicht der Zubereitung enthalten.

Das Öl und/oder Wachs in der erfindungsgemäßen texturierten Zusammensetzung ist aus der Gruppe Fettsäureester, Guerbetalkohole, Tri- oder Partialglyceride, Mono-/Dialkylether, Mono-/Dialkylcarbonate, öllösliche UV-Filter, Fettalkoholether, mikrokristalline Wachse, Kohlenwasserstoffe bzw. Mineralöl, Silikonöl, natürliche pflanzliche Öle und deren Mischungen ausgewählt.

Bevorzugte Öle und/oder Wachse sind Guerbetalkohole auf Basis von Fettalkoholen mit 6 bis 18, vorzugsweise 8 bis 10 Kohlenstoffatome (Eutanol G, Eutanol G 16), Ester von linearen $C_6$-$C_{22}$-Fettsäuren mit linearen oder verzweigten $C_6$-$C_{22}$-Fettalkoholen bzw. Ester von verzweigten $C_6$-$C_{13}$-Carbonsäuren mit linearen oder verzweigten $C_6$-$C_{22}$-Fettalkoholen, wie z. B. Myristylmyristat (Cetiol® MM), Myristylpalmitat, Myristylstearat, Myristylisostearat, Myristyloleat, Myristylbehenat, Myristylerucat, Cetylmyristat, Cetylpalmitat (Cutina® CP), Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Isopropyl Myristate, Isopropyl Palmitate, Oleylmyristat, Oleylpalmitat, Oleylstearat, Oleylisostearat, Oleyloleat, Oleylbehenat, Oleylerucat (Cetiol® J 600), Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat, Ethylhexyl Stearate (Cetiol® 868), Hexyl Laurate (Cetiol® A), C12.15 Alkyl Benzoate (Cetiol® AB), Dibutyl Adipate (Cetiol® B), Coco-Caprylate (Cetiol® C5), Coco-Caprylate/Caprate (Cetiol® LC, Cetiol® C 5C), Propylheptyl Caprylate (Cetiol® Sensoft), Cetearyl Isononanoate (Cetiol® SN), Decyl Oleate (Cetiol® V), Cetearyl Ethylhexanoate (Luvitol® EHO), Daneben eignen sich Ester von $C_{18}$-$C_{38}$-Alkylhydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) wie Propylene Glycol Dicaprylate/Dicaparte (Myritol® PGDC). und/oder Guerbetalkoholen, Triglyceride auf Basis $C_6$-$C_{10}$-Fettsäuren flüssige Mono-/Di-/Triglyceridmischungen auf Basis von $C_6$-$C_{18}$-Fettsäuren (Myritol® 331, Myritol® 312, Myritol® 318), , Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z. B. Dica-prylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z. B. Finsolv® TN, Cetiol® AB), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen (Cetiol E). Weitere geeignete Emollients sind pflanzliche Öle (Cegesoft® GPO, Cegesoft® PFO, Cegesoft® PS 6, Cegesoft® SBE, Cegesoft® SH) und Mischungen daraus (Cegesoft® VP), Silikonöle, Kohlenwasserstoffe wie Mineralöle, Paraffinum Liquidum, Undecane / Tridecane (Cetiol® Ultimate), Hydrogenated Polyisobutene (Luvitol® Light), Mineralöle, Isoparaffine, Paraffine.

Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von $C_{18}$-$C_{38}$-Alkylhydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen..

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel

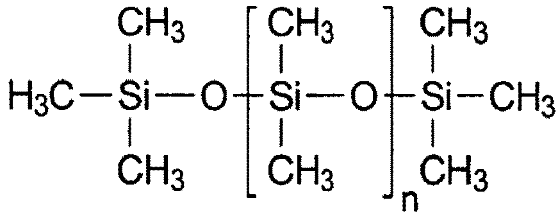

auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handelsbezeichnungen Abil 10 bis 10 000 bei Evonik Evonik erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Evonik erhältlich sind. Aber auch andere Silikonöle sind vorteilhaft im Sinne der vorliegenden Erfindung zu verwenden, beispielsweise Cetyidimethicon, Hexamethylcyclo- trisiloxan, Polydimethylsiloxan, Poly (methylphenylsiloxan). Erfindungsgemäß besonders bevorzugte Silikone sind Dimethicon und Cyclomethicon.

Die erfindungsgemäßen Zubereitungen können weiterhin biogenen Wirkstoffe, Insekten-Repellentien, Tyrosinase Inhibitoren, Konservierungsmittel, Parfümöle, Überfettungsmittel, Stabilitsatoren und/oder Hydrotrope enthalten.

Ein weiterer Gegenstand der Erfindung betrifft daher kosmetische und/oder pharmazeutische Zubereitungen, enthaltend 0,1 bis 80 Gew.-% Dipropylheptylcarbonat und mindestens einen biogenen Wirkstoff, Insekten-Repellent, Tyrosinase Inhibitor, Konservierungsmittel, Parfümöl, Stabilisator und/oder Hydrotrop.
Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Aloe vera, Prunusextrakt, Bambaranussextrakt und Vitaminkomplexe zu verstehen.

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als Konservierungsmittel eignen sich beispielsweise Phenoxyethanol, Formaldehydlösung, Parabene, Pentandiol oder Sorbinsäure, Benzoesäure und deren Salze, Benzyl Alcohol, Benzyl Salicylate. Dehydroacetic Acid, Methylthiazolinon oder Sorbinsäure und deren Salze sowie die unter der Bezeichnung Surfacine® bekannten Silberkomplexe sowie die in Anlage 6, Teil A und B der Kosmetikverordnung aufgeführten weiteren Stoffklassen. Daneben kommen Substanzen zum Einsatz, die als Konservierungsmittelhelfer fungieren wie Ethylhexylglycerin und Caprylyl Glycol sowie Polyole bzw. Alcohol wie Propandiol, Phenylpropanol, Phenethyl Alkohol und Undecyl Alkohol, sowie die unter der Bezeichnung Surfacine® bekannten Silberkomplexe. Weiterhin eignen sich als Konservierungsmittel die in WO 07/048757 beschriebenen 1,2 Alkandiole mit 5 bis 8 C-Atomen.

Als Konservierungsmittel eignen sich insbesondere die gemäß Annex VI der Kommissions Direktive (in der Fassung Commission Directive 2007/22/EC of 17 April 2007 amending Council Directive 76/768/EEC, concerning cosmetic products, for the purposes of adapting Annexes IV and VI thereto to technical progress) zugelassen Stoffe, auf die hier explizit Bezug genommen wird.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als Stabilisatoren können Metallsalze von Fettsäuren, wie z. B. Magnesium-, Aluminium- und/oder Zinkstearat bzw. -ricinoleat eingesetzt werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Beispiele

Körpermilch intensiv

| Phase | Bestandteile | INCI | Gew.-% | Funktion |
|---|---|---|---|---|
| A | Dehymuls PGPH | Polyglyceryl-2 Dipolyhydroxystearat | 4 | Emulgator (W/O) |
| | Monomuls 90-O 18 | Glyceryl Stearat | 1 | Emulgator (W/O) |
| | Zink Stearat | Zink stearat | 0,50 | Stabilisierungsmittel |
| | Paracera M | Cera Microcristallina | 0,50 | Konsistenzmittel |
| | Sonnenblumenöl | Helianthus Annuus (Sonnenblumen) Samen Öl | 6 | Weichmacher |
| | Cetiol 4 all | Dipropylheptyl carbonat | 9 | Weichmacher |
| | Cetiol 868 | Ethylhexyl Stearat | 8 | Weichmacher |
| | Cetiol SB 45 | Butyrospermum Parkii Butter | 0,50 | Weichmacher |
| | Copherol F 1300 C | Tocopherol | 0,50 | Aktiver Bestandteil |
| B | Glycerin | Glycerin | 3 | Feuchthaltemittel |
| | Magnesium Sulfat | Magnesium Sulftat | 1 | Stabilisierungsmittel |
| | Wasser | Wasser | 64 | |
| C | Euxyl PE 9010 | Phenoxyethanol, Ethylhexylglycerin | 1 | Konservierungsmitt el |
| | Parfüm | Parfüm | 0,40 | Duftstoff |
| Viskosität | | | 9000 mPas | |

Lippenstift

| Phase | Bestandteile | INCI | Gew.-% | Funktion |
|---|---|---|---|---|
| A | Candelilla Wachs SP 75 | Candelilla Cera | 2,70 | Konsistenzmittel |
| | Performalene PL | Polyethylene | 5 | Konsistenzmittel |
| | Cosmedia DC | Hydrogenated Dimer Dilinoleyl/ Dimethylcarbonat Copolymer | 30 | Filmbildendes Mittel |
| | Lameform TGI | Polyglyceryl-3 Diisostearat | 20 | Emulgator (W/O) |
| | Eutanol G | Octyldodecanol | 10 | Weichmacher |
| | Cetiol 4 all | Dipropylheptyl carbonat | 10,21 | Weichmacher |
| | Cetiol J 600 | Oleyl Erucat | 10 | Weichmacher |
| | Beeswax White SP 422 | Bienenwachs | 5 | Konsistenzmittel |
| B | OX DE Titane Organophile W877 | Titanium Dioxid | 0,20 | Farbstoff |

(fortgesetzt)

| Phase | Bestandteile | INCI | Gew.-% | Funktion |
|---|---|---|---|---|
| | SunCroma D&C Red 6 Ba Lake C19-012 | CI 15850 | 0,24 | Farbstoff |
| | SunCroma D&C Red 7 Ca Lake C19-003 | CI 15850 | 0,05 | Farbstoff |
| C | Reflecks Dimensions Sparkling gold G230D | Calcium Sodium Borosilicate, Titanium Dioxide | 2 | Effektpigment |
| | Reflecks Dimensions Sparkling Red G430D | Calcium Sodium Borosilicate, Titanium Dioxide | 2 | Effektpigment |
| | Ultra Filing Spheres C00487 | Ethylhexyl Palmitat, Trihydroxystearin, Sodium Hyaluronat, Glucomannan | 2 | Aktiver Bestandteil |
| | Vitamin E-acetat Care | Tocopheryl Acetat | 0,50 | Aktiver Bestandteil |
| | Tinogard TL | Benzotriazolyl Dodecyl p-Cresol | 0,10 | Lichtschutzmittel |

Lip Gloss

| Phase | Bestandteil | INCI | Gew.-% | Funktion |
|---|---|---|---|---|
| A | Cetiol 4 all | Dipropylheptyl carbonat | 8 | Weichmacher |
| | Eutanol G | Octyldodecanol | 5,65 | Trägerstoff |
| | Phytosoothe LS 9766 | Brassica Campestris (Rapeseed) Sterols, Cetearyl Alkohol | 1 | Aktiver Bestandteil |
| | Uvinul MC 80 | Ethylhexyl Methoxycinnamate | 8 | UV-B-Filter |
| | Uvinul A Plus Granular | Diethylamino Hydroxybenzoyl Hexyl Benzoat | 2 | UV-A-Filter |
| | Tinogard TT | Tetradibutyl Pentaerithrityl Hydroxyhydrocinnamat | 0,03 | Antioxidans |
| B | Panalane H 300 E | Hydrogenated Polyisobutene | 35 | Weichmacher |
| | Cosmedia DC | Hydrogenated Dimer Dilinoleyl/Dimethylcarbonat Copolymer | 10 | Filmbildendes Mittel |
| | Aerosil R 974 | Silica Dimethyl Silylat | 4,50 | Rheologiemodifikator |
| | Lameform TGI | Polyglyceryl-3 Diisostearat | 18 | Emulgator (W/O) |
| C | SunCroma D&C Red 6 Ba Lake C19-012 | CI 15850 | 2 | Farbstoff |
| | SunCroma D&C Yellow 10 Al Lake C71-5171 | CI 47005 | 1 | Farbstoff |
| | Chione HD Crisp Gold S230V | Synthetic Fluorphlogopite, Titanium Dioxid | 1,50 | Effektpigment |
| | Chione HD Digital Pink S430V | Synthetic Fluorphlogopite, Titanium Dioxid | 0,80 | Effektpigment |

(fortgesetzt)

| Phase | Bestandteil | INCI | Gew.-% | Funktion |
|---|---|---|---|---|
| D | Vitamin E-acetat Care | Tocopheryl Acetat | 0,50 | Aktiver Bestandteil |
| | C00087 Hyaloronic FS | Ethylhexyl Palmitat, Silica Dimethyl Silylate, Butylene Glycol, Sodium Hyaluronat, Caprylyl Glycol, Phenoxyethanol, Hexylene Glycol | 2 | Aktiver Bestandteil |
| | Tinogard TL | Benzotriazolyl Dodecyl p-Cresol | 0,02 | Lichtschutzmittel |
| | Parfüm | Parfüm | q.s. | Duftstoff |

Balsam zur Befeuchtung und zum Schutz der Lippen

| Phase | Komponente Handelsname | INCI | Gew.-% |
|---|---|---|---|
| I. | Cerilla Raffinée G* | Candelilla (Euphorbia Cerifera) Wax | 7,53 |
| | CUTINA® LM conc. | Polyglyceryl-2 Dipolyhydroxystearate and Octyldodecanol and Copernicia Cerifera (Carnauba) Wax and Euphorbia Cerifera (Candelilla) Wax and Beeswax and Cetearyl Glucoside and Cetearyl Alcohol | 6,57 |
| | Colophane claire type Y | Rosin | 1,89 |
| | Ceraube T1* | Carnauba(Copernica Cerifera)Wax | 1,86 |
| | Cerabeil blanche 1* | Beeswax | 5.31 |
| | Undecane (and) Tridecane | | 15.57 |
| | CETIOL® 4 All | Dipropylheptylcarbonate | 21,71 |
| | Crodamol ML (Croda) | Myristyl Lactate | 1,13 |
| | ELESTAB®366 | | 0.43 |
| II. | Castor oil | Castor Oil | 35.00 |
| III. | IRWINOL® LS 9319 | African wild mango butter | 3.00 |
| * erhältlich von Lambert- Riviere (France) | | | |

Herstellung: Phase I wurde bei 85°C geschmolzen, Phase II wurde zugefügt und die Temperatur auf 80°C gehalten. Phase III wurde kurz vor dem Einfüllen in die Gießform (welche mit Dimethicon 50 cts befeuchtet und auf 40 °C vorgeheizt war) zugegeben. Die Masse wurde in die Gießform gegeben und auf 40 °C abgekühlt. Die Gießformen wurde im Gefrierschrank auf nahe 0 °C abgekühlt.

W/O Creme

| Phase | Komponente / Handelsname | INCI | Gew.-% |
|---|---|---|---|
| I. | MONOMULS® 90 O 18 | Glyceryl Oleate | 2,00 |
| | LAMEFORM® TGI | Polyglyceryl 3 Diisostearate | 4,00 |
| | CETIOL® A | Hexyl Laurate | 12,00 |
| | Dipropylheptylcarbonate | | 12,00 |

(fortgesetzt)

| Phase | Komponente / Handelsname | INCI | Gew.-% |
|---|---|---|---|
| | SIPOL® C 16/18 OR | Cetearyl Alcohol | 1,00 |
| | Zinc stearate | Zinc Stearate | 2,00 |
| | Zinc Oxide | CI 77947 (or) Zinc Oxide | 15,00 |
| | Magnesium Sulphate | Magnesium Sulphate | 1,00 |
| | Glycerin | Glycerin | 3,00 |
| | Konservierungsmittel | | q.s. |
| | Benzyl Alcohol | Benzyl Alcohol | 0,40 |
| | HYDAGEN® B | Bisabolol | 0,50 |
| | Wasser | aqua | 100,00 |

Die ersten 7 Komponenten wurden bei 85°C geschmolzen. Magnesium Sulfate und Glycerin wurden im Wasser gelöst und diese Mischung wurde auf 85 °C erhitzt. Diese wässrige Phase wurde zur Ölphase gegeben und dispergiert. Unter kontinuierlichem Rühren wurde bis auf 40°C abgekühlt und dann wurden Benzyl Alkohol und Hydagen B gemischt und zu der Emulsion gegeben. Unter weiterem Rühren wurde bis auf 30°C abgekühlt und homogenisiert.

Tabelle 1: O/W-Sonnenschutzemulsionen

| Die im Folgenden genannten Beispiele enthalten alle das gemäß Herstellbeispiel 1 erhaltene Pentadecan oder Heptadecan Gemisch. Alle Angaben sind in Gew.-%. | | | | | | | | | | | |
|---|---|---|---|---|---|---|---|---|---|---|---|
| Komponente | 1 | 2 | 3 | 4 | 5 | 6 | 7 | 8 | 9 | 10 | 11 |
| L = Lotion, C = Creme, | L | C | S | L | C | L | L | C | L | C | L |
| Eumulgin® VL 75 | | | | | | 4 | 4 | 2 | | | |
| Eumulgin® B2 | 2 | | | | | | | | | | |
| Tween® 60 | | | | 1 | | | | | | | |
| Myrj® 51 | | 3 | | 2 | | | | | | | |
| Cutina® E 24 | 1 | | | 1 | | | | | | | |
| Hostaphat® KL 340 N | | | | | | | | | 2 | | |
| Lanette® E | | | 0.5 | | | | | 0.5 | | | |
| Amphisol® K | | | 1 | | | 1 | | 0.5 | | 1 | |
| Natriumstearat | | | | | | | 1 | | | | 2 |
| Emulgade® PL 68/50 | | | 1 | | 5 | | | | | 4 | |
| Tego® Care 450 | | | | | | | | | | 3 | |
| Cutina® MD | 2 | | | 6 | | | 4 | | | 6 | |
| Lanette® 14 | 1 | | | 1 | | | | 2 | | | 4 |
| Lanette®O | 1 | 6 | | | 5 | 2 | | 2 | | | |
| Antaron V 216 | | | 1 | | 2 | 2 | | | | 1 | |
| Lanolin, wasserfrei USP | | | | | | | 5 | | | | |
| Dipropylheptylcarbonate | 2 | 2 | 4 | 1 | 2 | 2 | 2 | 1 | 2 | 2 | 1 |
| Myritol® PC | | | | | 5 | | | | | | |
| Myritol® 331 | 5 | | 8 | | | 6 | | 10 | | 2 | |
| Finsolv® TN | | | 1 | | | | | 1 | 8 | | |

(fortgesetzt)

| Komponente | 1 | 2 | 3 | 4 | 5 | 6 | 7 | 8 | 9 | 10 | 11 |
|---|---|---|---|---|---|---|---|---|---|---|---|
| Cetiol® CC | | 2 | 5 | | | 4 | 4 | 2 | | 2 | |
| Cetiol® OE | | | 3 | | | | | | 2 | 3 | |
| Dow Corning DC® 244 | 4 | | 1 | | 5 | | | 2 | | | 2 |
| Dow Corning DC® 2502 | | 1 | | | 2 | | | | | | |
| Silikonöl Wacker AK® 350 | | 2 | | | | | | | | | |
| Cetiol® 868 | | | | | 2 | | 8 | | | | 7 |
| Cetiol® J 600 | | | | | 3 | 2 | | | | 5 | |
| Mineralöl | | | | 9 | | | | | | | |
| Cetiol® B | | | 1 | | | | | | | 2 | |
| Eutanol® G | | | | | | | | | | | |
| Eutanol® G 16 | | | | | | | | | | | |
| Cetiol® PGL | | 5 | | | | | | | | 5 | |
| Mandelöl | | | 2 | | | | 1 | | | | |
| Photonyl® LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl® LS | 1 | | | | | | | | | | |
| Neo Heliopan® Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan® 303 | 3 | 5 | 9 | 4 | | | | | | | |
| Neo Heliopan® BB | | | | | 1 | | | | | | 2 |
| Neo Heliopan® MBC | 2 | | | 3 | | 2 | 2 | 2 | | | 1 |
| Neo Heliopan® OS | | | | | | | | | 10 | 7 | |
| Neo Heliopan® E 1000 | | 7.5 | | 6 | | | | | | | 6 |
| Neo Heliopan® AV | | | 7.5 | | | 7.5 | 4 | 5 | | | |
| Uvinul® T 150 | 2 | | | | 2.5 | | | 1 | | | |
| Parsol® 1789 | | 1 | 1 | | | | 2 | | 2 | 2 | |
| Zinkoxid NDM | 10 | | 5 | | | 10 | | 3 | | 5 | 4 |
| Eusolex® T 2000 | 1 | 2 | 4 | 1 | 5 | 1 | 3 | 3 | 2 | 2 | 4 |
| Veegum® Ultra | | | 0.75 | | | | | 1 | 1 | | |
| Keltrol® T | | | 0.25 | | | | | 0.5 | 0.5 | | |
| Carbopol® 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |

(fortgesetzt)

| Komponente | 1 | 2 | 3 | 4 | 5 | 6 | 7 | 8 | 9 | 10 | 11 |
|---|---|---|---|---|---|---|---|---|---|---|---|
| Konservierungsmittel, NaOH | q.s. | | | | | | | | | | |
| Wasser | Ad 100 | | | | | | | | | | |

Tabelle 2: O/W-Sonnenschutzemulsionen

| Komponente | 12 | 13 | 14 | 15 | 16 | 17 | 18 | 19 | 20 | 21 | 22 |
|---|---|---|---|---|---|---|---|---|---|---|---|
| L = Lotion, C = Creme, | L | | | C | L | C | S | C | C | L | L |
| Eumulgin® VL 75 | 4 | 3 | 4.5 | | 3 | | | | 4 | | |
| Eumulgin® B2 | | | | | | | | | | 1 | |
| Tween® 60 | | | | | | | | | | 1 | |
| Cutina® E 24 | | | | 2 | | | | | | | |
| Hostaphat® KL 340 N | | | | | | | | | | 0.5 | |
| Lanette® E | 0.5 | | 0.5 | 0.5 | | | 0.1 | | 0.5 | | |
| Amphisol® K | 0.5 | | | | | 1 | 1 | 1 | | | |
| Natriumstearat | | | | | 1 | | | | | | |
| Emulgade® PL 68/50 | | 6 | | | | 4.5 | 1 | 5 | | | |
| Tego® Care 450 | 1 | | | | | | | | 4 | | |
| Cutina® MD | 1 | | | 8 | 6 | 1 | | | | 4 | 1 |
| Lanette® 14 | | 2 | | | | | | 2 | | 1 | |
| Lanette® O | | | | 2 | | | | | 1 | 1 | |
| Antaron V 220 | 1 | | | 2 | | | 0.5 | | | 2 | 0.5 |
| Dipropylheptylcarbonate | 4 | 2 | 4 | 6 | 10 | 4 | 2 | 8 | 2 | 1 | 3 |
| Myritol® PC | | | | | | | | | 5 | | |
| Myritol® 331 | 12 | | 12 | | | 8 | 8 | | | 10 | 8 |
| Finsolv® TN | | | | | 5 | | | 3 | 3 | | |
| Cetiol® CC | 6 | | 6 | | | 5 | 5 | | | | |
| Cetiol® OE | | | | | 2 | | | | | | 2 |
| Dow Corning DC® 244 | | 2 | | | 1 | | | | | | |
| Dow Corning DC® 2502 | | 1 | | | 1 | | | | | | |
| Ceraphyl® 45 | | | | | | | | | | 2 | 2 |
| Silikonöl Wacker AK® 350 | | | | | 1 | | | | | | |
| Cetiol® 868 | | 2 | | | | | | | | | |
| Cetiol® J 600 | | 2 | | | | | | | | | |
| Cetiol® B | 4 | | 4 | | | | | 4 | | | |
| Eutanol® G | | 3 | | 5 | | 3 | | | | | |
| Eutanol® G 16 S | 10 | | | | | | | | | | |
| Cetiol® PGL | | | | 5 | | | | | 2 | | |
| Photonyl® LS | | | | | | | | | | 2 | |

(fortgesetzt)

| Komponente | 12 | 13 | 14 | 15 | 16 | 17 | 18 | 19 | 20 | 21 | 22 |
|---|---|---|---|---|---|---|---|---|---|---|---|
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan® Hydro (Na-Salz) | | | | | | | | | | 3 | |
| Neo Heliopan AP (Na-Salz) | | 2 | | 2 | | | 2 | | | | 1 |
| Eusolex® OCR | 6 | | 9 | | 5 | 7 | 9 | | 4 | | 7 |
| Neo Heliopan® BB | | | | | | | | 1 | 1 | | 1 |
| Neo Heliopan® MBC | | 2 | | 1 | | | | 3 | 1 | | 3 |
| Neo Heliopan® OS | 2 | | | | | | | | 7 | | |
| Neo Heliopan® E1000 | | 4 | | | | | | 5 | | | |
| Neo Heliopan® AV | | 4 | 7.5 | 5 | | | | 5 | 4 | 7.5 | |
| Uvinul® T 150 | 1 | | | | | | | | 1.3 | 1 | 1 |
| Parsol® 1789 | 1 | | | | | | | | 2 | | 1 |
| Z-Cote® HP 1 | 7 | 2 | 5 | 2 | | 7 | 5 | | 6 | | 2 |
| Eusolex® T 2000 | 5 | 2 | | | 10 | | | 10 | | 2 | |
| Veegum® Ultra | 1.5 | | 1.5 | | | 1.5 | 1.2 | | 1 | | |
| Keltrol® T | 0.5 | | 0.5 | | | 0.5 | 0.4 | | 0.5 | | |
| Pemulen® TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

Tabelle 3: W/O-Sonnenschutzemulsionen

| Komponente | 23 | 24 | 25 | 26 | 27 | 28 | 29 | 30 | 31 | 32 | 33 |
|---|---|---|---|---|---|---|---|---|---|---|---|
| L = Lotion; C = Creme | C | L | C | L | C | L | L | L | L | C | C |
| Dehymuls® PGPH | 4 | 2 | 1 | 3 | 3 | 1 | 1 | 2 | 2 | 4 | 1 |
| Monomuls® 90-018 | | | 2 | | | | | | | | |
| Lameform® TGI | 2 | | 4 | | 3 | | | | | 1 | 3 |
| Abil® EM 90 | | | | | | | 4 | | | | |
| Glucate® DO | | | | | | | | | | | 3 |
| Isolan® PDI | | | | | | 4 | | 2 | | | |
| Arlacel® 83 | | | | 2 | | | | | | | |
| Elfacos® ST9 | | | | | | | | | 2 | | |
| Elfacos® ST37 | | | | | | | | | | | |
| Arlacel® P 135 | | 2 | | | | | | | | | |
| Dehymuls® HRE 7 | | | | | | | | | | | |

(fortgesetzt)

| Komponente | 23 | 24 | 25 | 26 | 27 | 28 | 29 | 30 | 31 | 32 | 33 |
|---|---|---|---|---|---|---|---|---|---|---|---|
| Zinkstearat | 1 | | | 1 | 1 | | | 1 | | 1 | |
| Bienenwachs | 1 | | 2 | 1 | | 1 | | 3 | | 2 | 3 |
| Tego® Care CG | | | | | 1 | | | | | | .5 |
| Prisorine® 3505 | 1 | | 1 | 1 | | 1 | 1 | | | | 1 |
| Wollwachsalkohol, wasserfrei, USP | | | | | | | | | | | 1 |
| Antaron V 216 | 2 | | | | | | | | | | |
| Dipropylheptylcarbonate | 3 | 4 | 2 | 1 | 10 | 2 | 2 | 6 | 3 | 12 | 1 |
| Myritol® PC | | | | | 3 | | | 4 | | | |
| Myritol® 331 | 10 | | | | 3 | 6 | | | | | 8 |
| Finsolv® TN | | | | 5 | | | 5 | | | | |
| Cetiol® CC | 12 | 22 | | | | 2 | | | 2 | | 5 |
| Cetiol® OE | | | | | 4 | | 5 | | 4 | 2 | |
| Dow Corning DC® 244 | | | | | | | 2 | | | | |
| Dow Corning DC® 2502 | | | 1 | | 2 | | | | | | |
| Prisorine® 3758 | | | | | | | | | | 2 | |
| Silikonöl Wacker AK® 350 | | | | 4 | | | | 3 | | | |
| Cetiol® 868 | | | | | | | | | | 2 | |
| Eutanol® G 16 | | 3 | | | | | | | | | |
| Eutanol® G 16S | | | | | | | | | | | |
| Cetiol® J 600 | | | 4 | | | 2 | | | | | |
| Ceraphyl® 45 | | | | 2 | | | | 2 | | 6 | |
| Cetiol® B | | | 2 | 4 | | | | | | 3 | |
| Eutanol® G | | | 3 | | 2 | | | 8 | | | |
| Cetiol® PGL | | 11 | | | 2 | 4 | | | 9 | | |
| Mandelöl | | | | | 1 | | 5 | | | | |
| Photonyl® LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1,0 | | | | | | | | | | |
| Magnesiumsulfat x 7 Wasser | 1 | | | | | | | | | | |
| Neo Heliopan® Hydro (Na-Salz) | | 2 | | 3 | | | | 2 | | | |
| Neo Heliopan AP (Na-Salz) | 2 | 1 | | | 2 | | | 1 | 2 | | 1 |
| Neo Heliopan® 303 | | | | | 4 | | | | | 6 | |
| Neo Heliopan® BB | | 4 | 2 | | | | 2 | | | | |
| Neo Heliopan® MBC | | | | | | | | 4 | | 3 | |
| Neo Heliopan® OS | | | | | | | | | | | |
| Neo Heliopan® E 1000 | | | | | | | | | 5 | | |
| Neo Heliopan® AV | | 3 | 6 | 6 | | 7.5 | 7.5 | | 5 | | 7.5 |

(fortgesetzt)

| Komponente | 23 | 24 | 25 | 26 | 27 | 28 | 29 | 30 | 31 | 32 | 33 |
|---|---|---|---|---|---|---|---|---|---|---|---|
| Uvinul® T 150 | | | | | 2.5 | | | 1 | | 2 | |
| Parsol® 1789 | | 2 | | | | | | 1 | | 2 | |
| Zinkoxid NDM | | 4 | | 2 | | 6 | | 2 | | 2 | |
| Eusolex® T 2000 | 15 | | 10 | | 5 | | 4 | | 4 | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 1 | 100, | q.s. | | | | | | | | |

Tabelle 4: W/O-Sonnenschutzemulsionen

| Komponente | 34 | 35 | 36 | 37 | 38 | 39 | 40 | 41 | 42 | 43 | 44 |
|---|---|---|---|---|---|---|---|---|---|---|---|
| L = Lotion; C = Creme | L | C | L | L | C | L | L | L | L | C | C |
| Dehymuls® PGPH | 3 | 1 | 5 | 1 | 1 | 3 | 2 | 4 | 0.5 | 1 | 4 |
| Monomuls® 90-018 | | 1 | | | | | | | | | |
| Lameform® TGI | | | | | 4 | | | 1 | | 3 | 1 |
| Abil® EM 90 | | | | 1 | | | | | | 2 | |
| Glucate® DO | | | | 3 | | | | | 2 | | |
| Isolan® PDI | | 3 | | | | | 4 | | | | |
| Arlacel® 83 | | | | | | 3 | | | | | |
| Elfacos® ST9 | | | | | | | | | | | 2 |
| Elfacos® ST37 | 2 | | | | | | | | | | |
| Arlacel® P 135 | | | | | | 3 | | | | | |
| Dehymuls® HRE 7 | | | | | | | | | 4 | | |
| Zinkstearat | | 2 | 2 | 1 | 1 | | | 1 | 1 | | |
| Bienenwachs | | 1 | | 2 | 2 | | 3 | | 2 | 1 | 1 |
| Tego® Care CG | | | | | | | | | | | |
| Isostearinsäure | 1 | 1 | | | | | 1 | 1 | | 1 | 1 |
| Wollwachsalkohol, wasserfrei | | | | | | | | | | | |
| Antaron V 220 | | 0.5 | 2 | 1 | 1 | 1 | | | | | |
| Dipropylheptylcarbonate | 2 | 4 | 3 | 3 | 2 | 2 | 1 | 3 | 3 | 1 | 4 |
| Myritol® PC | | | | | | | | | | | |
| Myritol® 331 | 4 | 2 | 3 | | 5 | | | 8 | 5 | 4 | |
| Finsolv® TN | | 5 | 5 | | | 7 | | | | | |
| Cetiol® CC | 3 | 1 | | | | | 3 | 16 | | | 12 |
| Cetiol® OE | | 3 | | 2 | | | 3 | | | | |
| Dow Corning DC® 244 | | 4 | | 2 | | | | | | | |
| Dow Corning DC® 2502 | | | | 1 | | | | | | | |

(fortgesetzt)

| Komponente | 34 | 35 | 36 | 37 | 38 | 39 | 40 | 41 | 42 | 43 | 44 |
|---|---|---|---|---|---|---|---|---|---|---|---|
| Prisorine® 3578 | | 1 | | | | | | | | | |
| Silikonöl Wacker AK® 350 | | | | 1 | | | | | | | |
| Cetiol® 868 | | | | | | | | | | | |
| Eutanol® G 16 | | | | | | | | | | | 3 |
| Eutanol® G 16S | | | | | | | | | | | 7 |
| Cetiol® J 600 | | | | 3 | | | | | | | |
| Ceraphyl® 45 | | | | 1 | | | | | 5 | 4 | |
| Cetiol® B | | | | | 3 | 3 | | 2 | 2 | | |
| Eutanol® G | | | | 2 | | | 4 | | 5 | | |
| Cetiol® PGL | | | | | | | 5 | 2 | | | |
| Mandelöl | | | 2 | | | | | | | | |
| Photonyl® LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan® Hydro (Na-Salz) | | 4 | | | | | | 4 | | | |
| Neo Heliopan AP (Na-Salz) | 2 | | | 1 | 2 | | 1 | | | | |
| Neo Heliopan® 303 | 6 | 2 | | | | | | | 6 | | |
| Neo Heliopan® BB | | 2 | | 2 | | 2 | | | | | |
| Neo Heliopan® MBC | 2 | | | | 3 | | 4 | | 2 | | |
| Neo Heliopan® OS | | | | | 10 | | 8 | | | | |
| Neo Heliopan® E 1000 | | | 5 | 6 | | | | | | 5 | |
| Neo Heliopan® AV | | 5 | 5 | | | 7.5 | | | | 5 | |
| Uvinul® T 150 | 1 | | | 2 | 2 | | | | 3 | 2 | |
| Parsol® 1789 | | 1 | 1 | | | | 1 | | 0.5 | | |
| Z-Cote® HP 1 | 4 | 10 | | | 2 | | 2 | 5 | | | 5 |
| Titandioxid T 805 | | | 10 | 2 | | 3 | | 7 | 2 | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser, Konservierungsmittel | ad 100, q.s. | | | | | | | | | | |

Tabelle 5: W/O-Pflegeemulsionen

| Komponente | 45 | 46 | 47 | 48 | 49 | 50 | 51 | 52 | 53 | 54 | 55 |
|---|---|---|---|---|---|---|---|---|---|---|---|
| L = Lotion. C = Creme | C | L | C | L | C | L | L | L | C | C | C |
| Dehymuls® PGPH | 1 | 3 | 1 | 2 | 3 | 1 | 1 | 2 | 1 | 1 | 1 |

(fortgesetzt)

| Komponente | 45 | 46 | 47 | 48 | 49 | 50 | 51 | 52 | 53 | 54 | 55 |
|---|---|---|---|---|---|---|---|---|---|---|---|
| Monomuls® 90-018 | 2 | | | | | | | | 2 | | 2 |
| Lameform® TGI | 4 | 1 | | | 3 | | | 1 | 4 | 3 | 3 |
| Abil® EM 90 | | | | | | | 4 | | | | |
| Isolan® PDI | | | | | | 4 | | | | | |
| Glucate® DO | | | | 5 | | | | | | | |
| Arlacel® 83 | | | 5 | | | | | | | | |
| Dehymuls® FCE | | | | | | | | | | | |
| Dehymuls® HRE 7 | | | | | | | | 4 | | 1 | |
| Zinkstearat | 2 | 1 | | 1 | 1 | | | 1 | 1 | 1 | |
| Bienenwachs | 2 | | 3 | 1 | | 1 | | 1 | 3 | 2 | 1 |
| Tego Care® CG | | | | | 1 | | | | | | 0.5 |
| Prisorine® 3505 | | | 1 | 1 | | 1 | 1 | | | | 1 |
| Dry Flo® Plus | 5 | 2 | | 5 | 2 | 3 | 1 | | 2 | 5 | 1 |
| SFE 839 | | | 5 | | | | 3 | 5 | | | |
| Lanolin; anhydrous USP | | | 5 | | | | | | | 4 | |
| Dipropylheptylcarbonate | 3 | 4 | 2 | 12 | 10 | 2 | 2 | 6 | 3 | 12 | 1 |
| Cegesoft® C 17 | | | 3 | | | | | | | 1 | |
| Myritol® PC | | | | | | 2 | | 4 | | | |
| Myritol® 331 | 6 | | | | 2 | 6 | 2 | | | | |
| Finsolv® TN | | | | 5 | | 2 | 5 | | | | |
| Cetiol® A | | 6 | | | | 4 | | | | | |
| Cetiol® CC | | 8 | | | 2 | 2 | 2 | | | | 5 |
| Cetiol® SN | | 5 | | | | | | 3 | | | |
| Cetiol® OE | 3 | | | | 4 | | 2 | | 4 | 2 | 8 |
| Dow Corning DC® 244 | | | | | 1 | | 2 | | | | |
| Dow Corning DC® 2502 | | | 1 | | 2 | | | | | | |
| Prisorine® 3758 | | | | | 3 | | | | | | |
| Silikonöl Wacker AK® 350 | | | | 4 | | | | 3 | | | |
| Komponente | 45 | 46 | 47 | 48 | 49 | 50 | 51 | 52 | 53 | 54 | 55 |
| Cetiol® 868 | | | | | | | | | | 2 | |
| Cetiol® J 600 | | | 4 | | | 2 | | | | | |
| Ceraphyl® 45 | | | | 2 | | | | 2 | | 6 | |
| Cetiol® B | | | 2 | 4 | | | | | | 3 | |
| Eutanol® G 16 | | 1 | | | 1 | | | | | 3 | 7 |
| Eutanol® G | | | 3 | | 1 | | | 8 | | | |
| Cetiol® PGL | | | | | | 4 | | | 9 | | |
| Mandelöl | | | | | 3 | | 5 | | | | |
| Insect Repellent® 3535 | 2 | | | | | | | | | | |

(fortgesetzt)

| Komponente | 45 | 46 | 47 | 48 | 49 | 50 | 51 | 52 | 53 | 54 | 55 |
|---|---|---|---|---|---|---|---|---|---|---|---|
| N,N-Diethyl-m-toluamid | | | | 3 | | | | 5 | | | |
| Photonyl® LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone® 38 | | | | | 1 | | | | | | |
| Aluminum chlorohydrate | | 8 | | | | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

Tabelle 6: W/O-Pflegeemulsionen

| Komponente | 56 | 57 | 58 | 59 | 60 | 61 | 62 | 63 | 64 | 65 | 66 |
|---|---|---|---|---|---|---|---|---|---|---|---|
| L = Lotion. C = Creme | L | C | L | L | C | L | L | L | L | C | C |
| Dehymuls® PGPH | 3 | 1 | 5 | 1 | 1 | 3 | 3 | 4 | 1 | 1 | 1 |
| Monomuls® 90-018 | | 1 | | 1 | | | | | | | |
| Lameform® TGI | | | | | 4 | | | 1 | | 3 | |
| Abil® EM 90 | | | | 3 | | | | | | 2 | |
| Isolan® PDI | | 3 | | | | | | | | | 4 |
| Glucate® DO | 1 | | | | | | | | | | |
| Arlacel® 83 | | | | | | 3 | | | | | |
| Dehymuls® FCE | | | | | 4 | | 1 | | | | |
| Dehymuls® HRE 7 | | | | | | | | | 7 | | |
| Zinkstearat | | 2 | 2 | 1 | 1 | 1 | | 1 | 1 | | 1 |
| Bienenwachs | | 4 | | 2 | 1 | 2 | 2 | 1 | 2 | 3 | 5 |
| Tego® Care CG | | | | | | | | | | | |
| Prisorine® 3505 | 1 | 1 | | | | | 1 | 1 | | 1 | 1 |
| Dry Flo® Plus | 1 | 1 | 1 | 1 | 2 | 2 | 1 | 1 | 2 | 2 | 1 |
| SFE® 839 | | 5 | | | | 4 | | | | | |
| Emery® 1780 | | | | | | | | | | | |
| Lanolin anhydrous USP | | 7 | 3 | | | | | | | | |
| Dipropylheptylcarbonate | 3 | 4 | 4 | 8 | 10 | 2 | 8 | 6 | 3 | 12 | 7 |
| Cegesoft® C 17 | | 2 | | | | | | | | | |
| Myritol® PC | | | | 8 | | | | | | | |

(fortgesetzt)

| Komponente | 56 | 57 | 58 | 59 | 60 | 61 | 62 | 63 | 64 | 65 | 66 |
|---|---|---|---|---|---|---|---|---|---|---|---|
| Myritol® 331 | 4 | | 3 | | 5 | 3 | | | 5 | 4 | |
| Finsolv® TN | | | 5 | | | 7 | | | | | |
| Cetiol® A | | | | | | | | 6 | | | |
| Cetiol® CC | 3 | | | 6 | | 3 | 3 | | | 8 | |
| Cetiol® SN | | | | | 5 | | | | | | |
| Cetiol® OE | | 3 | | 2 | | | 3 | | | | 8 |
| Dow Corning ® DC 244 | | 4 | | 2 | | 2 | | | | | |
| Dow Corning® DC 2502 | | | | 1 | | | | | | | |
| Prisorine® 3758 | | | | | | 1 | | | | | |
| Silikonöl Wacker AK® 350 | | | | 1 | | 1 | | 4 | | | |
| Cetiol® 868 | | | | | | | | | | | 10 |
| Cetiol® J 600 | 4 | | | 3 | | | | | | | |
| Ceraphyl® 45 | | | | 1 | | | | | 5 | 4 | |
| Cetiol® B | | | | | 3 | 3 | | 2 | 2 | | |
| Eutanol® G 16 | 1 | | | | | | 3 | | | | |
| Eutanol® G | | | | 2 | | | 2 | | 5 | | |
| Cetiol® PGL | | | 10 | | | | 4 | 6 | | | 3 |
| Mandelöl | | | 2 | | 5 | | 2 | | | | |
| Photonyl® LS | | | | 2 | | | | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Bentone® 38 | | | | | | 1 | | | | | |
| Propylencarbonat | | | | | | 0.5 | | | | | |
| Ethanol | | | | 8 | | 10 | | | | | |
| Butylenglykol | 5 | 1 | | 3 | 3 | | | | 8 | 2 | 1 |
| Glycerin | | | 6 | 2 | | | 5 | 5 | | 3 | 5 |
| Wasser, Konservierungsmittel | ad 100, q.s. | | | | | | | | | | |

Tabelle 7: O/W-Pflegeemulsionen

| Komponente | 67 | 68 | 69 | 70 | 71 | 72 | 73 | 74 | 75 | 76 | 77 |
|---|---|---|---|---|---|---|---|---|---|---|---|
| L = Lotion, C = Creme | C | C | C | L | C | L | L | C | L | C | C |
| Eumulgin® VL 75 | | | | | | 4 | | | | | |
| Dehymuls® PGPH | | 2 | | | | | | | | | |
| Generol® R | | | 1 | | | | | | | | |
| Eumulgin® B2 | | | 0.8 | | | | | | | | |

(fortgesetzt)

| Komponente | 67 | 68 | 69 | 70 | 71 | 72 | 73 | 74 | 75 | 76 | 77 |
|---|---|---|---|---|---|---|---|---|---|---|---|
| Tween® 60 | | | | 1 | | | | | | | |
| Cutina® E 24 | | | 0.6 | 2 | | | | | | | |
| Hostaphat® KL 340 N | | | | | | | | | 2 | | |
| Lanette® E | | | | | | | | 1 | | | |
| Amphisol® K | | 0.5 | | | | 1 | | | | 1 | 0.5 |
| Natriumstearat | | | | | 0.5 | | | | | | |
| Emulgade® PL 68/50 | | 2.5 | | | | | | | | 4 | |
| Tego® Care CG | | | | | | | | | | | 2 |
| Tego® Care 450 | | | | | | | | 5 | | | |
| Cutina® MD | | 1 | | 6 | 5 | | 4 | | | 6 | |
| Lanette® 14 | | | | 1 | | | | 2 | | | 4 |
| Lanette® O | 4.5 | | 4 | | 1 | 2 | | | | | 2 |
| Novata® AB | | 1 | | | | | | | | | 1 |
| Lanolin, wasserfrei, USP | | | | | | | 5 | | | | |
| Cetiol® SB 45 | | | 1.5 | | | | 2 | | | | |
| Dipropylheptylcarbonate | 3 | 4 | 2 | 1 | 10 | 2 | 2 | 6 | 3 | 12 | 1 |
| Cegesoft® C 17 | | | | | | | | | | | |
| Myritol® PC | | | | | 5 | | | | | | |
| Myritol® 331 | 2 | 5 | | | | 6 | | 12 | | | |
| Finsolv® TN | | | | | | 2 | | | 8 | | |
| Cetiol® CC | 4 | 6 | | | | 4 | 4 | | | | 5 |
| Cetiol® OE | | 7 | 7 | | | | | | 4 | 3 | |
| Dow Corning DC® 245 | | | 2 | | 5 | 1 | | | | | |
| Dow Corning DC® 2502 | | | | | 2 | 1 | | | | | |
| Prisorine® 3758 | | | | | | 1 | | | | | |
| Silikonöl Wacker AK® 350 | 0.5 | 0.5 | 0.5 | | | 1 | 4 | | | | |
| Cetiol® 868 | | | | | 2 | | 4 | | | | |
| Cetiol® J 600 | 2 | 3 | 3 | | 3 | 2 | | | | 5 | |
| Ceraphyl® 45 | | | | | | | 3 | | | | |
| Cetiol® SN | | | 5 | | | | | | | | |
| Cetiol® B | | | | | | | | | | 2 | |
| Eutanol® G | | 2 | | 5 | | | | | | | |
| Cetiol® PGL | | | | 7 | | | | | 5 | 5 | |
| Dry Flo ® Plus | 5 | 3 | 2 | 1 | 2 | 2 | 1 | 2 | 1 | 1 | 1 |
| SFE 839 | 5 | | | | | | | | | | 2 |
| Mandelöl | | | | | | | 1 | | 5 | | |
| Insect Repellent® 3535 | | 2 | 4 | | | 2 | | | | 3 | |
| N, N-Diethyl-m-toluamid | | 2 | | | | | | | | 3 | |

(fortgesetzt)

| Komponente | 67 | 68 | 69 | 70 | 71 | 72 | 73 | 74 | 75 | 76 | 77 |
|---|---|---|---|---|---|---|---|---|---|---|---|
| Photonyl® LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum® ultra | | | | | | | | | 1 | | |
| Keltrol® T | | | 0.4 | | | | | | 0.5 | | |
| Pemulen® TR 2 | 0.3 | | | | | | | 0.3 | | | |
| Carbopol® Ultrez 10 | | | 0.3 | 0.2 | | 0.2 | | | 0.1 | 0.3 | |
| Cosmedia SP | | 0,3 | | | 0.2 | | | | | | 0.2 |
| Aluminum Chlorohydrate | | | 7 | | | | | | | | |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s.,pH 6,5 - 7,5 | | | | | | | | | | |

Tabelle 8: O/W-Pflegeemulsionen

| Komponente | 78 | 79 | 80 | 81 | 82 | 83 | 84 | 85 | 86 | 87 | 88 |
|---|---|---|---|---|---|---|---|---|---|---|---|
| L = Lotion, C = Creme | C | C | L | C | L | C | L | L | L | L | C |
| Eumulgin® VL 75 | 4 | 3 | | | | | 1 | | | | 2 |
| Generol® R | | | | | | 2 | | | | | |
| Eumulgin® B2 | | | | | | 2 | | | | 1 | |
| Tween® 60 | | | | | | | | | | 1 | |
| Cutina® E 24 | | | | 2 | | | | | | | |
| Hostaphat® KL 340 N | | | | | | | | | | | |
| Lanette® E | 0.5 | | | | | | | | | | 1 |
| Amphisol® K | 0.5 | 1 | | | | | | 1 | 1 | | |
| Natriumstearat | | | | | 1 | | | | | | |
| Emulgade® PL 68/50 | | 6 | | | | | | 5 | | | 4 |
| Tego® Care CG | | | | | | | | | | | |
| Tego® Care 450 | | | | | | | | | 4 | | |
| Cutina® MD | 3 | | 3 | 8 | 6 | 8 | | | | 4 | |
| Lanette® 14 | | 2 | | | | | | 2 | | 1 | |
| Lanette® O | 2 | | | 2 | | 3 | 1 | | 1 | 1 | 6 |
| Novata® AB | | | | | | | | | | | |
| Lanolin, wasserfrei, USP | | | | | | 4 | | | | | |
| Cetiol® SB 45 | | | | | | | 2 | | | | |
| Dipropylheptylcarbonate | 3 | 4 | 2 | 1 | 10 | 2 | 2 | 6 | 3 | 12 | 1 |

(fortgesetzt)

| Komponente | 78 | 79 | 80 | 81 | 82 | 83 | 84 | 85 | 86 | 87 | 88 |
|---|---|---|---|---|---|---|---|---|---|---|---|
| Cegesoft® C 17 | 4 | | | | | | | | | | |
| Myritol® PC | 6 | | | | | 5 | | | 5 | | |
| Myritol® 331 | 5 | | 5 | | | | 7 | | | 10 | 3 |
| Finsolv® TN | | 5 | | | 5 | | | 3 | 3 | | 1 |
| Cetiol® CC | | | | | | | | | | | 2 |
| Cetiol® OE | | | | | 2 | | 2 | | 5 | | |
| Dow Corning DC® 245 | | 2 | | | 1 | | | | | 8 | 2 |
| Dow Corning DC® 2502 | | 1 | | | 1 | | | | | | 3 |
| Prisorine® 3758 | 3 | | | | | | | | | | 2 |
| Silikonöl Wacker AK® 350 | | | | | 1 | | | | | | 1 |
| Cetiol® 868 | | 2 | | | | | | | | | |
| Cetiol® J 600 | | 2 | | | | | | | | | |
| Ceraphyl® 45 | | | | | | | 3 | | | | |
| Cetiol® SN | | | | | | | | | | | |
| Cetiol® B | | | 5 | | | 5 | | 4 | | | 3 |
| Eutanol® G | | 3 | 5 | | 5 | | | | | | |
| Cetiol® PGL | | | | | | | | 5 | 2 | | |
| Dry Flo® Plus | | 1 | | | 2 | | 1 | 1 | | | 1 |
| Silicon Resin | 3 | | | 3 | | | | | 3 | | 1 |
| Nylon -12 | | 3 | 2 | | | 5 | | | | 2 | 1 |
| SFE 839 | 1 | 1 | | | 3 | | | | | | 1 |
| Mandelöl | | | | | 2 | | | | | | |
| Photonyl® LS | | | | | 2 | | | | | | |
| Panthenol | 1 | | | | | | | | | | |
| Bisabolol | 0.2 | | | | | | | | | | |
| Tocopherol / Tocopherylacetat | 1 | | | | | | | | | | |
| Colesterol | 0.1 | | | | | | | | | | |
| Ceramide 3 | 1 | | | | | | | | | | |
| Veegum® Ultra | | | | | | | | | 1 | | |
| Keltrol® T | | | | | | | | | 0.5 | | |
| Carbopol® ETD 2001 | | 0.3 | | 0.3 | | 0.5 | 0.2 | 0.2 | | | |
| Pemulen® TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

Tabelle 9: Sprayformulierungen

| Komponente | 89 | 90 | 91 | 92 | 93 | 94 | 95 | 96 | 97 | 98 | 99 |
|---|---|---|---|---|---|---|---|---|---|---|---|
| S = Körperspray, S* = Sonnenschutzspray | S | S | S | S | S | S* | S* | S* | S* | S* | S* |
| Emulgade® SE-PF | 8,9 | | 7,5 | 7.5 | 4,3 | 9.8 | 8.2 | 9.9 | | | |
| Eumulgin® B2 | 3,1 | | 3 | | | | | 4.2 | | | |
| Eumulgin® B3 | | | | | | 4.2 | 3.3 | | | | |
| Eumulgin® HRE 40 | | | | | 4,7 | | | | | | |
| Cutina® E 24 | | 5,9 | | 4 | | | | | | | |
| Amphisol® K | | | | | | | | | 1 | 1 | 1 |
| Eumulgin® VL 75 | | | | | | | | | | | 2 |
| Emulgade® PL 68/50 | | 0.5 | | | | | | | 2.5 | 1 | |
| Cutina® MD | | 3,1 | | | | | | | | | |
| Antaron V 220 | | | | | | 1 | 1 | 1 | | 1 | 1 |
| Dipropylheptylcarbonate | 11 | 5 | 7 | 7 | 7 | 5 | 4 | 5 | 5 | 4 | 6 |
| Myritol® PC | | | | | | | | | | | |
| Myritol® 331 | | | 3 | 4 | 3 | 3 | 3 | 3 | | | |
| Finsolv® TN | | 4 | | | | | | 8 | | | |
| Cetiol® CC | 6 | | | 5 | 5 | 2 | 2 | 4 | | | |
| Cetiol® OE | | 5 | 7 | | | 2 | | | | | |
| Dow Corning DC® 244 | | 4 | 4 | 5 | | | | | | | |
| Cetiol® 868 | 3 | | | | | | | | | | |
| Cetiol® J 600 | | | | 2 | 2 | | | | | | |
| Cetiol® B | | | | | | | 2 | | | | |
| Eutanol® G | 2 | | | | 1 | | | | | | |
| Photonyl® LS | 2 | | | | | | 2 | | | 2 | 2 |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan® Hydro (Na-Salz) | | | | | | 2 | | | | 3 | |
| Neo Heliopan AP (Na-Salz) | | | | | | 2 | 2 | 2 | | | 1 |
| Eusolex® OCR | | | | | | | 2 | | | | 3 |
| Neo Heliopan® BB | | | | | | | | | | 1 | |
| Neo Heliopan® MBC | | | | | | 2 | 2 | 2 | | 1 | 1 |
| Neo Heliopan® OS | | | | | | 5 | | | | | |
| Neo Heliopan® AV | | | | | | 6 | 6 | 2 | | 7.5 | 2 |
| Uvinul® T 150 | | | | | | 1 | 1 | 1 | | 1 | |
| Parsol® 1789 | | | | | | 1 | | 1 | | 1 | |
| Z-Cote® HP 1 | 1 | 2 | 2 | | | | | 1 | 1 | 2 | 2 |
| Eusolex® T 2000 | 1 | 1 | 1 | 2 | 2 | 3 | 3 | | | 2 | 2 |

(fortgesetzt)

| Komponente | 89 | 90 | 91 | 92 | 93 | 94 | 95 | 96 | 97 | 98 | 99 |
|---|---|---|---|---|---|---|---|---|---|---|---|
| Veegum® Ultra | | | | | | | | | | | 1.5 |
| Laponite®XLG | | | | | | | | | | 1.5 | |
| Keltrol® T | | | | | | | | | | | 0.5 |
| Pemulen® TR 2 | | | | | | | | | 0.2 | | |
| Insect Repellent® 3535 | 1 | | | | | | | | | | |
| N, N-Diethyl-m-toluamid | 1 | | | | | | | | | | |
| Ethanol | | | | | | | | | | | |
| Butylenglykol | | | | | | | 1 | | | 2 | 1 |
| Glycerin | | | | | | 3 | 2 | 3 | 2 | | 3 |
| Wasser/ Konservierungsmittel/ NaOH | ad 100/ q.s./ q.s | | | | | | | | | | |

Tabelle 10A: Antiperspirant Suspensionsstifte sowie Soft Solids - Mengenangaben in Gew.-%

| Zusammensetzung | 1 | 2 | 3 | 4 | 5 | 6 |
|---|---|---|---|---|---|---|
| Distearylether | - | 15 | - | - | - | - |
| Stearylalkohol | - | - | 15 | 10 | 14,7 | - |
| Guerbetalkohol C36 | 15 | - | - | - | - | - |
| Tribehenin | - | - | - | - | - | 20 |
| Hydrogenated Castor Oil | - | - | 4 | - | 3,7 | - |
| Dipropylheptylcarbonate | 30 | 60 | 51 | 60 | 31,6 | 55 |
| Octyldodecanol | 5 | - | - | - | - | - |
| Dicaprylylether | 5 | - | - | - | - | - |
| Hexyldecanol + Hexyldecyl Lau rate | - | 10 | - | - | - | - |
| Cyclomethicone | 20 | - | - | - | 30 | - |
| Dry Flo Plus* | - | - | 5 | - | - | - |
| Silica | - | - | - | 2,5 | - | - |
| Talc | - | - | - | 2,5 | - | - |
| Aluminium Zirconium Tetrachlorohydrex GLY | - | - | 25 | 25 | - | 25 |
| Aluminium Chlorohydrate | 25 | 15 | - | - | 20 | - |
| * National Starch | | | | | | |

Tabelle 10B: Antiperspirant Suspensionsstifte sowie Soft Solids - Mengenangaben in Gew.-%

| Zusammensetzung | 7 | 8 |
|---|---|---|
| 12-Hydroxystearinsäure | 10 | 5 |
| Dipropylheptylcarbonate | 65 | 65 |
| Dry Flo Plus* | - | 5 |
| Aluminium Zirconium Tetrachlorohydrex GLY | 25 | 25 |
| * National Starch | | |

Tabelle 11: Rezepturen 1 bis 13

| Komponenten INCI (Handelsname) | 1 | 2 | 3 | 4 | 5 | 6 | 7 | 8 | 9 | 10 | 11 | 12 | 13 |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade® SE) | | | | | | | | | | | | 10.7 | 5.1 |
| Ceteareth-20 (Eumulgin® B2) | | | | | | | | | | | | 5.8 | 3.4 |
| Cetearyl Glucoside, Cetearyl Alcohol (Emulgade® PL 68/50) | 1 | | 1 | 1 | | 2 | 2 | | 2 | | 2 | | |
| Polyglyceryl-2 Dipolyhydroxystearate, Lauryl Glucoside, Glycerin (Eumulgin® VL 75) | | 1 | | | 1 | | | 3 | | 2.5 | | | |
| Sodium Cetearyl Sulfate (Lanette® E) | 1 | 1 | 1 | | 1 | | | | 1 | 1 | | | |
| Dipropylheptylcarbonate | 5 | 4 | 8 | 3 | 5 | 8 | 4 | 2 | 4 | 3 | 5 | 10 | 2 |
| Dicaprylyl Carbonate (Cetiol® CC) | 5 | 5 | 5 | | | | | 4 | | 5 | 3 | 4 | |
| Cocoglycerides (Myritol® 331) | 3 | 4 | | 4 | 4 | | | | 5 | | | 3 | 3 |
| Dicaprylyl Ether (Cetiol® OE) | | | | | 5 | | 3 | | 2 | | | | |
| Dibutyl Adipate (Cetiol® B) | | | | 4 | | | | 4 | | 4 | | | |
| Hydrogenated Dimer Dilinoleyl/Dimethylcarbonate Copolymer (Cosmedia® DC) | 1 | 1 | 1 | 1 | 1 | 1.5 | 1.5 | 2 | 3 | 2 | 1.5 | 2 | 2 |
| Ethyl Butylacetylaminopropionate | | | | | | | | | | | 5 | 5 | |
| Tocopherol | 1 | 1 | 1 | 1 | 1 | 1 | 1 | 1 | 1 | 1 | 1 | 1 | 1 |
| Zinc oxide, nanonisiert, gecoated | 5 | 5 | 5 | 5 | 5 | 5 | 2 | | 5 | 3 | | | |
| Titanium dioxide, nanonisiert, | | | | 5 | 5 | | 2 | 3 | 5 | 2 | 5 | 3 | 2 |
| Gecoated | | | | | | | | | | | | | |
| Ethyl hexyl Methoxycinnamate | 7.5 | 7.5 | 7.5 | | | | 3 | 1 | 3 | 5 | | 5 | 5 |
| Octocrylene | 9 | 9 | 9 | | | 2 | 1 | | | | 2 | | 1.5 |
| Butyl Methoxydibenzoylmethane | | | | | | 2 | 2 | | | 1 | 2 | 2 | |
| 4-Methylbenzylidene Camphor | | | | | | | 2 | | | | | | 2 |
| Ethylhexyl Triazone | | | | | | 1 | 1 | 2 | | | 1 | | |
| Diethylhexyl Butamido Triazone | | | | | | 1 | 1 | 2 | | | 1 | 2 | |
| Phenylbenzimidazole Sulfonic Acid als Na-Salz, 15% wäßrige Lsg. | | | | | | | | | | | | | 13.3 |

(fortgesetzt)

| Komponenten INCI (Handelsname) | 1 | 2 | 3 | 4 | 5 | 6 | 7 | 8 | 9 | 10 | 11 | 12 | 13 |
|---|---|---|---|---|---|---|---|---|---|---|---|---|---|
| Glycerin | 3 | 3 | 3 | 3 | 3 | 3 | 3 | 3 | 3 | 3 | 3 | 5 | 5 |
| Magnesium aluminium silicate (and) cellulose Gum | 0.75 | 0.75 | | 0.5 | 0.5 | | 0.5 | | | 0.35 | | | |
| Xanthan Gum | 0.25 | 0.25 | | 0.5 | 0.5 | | 0.5 | | | 0.35 | | | |
| Sodium Polyacrylate (Cosmedia® SP) | | | 0.1 | | | 0.1 | 0.2 | | | | 0.1 | | |
| Acrylates/C10-30 Alkyl Acrylate Crosspolymer | | | | | | | | 0.2 | 0.1 | | | | |
| Wasser, Perfume, Preservatives | q.s. | q.s. | q.s. | q.s. | q.s. | q.s. | q.s. | q.s. | q.s. | q.s. | q.s. | q.s. | q.s. |

In der Tabelle 12 werden Sonnenschutzformulierungen vom Typ O/W beschrieben, in der Tabelle 13 werden Pflegeemulsionen beschrieben. Durch den Einsatz des erfindungsgemäßen Dipropylheptylcarbonats wird das sensorische Verhalten bei Applikation positiv beeinflusst. Die Mengenangaben beziehen sich jeweils auf Gew.-% der handelsüblichen Substanzen in der Gesamtzusammensetzung.

Tabelle 12: O/W-Sonnenschutzemulsionen

| Komponente | 12 | 13 | 14 | 15 | 16 | 17 | 18 | 19 | 20 | 21 | 22 |
|---|---|---|---|---|---|---|---|---|---|---|---|
| L = Lotion, C = Creme, S = Spray | L | C | L | C | L | C | S | C | C | L | L |
| Eumulgin® VL 75 | 2 | | | | 3 | | | | 1 | | |
| Eumulgin® B2 | | | | 2 | | | | | | 1 | |
| Tween® 60 | | | | | | | | | | 1 | |
| Cutina® E 24 | | | | 0.5 | | | | | | | |
| Hostaphat® KL 340 N | | | | | | | | | | | 0.5 |
| Eumulgin® SG | | | 0,5 | | | 0,5 | | 0,3 | 0,1 | | |
| Lanette® E | | | | | | | 0.1 | | 0.5 | | |
| Amphisol® K | 0.5 | | | | | | 1 | | | | |
| Natriumstearat | | | | | 1 | | | | | | |
| Emulgade® PL 68/50 | | 2 | 1 | | | 3 | | | | | |
| Tego® Care 450 | | 2 | | | | | | | 2 | | |
| Cutina® MD | | | | 2 | 1 | 3 | | | | | 1 |
| Lanette® 14 | | 1 | | | | | | | | | |
| Lanette® O | | | | 2 | | | | 2 | 1 | 1 | |
| Cutina® PES | 1 | 1 | | 2 | | | | | | 1 | |
| Allianz® OPT | 1 | | | 1 | 1 | | | 2 | | | 2 |
| Cosmedia® DC | | 1.5 | 2 | | | 1.5 | 2 | | 1.5 | 1.5 | |
| Lanolin, wasserfrei, USP | | | | | | 1 | 1 | | | | |
| Dipropylheptylcarbonate | 6 | 2 | 4 | 7 | 3 | 7 | 6 | 6 | 4 | 4 | 5 |
| Myritol® PC | | | | | | | | | 5 | | |
| Myritol® 331 | 6 | | 4 | | | 5 | 8 | | | 10 | 8 |
| Finsolv® TN | | | | | 5 | | | 3 | 3 | | |
| Cetiol® CC | 6 | | 6 | | | 5 | 5 | | | | |
| Cetiol® OE | | | | | 2 | | | | | | 2 |
| Dow Corning DC® 244 | | 2 | | | 1 | | | | | | |
| Dow Corning DC® 2502 | | 1 | | | 1 | | | 3 | | | |
| Ceraphyl® 45 | | | | | | | | | | 2 | 2 |
| Silikonöl Wacker AK® 350 | | | | | 1 | | | | | | |
| Cetiol® 868 | | 2 | | | | | | | | | |
| Cetiol® J 600 | | 2 | | | | | | | | | |
| Cetiol® B | 4 | | 4 | | | | | 4 | | | |
| Eutanol® G | | 3 | | | | 3 | | | | | |
| Eutanol® G 16 S | 3 | | | | | | | | | | |
| Cetiol® PGL | | | | | | | | | 2 | | |

(fortgesetzt)

| Komponente | 12 | 13 | 14 | 15 | 16 | 17 | 18 | 19 | 20 | 21 | 22 |
|---|---|---|---|---|---|---|---|---|---|---|---|
| Photonyl® LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan® Hydro (Na-Salz) | | | | | | | | | | 3 | |
| Eusolex® OCR | 6 | | 9 | | 5 | 7 | 9 | | 4 | | 7 |
| Neo Heliopan® AP (Na-Salz) | | | | 0.5 | | 1 | | | | | |
| Neo Heliopan® BB | | | | | | | | 1 | 1 | | 1 |
| Neo Heliopan® MBC | | 2 | | 1 | | | | 3 | 1 | | 3 |
| Neo Heliopan® OS | 2 | | | | | | | | 7 | | |
| Neo Heliopan® E1000 | | 4 | | | | | | 5 | | | |
| Neo Heliopan® AV | | 4 | 7.5 | 5 | | | | 5 | 4 | 7.5 | |
| Uvinul® A PLUS | | | | | 1 | | 2 | | | | |
| Uvinul® T 150 | 1 | | | | | | | | 1.3 | 1 | 1 |
| Tinosorb® M | | 2 | | | 2 | | 2 | | | | |
| Tinosorb® S | | 1 | | | 2 | | 2 | | | | |
| Parsol® 1789 | 1 | | | | | | | | 2 | | 1 |
| Z-Cote® HP 1 | 7 | 2 | 5 | | | 7 | 5 | | 6 | 2 | 1 |
| Eusolex® T 2000 | 5 | 2 | | 1 | 10 | | | 10 | | 2 | 1 |
| Veegum® Ultra | 1.5 | | 1.5 | | | 1.5 | 1.2 | | 1 | | |
| Keltrol® T | 0.5 | | 0.5 | | | 0.5 | 0.4 | | 0.5 | | |
| Cosmedia® SP | | | 0.2 | 0.3 | | | 0.1 | | | 0.2 | |
| Pemulen® TR 2 | | 0.3 | | 0.3 | | | | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

Tabelle 13: O/W-Pflegeemulsionen

| Komponente | 34 | 35 | 36 | 37 | 38 | 39 | 40 | 41 | 42 | 43 | 44 |
|---|---|---|---|---|---|---|---|---|---|---|---|
| L = Lotion, C = Creme | C | C | L | C | L | C | L | L | L | L | C |
| Eumulgin® VL 75 | | | 5 | | 4 | | | | | | 2 |
| Generol® R | | | | | | 2 | | | | | |
| Eumulgin® B2 | | | | | | | | | | 1 | |
| Tween® 60 | | | | | | | | | | 1 | |
| Cutina® E 24 | | | | 0.5 | | | | | | | |
| Eumulgin® SG | | | 0,1 | 0,5 | | 0,4 | | 0,2 | 0,1 | | |

(fortgesetzt)

| Komponente | 34 | 35 | 36 | 37 | 38 | 39 | 40 | 41 | 42 | 43 | 44 |
|---|---|---|---|---|---|---|---|---|---|---|---|
| Lanette® E | 0.5 | | | | | | | | | | |
| Amphisol® K | 0,5 | 0.5 | | | | | | | | | |
| Natriumstearat | | | | | 1 | | | | | | |
| Emulgade® PL 68/50 | | 2 | | 2 | | | | 3 | 4 | | |
| Tego® Care 450 | | 1 | | | | | | | 1 | | |
| Cutina® MD | 2 | 1 | 1 | 1 | | 5 | | | | 2 | |
| Lanette® 14 | | | | | 1 | | | 2 | | 1 | |
| Lanette® O | 2 | | | 2 | 1 | 3 | 1 | | 1 | 1 | 3 |
| Cutina® PES | 1 | 2 | | 3 | 1 | | | | | | 3 |
| Novata® AB | | | | | | | | | 1 | 1 | |
| Lanolin, wasserfrei, USP | | | | | | 4 | | | | | |
| Cosmedia® DC | | | 2 | | | 1.5 | | | 1 | 1 | |
| Cetiol® SB 45 | | | | | | | 2 | | | | |
| Cegesoft® C 17 | 2 | | | | | | | | | | |
| Dipropylheptylcarbonate | 5 | 5 | 4 | 4 | 3 | 4 | 5 | 4 | 5 | 10 | 2 |
| Myritol® PC | 6 | | | | | 5 | | | | | |
| Myritol® 331 | 2 | | 5 | | | | 2 | | | | 3 |
| Finsolv® TN | | | | 3 | 5 | | | 3 | 3 | | 1 |
| Cetiol® CC | | | | 3 | | | 4 | 3 | | | |
| Cetiol® OE | | | | | 2 | | 2 | | 5 | | |
| Dow Corning DC® 245 | | 2 | | | 1 | 4 | | | | 8 | 2 |
| Dow Corning DC® 2502 | | 1 | | | 1 | | | | | | 3 |
| Prisorine® 3758 | 3 | | | | | | | | | | 2 |
| Silikonöl Wacker AK® 350 | | | | | 1 | | | | | | 1 |
| Cetiol® 868 | | 2 | | | | | | | | | |
| Cetiol® J 600 | | 2 | | 2 | | | | | | | |
| Ceraphyl® 45 | | | | | | | 3 | | | | |
| Cetiol® SN | | | | 5 | | | | | | | |
| Cetiol® B | | | 5 | | | 5 | | 4 | | | 3 |
| Eutanol® G | | 3 | 5 | | 5 | | | | | | |
| Cetiol® PGL | | | | | | | | 5 | 2 | | |
| Dry Flo® Plus | | 1 | | | | | 2 | | | | 1 |
| SFE 839 | 1 | 1 | | | | 2 | | 2 | | 2 | |
| Nylon-12 | | | 2 | | 2 | | | | 2 | | |
| Silica **Dimethyl** Silylate | | | | 4 | | | | | | | |
| Mandelöl | | | | | | 2 | | | | | |
| Photonyl® LS | | | | | | 2 | | | | | |
| Panthenol | 1 | | | | | | | | | | |

(fortgesetzt)

| Komponente | 34 | 35 | 36 | 37 | 38 | 39 | 40 | 41 | 42 | 43 | 44 |
|---|---|---|---|---|---|---|---|---|---|---|---|
| Bisabolol | 0.2 | | | | | | | | | | |
| Tocopherol / Tocopherylacetat | 1 | | | | | | | | | | |
| Veegum® Ultra | | | | | | | | | 1 | | |
| Keltrol® T | | | | | | | | | 0.5 | | |
| Cosmedia® SP | 0.5 | | | | | 0.5 | 0.5 | 0.2 | | | 0.5 |
| Carbopol® ETD 2001 | | 0.3 | | 0.3 | | | | | | | |
| Pemulen® TR 2 | | | 0.3 | | | 0.3 | | | | | |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

Formulierungen für den Sonnenschutz und die Hautpflege vom Typ Wasser in Öl

In Tabelle 16 werden Sonnenschutzformulierungen vom W/O Emulsionstyp, in Tabelle 17 werden Pflegeemulsionen beschrieben. Durch den Einsatz des Dipropylheptylcarbonate wird das sensorische Verhalten bei Applikation positiv beeinflusst. Die Mengenangaben beziehen sich jeweils auf Gew.-% der handelsüblichen Substanzen in der Gesamtzusammensetzung.

Tabelle 16: W/O - Sonnenschutzformulierungen

| Ingredient | 1 | 2 | 3 | 4 | 5 | 6 | 7 | 8 | 9 | 10 | 11 |
|---|---|---|---|---|---|---|---|---|---|---|---|
| L = Lotion; C = Creme | C | L | C | L | C | L | L | L | L | C | C |
| Dehymuls® PGPH | 4 | 2 | 1 | 3 | 3 | 1 | 1 | 2 | 2 | 4 | 1 |
| Monomuls® 90-018 | | | 2 | | | | | | | | |
| Lameform® TGI | 2 | | 4 | | 3 | | | | | 1 | 3 |
| Abil® EM 90 | | | | | | | 4 | | | | |
| Isolan® PDI | | | | | | 4 | | 2 | | | |
| Zinkstearat | 1 | | | 1 | 1 | | | 1 | | 1 | |
| Bienenwachs | 1 | | 5 | 1 | | | | 5 | | 7 | 5 |
| Tego® Care CG | | | | | 1 | | | | | | 0.5 |
| Prisorine® 3505 | | | 1 | | | 1 | 1 | | | | 1 |
| Cosmedia DC | 3 | 4 | 2 | 1 | 1 | 2 | 2 | 2 | 3 | 1 | 1 |
| Dipropylheptylcarbonate | 5 | 4 | 4 | 3 | 2 | 4 | 3 | 4 | 2 | 3 | 5 |
| Myritol® 331 | 2 | | | | 3 | 6 | | | | | 3 |
| Finsolv® TN | | | | 5 | | | 2 | | | | |
| Cetiol® CC | 5 | | 2 | | 4 | 2 | | | 2 | 3 | 5 |
| Tegosoft DEC | | 4 | | 3 | | | 5 | 5 | | | |
| Cetiol® OE | | | | | 4 | | 5 | | 4 | 2 | |
| Dow Corning® DC 244 | | | 3 | | | | 2 | | 2 | 4 | |
| Dow Corning® DC 2502 | 1 | | 1 | | 2 | 1 | | | | | 1 |

(fortgesetzt)

| Ingredient | 1 | 2 | 3 | 4 | 5 | 6 | 7 | 8 | 9 | 10 | 11 |
|---|---|---|---|---|---|---|---|---|---|---|---|
| Silikonöl Wacker AK 350 | | 1 | | 4 | | | | 3 | | | |
| Cetiol® PGL | | 3 | | | | 2 | | | 4 | | |
| Cophero® F 1300 | 1 | | | | | | | | | | |
| Magnesium sulfat x 7 $H_2O$ | 1 | | | | | | | | | | |
| Neo Heliopan® Hydro (Na-Salz) | 2 | | 2.2 | | 3 | 3 | | | 1 | | 2 |
| Neo Heliopan® 303 | | 5 | | | | | | | 4 | | 4 |
| Uvasorb® HEB | 1 | | | 1 | 1 | | | | | | 2 |
| Neo Heliopan® MBC | 2 | | | | | 2 | 2 | 2 | | | |
| Uvinul® A plus | | | | | 2 | | | | 3 | 3 | |
| Neo Heliopan® AP (Na-Salz) | | 2 | 2 | | 1 | | | | 1 | | 6 |
| Neo Heliopan® AV | 3 | | 4 | 6 | 4 | 7.5 | 4 | 5 | | | 1 |
| Uvinul® T 150 | 1 | 1 | | | 2.5 | | | 1 | | | |
| Parsol® 1789 | 2 | 1 | | | | | 2 | | 2 | 2 | |
| Zinkoxid NDM | 2 | | | 4 | | 10 | | 3 | 5 | | 4 |
| Tinosorb® M | | 3 | | 3 | | | | 2 | | 2 | |
| Tinosorb® S | | 3 | | 3 | | | | 2 | | 2 | |
| Eusolex® T Aqua | | | 8 | | | | | 5 | | | |
| Eusolex® T 2000 | | 2 | 5 | | 5 | | 3 | 3 | | 3 | 4 |
| Ethanol | | | | | | | | | | 8 | |
| Glycerin | 5 | 3 | 3 | 3 | 5 | 3 | 2 | 3 | 10 | 4 | 3 |
| Wasser, Konservierungsmittel | ad 100, q.s. | | | | | | | | | | |

Tabelle 17: W/O-Pflegeemulsionen

| Komponente | 55 | 56 | 57 | 58 | 59 | 60 | 61 | 62 | 63 | 64 | 65 |
|---|---|---|---|---|---|---|---|---|---|---|---|
| L = Lotion. C = Creme | C | L | C | L | C | L | L | L | C | C | C |
| Dehymuls® PGPH | 1 | 3 | 1 | 2 | 3 | 1 | 1 | 2 | 1 | 1 | 1 |
| Monomuls® 90-018 | 2 | | | | | | | | 2 | | 2 |
| Lameform® TGI | 4 | 1 | | | 3 | | | 1 | 4 | 3 | 3 |
| Abil® EM 90 | | | | | | | 4 | | | | |
| Isolan® PDI | | | | | | 4 | | | | | |
| Glucate® DO | | | | 5 | | | | | | | |
| Arlacel® 83 | | | 5 | | | | | | | | |
| Dehymuls' FCE | | | | | | | | | | | |
| Dehymuls' HRE 7 | | | | | | | | 4 | | 1 | |
| Zinkstearat | 2 | 1 | | 1 | 1 | | | 1 | 1 | 1 | |
| Bienenwachs | 4 | | | 1 | | | | 1 | 4 | 7 | |
| Tego Care® CG | | | | | 1 | | | | | | 0.5 |

(fortgesetzt)

| Komponente | 55 | 56 | 57 | 58 | 59 | 60 | 61 | 62 | 63 | 64 | 65 |
|---|---|---|---|---|---|---|---|---|---|---|---|
| Prisorine® 3505 | | | 1 | 1 | | 1 | 1 | | | | 1 |
| Dry Flo® Plus | 2 | | | | | | 2 | | | | 2 |
| SFE 839 | | 2 | | | | 2 | 3 | 2 | | | |
| Nylon-12 | | | 2 | | 2 | | | | 2 | | |
| Silica **Dimethyl** Silylate | | | | 2 | | | | | | 2 | |
| Lanolin; anhydrous USP | | | 5 | | | | | | | 4 | |
| Dipropylheptylcarbonate | 3 | 4 | 2 | 12 | 10 | 2 | 2 | 6 | 3 | 12 | 1 |
| Cegesoft® C 17 | | | 3 | | | | | | | 1 | |
| Myritol® PC | | | | | | 2 | | 4 | | | |
| Myritol® 331 | 6 | | | | 2 | 6 | 2 | | | | 8 |
| Finsolv® TN | | | | 5 | | 2 | 5 | | | | |
| Cetiol® A | | 6 | | | | 4 | | | | | |
| Cetiol® CC | | 8 | | | 2 | 2 | 2 | | | | 5 |
| Cetiol® SN | | 5 | | | | | | 3 | | | |
| Cetiol® OE | 3 | | | | 4 | | 2 | | 4 | 2 | |
| Dow Corning DC® 244 | | | | | 1 | | 2 | | | | |
| Dow Corning DC® 2502 | | | 1 | | 2 | | | | | | |
| Prisorine® 3758 | | | | | 3 | | | | | | |
| Silikonöl Wacker AK® 350 | | | | 4 | | | | 3 | | | |
| Cetiol® 868 | | | | | | | | | | 2 | 7 |
| Cetiol® J 600 | | | 4 | | | 2 | | | | | |
| Ceraphyl® 45 | | | | 2 | | | | 2 | | 6 | |
| Cetiol® B | | | 2 | 4 | | | | | | 3 | |
| Eutanol® G 16 | | 1 | | | | | | | | 3 | |
| Eutanol® G | | | 3 | | | | | 8 | | | |
| Cetiol® PGL | | | | | | 4 | | | 9 | | |
| Mandelöl | | | | | 1 | | 5 | | | | |
| Insect Repellent® 3535 | 2 | | | | | | | | | | |
| N, N-Diethyl-m-toluamid | | | | 3 | | | | 5 | | | |
| Photonyl® LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone® 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |

(fortgesetzt)

| Komponente | 55 | 56 | 57 | 58 | 59 | 60 | 61 | 62 | 63 | 64 | 65 |
|---|---|---|---|---|---|---|---|---|---|---|---|
| Glycerin | 5 | 3 | 3 | | 5 | 3 | 2 | | 10 | 4 | |
| Wasser, Konservierungsmittel | Ad 100, q.s. | | | | | | | | | | |

Tabelle 18

| Komponente | 1 | 2 | 3 | 4 | 5 | 6 | 7 |
|---|---|---|---|---|---|---|---|
| Ethanol | | 4,00 | | 3,00 | | 4,50 | |
| Wasser dem. (add to) | 100 | 100 | 100 | 100 | 100 | 100 | 100 |
| Phenonip | 1,00 | 1,00 | 1,00 | 1,00 | 1,00 | 1,00 | 1,00 |
| Glycerin 99% | 3,00 | 3,00 | 3,00 | 3,00 | 3,00 | 3,00 | 3,00 |
| Ultragel® 300 (Polyquaternium-37) | 0,8 | 1,00 | 1,50 | 0,90 | 2,00 | 1,50 | 0,75 |
| Eumulgin® VL 75 (Lauryl Glucoside (and) Polyglyceryl-2 Dipolyhydroxystearate (and) Glycerin) | 1,80 | - | 0,80 | - | - | - | - |
| Emulgade® PL 68/50 (Cetearyl Glucoside (and Cetearyl Alcohol) | - | - | - | - | - | 3,00 | - |
| Eumulgin® SG (Sodium Stearylglutamate) | 0,10 | 0,12 | 0,30 | 0,10 | - | | 0,08 |
| Cutina® PES (Pentaerythrityl Distearate) | 2,50 | 3,5 0 | 1,70 | 4,00 | 2,00 | 4,00 | 5,00 |
| Cetiol® J600 (Oleyl Erucate) | 2,00 | - | 5,00 | - | - | 1,00 | 3,00 |
| Cetiol® CC (Dicaprylyl Carbonate) | 3,50 | - | 4,00 | 4,00 | 2,50 | 3,00 | 4,00 |
| Cetiol® PGL (Hexyldecanol (and) Hexyldecyl Laurate) | - | 2,00 | - | 4,00 | 5,00 | 5,00 | 1,00 |
| Tegosoft® DEC (Diethylhexyl Carbonate) | - | - | - | 2,50 | - | - | 3,00 |
| DC® 345 (Cyclopentasiloxane) | 4,00 | - | - | - | 2,00 | - | - |
| Cetiol® B (Dibutyl Adipate) | 5,00 | 3,5 | 5,00 | | | 2,00 | |
| Myritol® 331 (Cocoglycerides) | 3,00 | - | - | - | - | 4,00 | 4,50 |
| Dipropylheptylcarbonate | 3,00 | 3,00 | 5,00 | 2,00 | 7,00 | 5,00 | 3,00 |
| DHA (Dihydroxyaceton) | 2,00 | 1,00 | 2,50 | 1,00 | 2,00 | 2,00 | 3,50 |
| Uvinul® T 150 (Ethylhexyl Triazone) | - | | 1,00 | - | - | - | - |
| Tinosorb® M (Methylene Bis-Benzotriazoyl Tetramethylbutylphenol) | 2,00 | 1,00 | 1,00 | 2,00 | 1,00 | 2,00 | 1,00 |
| Tinosorb® S (Bis-Ethylhexyloxyphenol Methoxyphenyl Triazine) | - | 2,00 | - | - | 0,50 | - | - |
| Uvasorb® HEB (Diethylhexyl Butamino Triazone) | - | - | - | - | 1,00 | - | - |
| Neo Heliopan® AV (Ethylhexyl Methoxycinnamate | - | 5.00 | 5,00 | - | 3,00 | - | - |
| Parsol® 1789 (Butyl Methoxydibenzoylmethane) | - | 3.00 | 3,00 | - | 2,00 | - | - |
| Neo Heliopan® 303 (Octocrylene) | - | 3,50 | 2,00 | - | - | - | - |

Sprühbare W/O Emulsion (SPF 20)

| Phase | Bestandteil | INCI | Gew.-% |
|---|---|---|---|
| I | Dehymuls® LE | PEG-30 Dipolyhydroxystearate | 3.00 |

(fortgesetzt)

| Phase | Bestandteil | INCI | Gew.-% |
|---|---|---|---|
| | DC 245 | Cyclopentasiloxane | 5.00 |
| | Cetiol® A | Hexyl Laurate | 5.00 |
| | Cetiol® CC | Dicaprylyl Carbonate | 1,50 |
| | CETIOL® 4 All | Dipropylheptylcarbonate | 1,50 |
| | Cosmedia ® Gel CC | Dicaprylyl Carbonate (and) Stearalkonium Hectorite (and) Propylene Carbonate | 4.00 |
| | GranLux CC-50 | Titanium Dioxide Dispersion | 20.00 |
| II | Deionized Water | Aqua | qs 100 |
| | Butylene Glycol | Butylene Glycol | 3.00 |
| | Ajidew N50 | Sodium PCA | 2.00 |
| | Magnesium Sulphate | Magnesium Sulphate | 0.50 |
| | EDTA 4 Na | Tetrasodium EDTA | 0.05 |
| | Preservative | | q.s. |
| III | Parfum | | q.s. |
| Viscosity, Brook. RVT, 20°C, Sp 4, 5 rpm 2700 mPas | | | |

Sprühbare W/O Emulsion (SPF 20)

| Phase | Bestandteil | INCI | Gew.-% |
|---|---|---|---|
| I | Dehymuls® LE | PEG-30 Dipolyhydroxystearate | 4.00 |
| | Dehymuls ® PGPH | Polyglyceryl-2 Dipolyhydroxystearate | 4.00 |
| | Cetiol® 868 | Ethylhexyl Stearate | 7.00 |
| | CETIOL® 4 All | Dipropylheptylcarbonate | 2,70 |
| | CETIOL® Ultimate | Undecane (and) Tridecane | 2,70 |
| | Cosmedia®Gel CC | Dicaprylyl Carbonate (and) Stearalkonium Hectorite (and) Propylene Carbonate | 3.00 |
| | Cetiol ® OE | Dicaprylyl Ether | 5.00 |
| | Zincum N 29 | Zinc Oxide | 2.00 |
| II | GranLux CC-50 | Titanium Dioxide Dispersion | 20.00 |
| III | Deionized Water | Aqua | qs 100.00 |
| | Glycerine | Glycerine | 5.00 |
| | Sodium Chloride | Sodium Chloride | 0.50 |
| | Magnesium Sulphate | Magnesium Sulphate | 0.50 |
| | Preservative | | q.s. |
| IV | Parfum | | q.s. |
| Viscosität: Brookfield. RVT, 20°C, Sp 5, 10 rpm 10400mPas | | | |

Sonnenschutz Spray (SPF 6)

| Phase | Bestandteil | INCI | Gew.-% |
| --- | --- | --- | --- |
| I | Deionized Water | Aqua | qs 100 |
| | Butylene Glycol | Butylene Glycol | 3.00 |
| II | Veegum Ultra | Magnesium Aluminum Silicate | 0.50 |
| | Keltrol T | Xanthan Gum | 0.30 |
| III | Eumulgin ® VL 75 | Lauryl Glucoside (and) Polyglyceryl-2-Dipolyhydroxystearate (and) Glycerin | 6.00 |
| | Cetiol® CC | Dicaprylyl Carbonate | 3,50 |
| | CETIOL® Ultimate | Undecane (and) Tridecane | 1 |
| | CETIOL® 4 All | Dipropylheptylcarbonate | 2,50 |
| | Cetiol® AB | C12-15 Alkyl Benzoate | 7.00 |
| | GranLux CC-50 | Titanium Dioxide Dispersion | 10.00 |
| | Preservative | | q.s. |
| | Parfum | | q.s. |
| Viscosität: Brook. RVT, 20°C, Sp 4, 5 rpm 3500 mPas pH= 6.5 | | | |

Sonnenlotion (SPF 20)

| | Bestandteil | INCI | Gew.-% |
| --- | --- | --- | --- |
| I | Myritol® 331 | Cocoglycerides | 2.00 |
| | Cetiol® SB 45 | Shea Butter | 1.00 |
| | Cosmedia Gel CC | Dicaprylyl Carbonate (and) Stearalkonium Hectorite | 2.00 |
| | Uvasorb® HEB | Dioctyl Butamido Triazone | 3.00 |
| | CETIOL® Ultimate | Undecane (and) Tridecane | 2,00 |
| | CETIOL® 4 All | Dipropylheptylcarbonate | 5,00 |
| | Neo Heliopan® AV | Ethylhexyl Methoxycinnamate | 4.00 |
| | Neo Heliopan® E1000 | Isoamyl p-Methoxycinnamate | 4.00 |
| | Neo Heliopan® 303 | Octocrylene | 3.00 |
| | Copherol® 1250 C | Tocopheryl Acetate | 0.25 |
| | Cosmedia® DC | Hydrogenated Dimer Dilinoleyl/Dimethylcarbonate Copolymer | 2.00 |
| II | GranLux CC-50 | Titanium Dioxide Dispersion | 6.00 |
| | Satiaxane CX 91 | Xanthan gum | 0,30 |
| | Eumulgin® VL75 | Lauryl Glucoside (and) Polyglyceryl-2-Dipolyhydroxystearate (and) Glycerin | 3.13 |
| III | Deionized Water | Water | 58.22 |
| | 2 NaEDTA | Disodium EDTA | 0.10 |
| | Liponic EG-1 | Glycereth-26 | 3.00 |
| IV | Tinosorb M | Methylene bis-benzotriazolyltetramethylbutylphenol | 3.00 |

(fortgesetzt)

| | Bestandteil | INCI | Gew.-% |
|---|---|---|---|
| | Water | Water | 3.00 |
| | Preservative | | q.s. |
| | Parfum | | q.s |
| Viscosität Brook. RVT, 20°C, spindle 5, 10 rpm 2560 mPaspH= 6.70 | | | |

Sonnenlotion (SPF 50+)

| | Bestandteil | INCI | Gew.-% |
|---|---|---|---|
| I | Cetiol® B | Bibutyl Adipate | 2.00 |
| | Cosmedia® DC | Hydrogenated Dimer Dilinoleyl/Dimethylcarbonate Copolymer | 1.00 |
| | CETIOL® Ultimate | Undecane (and) Tridecane | 2,00 |
| | CETIOL® 4 All | Dipropylheptylcarbonate | 5.00 |
| II | Neo Heliopan® OS | Ethylhexyl Salicylate | 3.00 |
| | Tinosorb® S | Bis-ethylhexyloxyphenol Methoxyphenyl Triazine | 2.50 |
| | Neo Heliopan® AV | Ethylhexyl Methoxycinnamate | 6.00 |
| | Neo Heliopan® 303 | Octocrylene | 8.00 |
| | Neo Heliopan® 357 | Butyl Methoxydibenzoylmethane | 2.50 |
| III | Cosmedia® Gel CC | Dicaprylyl Carbonate (and) Steralkonium Hectorite (and) Propylene Carbonate | 2.00 |
| IV | Emulgade® PL 6850 | Cetearyl Glucoside (and) Cetearyl Alcohol | 2.50 |
| | Eumulgin ® VL 75 | Lauryl Glucoside (and) Polyglyceryl-2-Dipolyhydroxystearate (and) Glycerin | 3.00 |
| V | GranLux CC-50 | Titanium Dioxide Dispersion | 10.00 |
| VI | Deionized Water | Aqua | qs 100 |
| | 2 NaEDTA | Disodium EDTA | 0.10 |
| | Preservative | | q.s. |
| | Liponic EG-1 | Glycereth-26 | 3.00 |
| | Keltrol® T | Xanthan gum | 0.60 |
| | Veegum Ultra | Magnesium Aluminum Silicate | 2.00 |
| VII | Dry Flo Plus | Aluminum Starch Octenylsuccinate | 2.00 |
| VIII | Tinosorb® M | Methylene Bis-Benzotriazolyl Tetramethylbutylphenol | 5.00 |
| IX | Parfum | | q.s. |
| Viskosität Brookfield Helipath 20°C, TB, 10 rpm 16200 mPas pH= 6.7 | | | |

Anti-Ageing Sonnenschutz (SPF 30)

| | Bestandteil | INCI | Gew.-% |
|---|---|---|---|
| I | Myritol® 331 | Cocoglycerides | 2.00 |

(fortgesetzt)

| | Bestandteil | INCI | Gew.-% |
|---|---|---|---|
| | CETIOL® 4 All | Dipropylheptylcarbonate | 2,00 |
| | Cetiol® B | Bibutyl Adipate | 3.00 |
| | CETIOL® Ultimate | Undecane (and) Tridecane | 2.00 |
| | Uvinul A plus | Diethylamino Hydroxybenzoyl Hexyl Benzoate | 5.00 |
| | Uvinul T 150 | Ethylhexyl Triazone | 2.50 |
| | Cosmedia® Gel CC | Dicaprylyl Carbonate (and) Stearalkonium Hectorite | 2.00 |
| | Cosmedia® DC | Hydrogenated Dimer Dilinoleyl/Dimethylcarbonate Copolymer | 2.00 |
| II | GranLux CC-50 | Titanium Dioxide Dispersion | 10.00 |
| | Satiaxane CX 91 | Xanthan gum | 0.40 |
| | Eumulgin® VL75 | Lauryl Glucoside (and) Polyglyceryl-2-Dipolyhydroxystearate (and) Glycerin | 3.50 |
| III | Deionized Water | Aqua | 49.60 |
| | 2NaEDTA | Disodium EDTA | 0.10 |
| | Glycerin | Glycerin | 3.00 |
| | Preservative | | q.s. |
| IV | Photonyl® LS | Arginine (and) Disodium Adenosine Triphosphate (and) Mannitol (and) Pyridoxine HCl (and) RNA (and) Histidine HCl (and) Phenylalanine (and) Tyrosine | 2.00 |
| | Water | | 12.00 |
| V | Copherol® 1250 C | Tocopheryl Acetate | 0.50 |
| | Parfum | | q.s. |
| Viskosität: Brook. RVT, 20°C, Spindel 5, 10 rpm 3560 mPaspH= 6.50 | | | |

Anti-Falten Creme

| | Bestandteil | INCI | Gew.-% |
|---|---|---|---|
| I | Eumulgin ® BA 25 | Beheneth-25 | 3.30 |
| | Lameform® TGI | Polyglyceryl-3 Diisostearat | 0.70 |
| | CETIOL® 4 All | Dipropylheptylcarbonate | 2.00 |
| | Cutina ® PES | Pentaerythrityl Distearate | 2.00 |
| | Cetiol ®CC | Dicaprylyl Carbonate | 1.30 |
| | Cetiol ® AB | C12-C15 Alkyl Benzoate | 5.00 |
| | Cetiol ® LC | Coco-Caprylate/Caprate | 3.00 |
| | GranLux CC-50 | Titanium Dioxide Dispersion | 6.00 |
| | Phenonip | Phenoxyethanol (and) parabens | 0.80 |
| II | Deionized Water | Aqua | 61.85 |
| | 4NaEDTA | Tetrasodium EDTA | 0.05 |
| | Glycerin | Glycerin | 3.00 |
| | Butylene Glycol | Butylene Glycol | 2.00 |

(fortgesetzt)

| | Bestandteil | INCI | Gew.-% |
|---|---|---|---|
| III | Veegum Ultra | Magnessium Aluminum Silicate | 3.00 |
| | Keltrol T | Xanthan Gum | 0.30 |
| IV | Parfum | Perfume Waterfresh | 0.50 |
| | Nylon-12 | Nylon-12 | 3,00 |
| | Coviox ® T70 C | Tocopherol | 0.10 |
| V | Deionized Water | Aqua | 1.00 |
| | Plantactiv ® Aesculus | Escin | 0.10 |
| Viskosität, Brookfield, RVT, 20°C, Spindel 5, 5 rpm 11500 mPas pH= 6.2-6.7 | | | |

Sonnenschutzlotion SPF 30

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |
| | CETIOL® Ultimate | Undecane (and) Tridecane | 1.00 |
| | CETIOL® 4 All | Dipropylheptylcarbonate | 1.00 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia ® DC | Hydrogenated Dimer Dilinoleyl/Dimethylcarbonate Copolymer | 1.00 |
| II | GranLux CC-50 | Titanium Dioxide Dispersion | 14.00 |
| | Satiaxane CX 91 | Xanthan gum | 0.10 |
| | Cosmedia ® SP | Sodium Polyacrylate | 0.40 |
| | Eumulgin® VL75 | Lauryl Glucoside (and) Polyglyceryl-2-Dipolyhydroxystearate (and) Glycerin | 3.75 |
| III | Deionized Water | Aqua | qs 100.00 |

(fortgesetzt)

| | Bestandteil | INCI | Gew.-% |
|---|---|---|---|
| | Neo Heliopan® Hydro | Phenylbenzimidazole Sulfonic acid | 2.00 |
| | Trometamine | Trometamine | 1.60 |
| | Phenonip | | qs |
| Viscosität: Brookfield, RVT, 20°C, Spindel 5, 10 rpm 2240 mPas pH= 7.35 | | | |

Sonnencreme SPF 50+

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |
| | CETIOL® Ultimate | Undecane (and) Tridecane | 1.00 |
| | CETIOL® 4 All | Dipropylheptylcarbonate | 1.00 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia® DC | Hydrogenated Dimer Dilinoleyl/Dimethylcarbonate Copolymer | 2.00 |
| | Emulgade® PL 6850 | Cetearyl Glucoside (and) Cetearyl Alcohol | 4.50 |
| | Lanette® E | Sodium Cetearyl Sulfate | 1.0 |
| II | GranLux CC-50 | Titanium Dioxide Dispersion | 14.00 |
| III | Deionized Water | Aqua | qs 100.00 |
| | Neo Heliopan® Hydro | Phenylbenzimidazole Sulfonic Acid | 2.00 |
| | NaOH 50% | Sodium Hydroxide | 0.6 |
| | | Preservative | q.s. |
| | | Parfum | q.s. |
| Viskosität: Brookfield, Helipath 20°C, TE, 4 rpm , 195000 mPas pH= 7.5 | | | |

AP/Deo in Aerosol Form (Gew.-%)

| Bestandteil | INCI | A | B |
|---|---|---|---|
| IPM | Isopropyl Myristate | 10,8 | 10,8 |
| CETIOL® 4 All | Dipropylheptylcarbonate | 5.00 | 5.00 |
| Cosmedia® Gel CC | Dicaprylyl Carbonate (and) Stearalkonium Hectorite (and) Propylene Carbonate | - | 2.00 |

(fortgesetzt)

| Bestandteil | INCI | A | B |
|---|---|---|---|
| | Caprylic capric triglyceride(and)Stearalkonium Hectorite (and) Propylene Carbonate | 2.00 | - |
| Locron® P | Aluminium chlorhydrate | 3.00 | 3.00 |
| Hydagen® CAT | Triethyl Citrate | 1.00 | 1.00 |
| Perfume | Perfume | 1.20 | 1.20 |
| Propane | | 75.00 | 75.00 |

Foundation oder Sonnenschutz enthaltend Titanium Dioxid

| Ingredients | INCI Name | Gew.-% |
|---|---|---|
| CETIOL® 4 All | Dipropylheptylcarbonate | 94.00 |
| Cosmedia® Gel CC | Dicaprylyl Carbonate (and) Stearalkonium Hectorite (and) Propylene Carbonate | 1.00 |
| Titanium dioxide | | 5.00 |

Sonnenschutz Spray SPF 40 (Gew.-%)

| | Ingredient | INCI | A | B |
|---|---|---|---|---|
| I | Eumulgin ® VL 75 | Lauryl Glucoside (and) Polyglyceryl-2 Dipolyhydroxystearate (and) Glycerin | 6.0 | 6.0 |
| | Generol ® R | Brassica Campestris (Rapeseed) | 1.0 | 1.0 |
| | Cegesoft ® SH Cosmedia ® Gel CC | Sterols Shorea Stenoptera Dicaprylyl Carbonate(and) Stearalkonium Hectorite (and) Propylene Carbonate | 1.0 2.0 | 1.0 |
| | Eusolex 2292 | Ethylhexylmethoxycinnamate | 7.5 | 7.5 |
| | Tinosorb® S | Bis-ethylhexyloxyphenol methoxyphenyltriazine | 2.0 | 2.0 |
| | CETIOL® 4 All | Dipropylheptylcarbonate | 1.5 | 1,5 |
| | Cetiol ® AB | C12-15 Alkyl Benzoate | 4.0 | 4.0 |
| | Cosmedia ® DC | Hydrogenated Dimer Dilinoleyl / Dimethylcarbonate Copolymer | 1.00 | 1.00 |
| II | Water | | 61.45 | 63.45 |
| | EDTA 4Na (Prolabo) | Tetrasodium EDTA | 0,05 | 0.05 |
| | Elestab 50J | Chlorphenesin & Methylparaben | 0.60 | |
| III | Cosmedia®SP | Sodium Polyacrylate | 0.40 | 0.40 |
| IV | Tinsorb® M | Methylenebis benzotriazolyl tetramethylbutylphenol | 10.0 | 10.0 |
| Viskosität, Brookfield. RVT, 23° C, spindle 2,5 rpm, ph:6 3500mPas 700mPas | | | | |

Spray SPF 40

| | Bestandteil | INCI | A | B |
|---|---|---|---|---|
| I | Eumulgin® VL75 | Lauryl Glucoside (and) Polyglyceryl-2 Dipolyhydroxystearate (and) Glycerin | 6.0 | 6.0 |
| | Generol® | Brassica Campestris (Rapeseed) Sterols | 1.0 | 1.0 |

(fortgesetzt)

| | Bestandteil | INCI | A | B |
|---|---|---|---|---|
| | Cegesoft® SH | Shorea Stenoptera | 1.0 | 1.0 |
| | Cosmedia® Gel CC | Dicaprylyl Carbonate(and) Stearalkonium Hectorite (and) Propylene Carbonate | 3.0 | - |
| | Eusolex 2292 | Ethylhexylmethoxycinnamate | 7.5 | 7.5 |
| | Tinosorb S | Bis-ethylhexyloxyphenol methoxyphenyltriazine | 2.13 | 4.74 |
| | CETIOL® 4 All | Dipropylheptylcarbonate | 2.0 | 2.0 |
| | Cetiol® AB | C12-15 Alkyl Benzoate | 4.0 | 4.0 |
| II | Water | | 62.32 | 62.71 |
| | EDTA 4Na | Tetrasodium EDTA | 0.05 | 0.05 |
| | Elestab 50J | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.00 | 10.00 |
| Viskosität Book. RVT, 23° C, spindle 2,5 rpm, ph:6 A: 4020 mPas B: 730 mPas | | | | |

Feuchtigkeitsspendende Sonnenschutz Lotion W/O SPF 20

| | Bestandteil | INCI | A | B |
|---|---|---|---|---|
| I | Dehymuls® | PEG - 30 Dipolyhydroxystearate | 3.00 | 3.00 |
| | DC 245 | Cyclopentasiloxane | 5.00 | 5.00 |
| | Cetiol® OE | Dicaprylyl Ether | 4.00 | 4.00 |
| | Cetiol® A | Hexyl Laurate | 5.00 | 5.00 |
| | Cetiol® CC | Dicaprylyl Carbonate | 1.50 | 1.50 |
| | CETIOL® 4 All | Dipropylheptylcarbonate | 1.50 | 1.50 |
| II | Cosmedia® Gel CC | Dicaprylyl Carbonate(and) Stearalkonium Hectorite (and) Propylene Carbonate | 4.00 | |
| III | GranLux CC-50 | Titanium dioxide (and) Alumina (and) Dimethicone and) Stearic acid (and) Dicaprylyl Carbonate (and) Polyhydroxystearic acid | 20.00 | 20.00 |
| IV | Deionized water | Aqua | 49.55 | 53.55 |
| | Butylene Glycol | Butylene Glycol | 3.00 | 3.00 |
| | Ajidew N 50 | Sodium PCA | 2.00 | 2.00 |
| | Mg SO4 | Magnesium Sulfate | 0.50 | 0.50 |
| | EDTA, 4 Na | Tetrasodium EDTA | 0.05 | 0.05 |
| | Phenonip | Phenoxyethanol (and) Parabens | 0.50 | 0.50 |
| V | PerfumeSoleil/L27 8319/C | Floressence | 0.40 | 0.40 |
| Viskosität, Brook. RVT, spindle 4, speed 5 [mPas] | | | 2000 | 5000 |

Spray SPF 40 (Angaben in Gew.-%)

| | Bestandteil | INCI | A | B |
|---|---|---|---|---|
| I | Eumulgin® VL 75 | Lauryl Glucoside (and) Polyglyceryl-2 Dipolyhydroxystearate (and) Glycerin | 6.0 | 6.0 |
| | Generol® R | Brassica Campestris (Rapeseed) Sterols | 1.0 | 1.0 |
| | Cegesoft® SH | Shorea Stenoptera | 1.0 | 1.0 |
| | Cosmedia® Gel CC | Dicaprylyl Carbonate(and) Stearalkonium Hectorite (and) Propylene Carbonate | 2.0 | |
| | Eusolex 2292 | Ethylhexylmethoxycinnamate | 7.5 | 7.5 |
| | Tinosorb S | Bis-ethylhexyloxyphenol methoxyphenyltriazine | 2.0 | 2.0 |
| | CETIOL® 4 All | Dipropylheptylcarbonate | 1.5 | 1.5 |
| | CETIOL® Ultimate | Undecane (and) Tridecane | 1.5 | 1.5 |
| | Cetiol® AB | C12-15 Alkyl Benzoate | 4.0 | 4.0 |
| | Cosmedia ® DC | Hydrogenated Dimer Dilinoleyl/Dimethylcarbonate Copolymer | 1.0 | 1.00 |
| II | Water | | 61.45 | 63.45 |
| | EDTA 4Na | Tetrasodium EDTA | 0.05 | 0.05 |
| | Elestab 50J | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.0 | 10.0 |
| Viskosität, Brook. RVT, 23° C, spindle 2, 5 rpm, pH: 6 [mPas] A: 3500, B: 700 | | | | |

Mat finish fluid foundation

| | Bestandteil | INCI | Gew.-% |
|---|---|---|---|
| I | Lameform® TGI | Polyglyceryl-3 Diisostearat | 1.50 |
| | Eumulgin® BA25 | Beheneth-25 | 3.50 |
| | Cetiol® CC | Dicaprylyl Carbonate | 4.00 |
| | Myritol® 331 | Cocoglycerides | 2.00 |
| | CETIOL® Ultimate | Undecane (and) Tridecane | 1.00 |
| | CETIOL® 4 All | Dipropylheptylcarbonate | 1.00 |
| | Fitoderm® | Squalane | 3.00 |
| | Cutina® PES | Pentaerythiryl Distearate | 1.00 |
| | Cegesoft® SH | Shorea Stenoptera | 1.00 |
| | Phenonip | Phenoxyethanol (and) Parabens | 0.80 |
| | KF 96, 100cs | Dimethicone | 3.00 |
| | Cetiol® OE | Dicaprylyl Ether | 3.00 |
| II | Sun Croma C47-051 | Titanium Dioxide | 6.00 |
| | SunPuro Yellow Iron Oxide C33-9001 | Iron Oxide CI 77492 | 1.30 |
| | SunPuro Red Iron Oxide C33-8001 | Iron Oxide CI 77491 | 0.60 |

(fortgesetzt)

| | Bestandteil | INCI | Gew.-% |
|---|---|---|---|
| | SunPuro Black Iron Oxide C33-7001 | Iron Oxide CI 77499 | 0.20 |
| III | Deionized Water | Aqua | 52.50 |
| | Glycerine | Glycerin | 3.00 |
| | Butylene Glycol | Butylene Glycol | 2.00 |
| IV | Veegum Ultra | Magnesium Aluminium Silicate | 1.00 |
| | Keltrol T | Xanthan Gum | 0.40 |
| V | Cosmedia® Gel CC | Dicaprylyl Carbonate (and) Stearalkonium Hectorite (and) Propylene Carbonate | 3.00 |
| VI | Cosmedia® PMMA V12 | Polymethylmethacrylate | 2.00 |
| VII | Mirasil CDPDM | Cyclomethicone (and) Diphenyldimethicone | 3.00 |
| VIII | Perfume Caresse | | 0.20 |
| Viskosiät, Brook. RVT, spindle 3, speed 5, 8400mPas pH:6.7 | | | |

Samtige Reinigungsmaske

| | Bestandteil | INCI | Gew.-% |
|---|---|---|---|
| I | Emulgade® SE-PF | Glyceryl Stearate (and) Ceteareth-20 (and) Ceteareth-12 (and) Cetearyl Alcohol (and) Cetyl Palmitate | 8.00 |
| | Lanette® O | Cetearyl Alcohol | 2.00 |
| | Cegesoft® PS6 | Olus | 5.00 |
| | Cetiol® CC | Dicaprylyl Carbonate | 2.00 |
| | Cutina® PES | Pentaerythrityl Distearate | 3.00 |
| | CETIOL® 4 All | Dipropylheptylcarbonate | 2.00 |
| II | Cosmedia® Gel CC | Dicaprylyl Carbonate (and) Stearalkonium Hectorite (and) Propylene Carbonate | 5.00 |
| III | Deionized Water | Aqua | 45.00 |
| | Glycerine | Glycerin | 10.00 |
| | EDTA, 4Na | Tetrasodium EDTA | 0.10 |
| | Elestab® 50J | Chlorphenesin (and) Methylparaben | 0.40 |
| IV | Dry Flo AF | Corn Starch Modified | 3.00 |
| | Propylene Glycol | Propylene Glycol | 3.00 |
| V | Kaolin | Kaolin | 10.00 |
| VI | Perfume Concombre | | 0.20 |
| | Coviox® T70C | Tocopherol | 0.10 |
| VII | Purisoft ® LS 9602 | Glycerin (and) Moringa Pterygosperma | 2.00 |
| VIII | Green Covasol W7035 (a.s.0.1 %) | CI 42090 (and) CI19140 | 3.20 |
| Viskosität, Brook. RVT, Helipath TE, Speed 4, 254 000mPas pH:6.3 | | | |

Luxuriöse Körper Butter

| | Bestandteil | INCI | Gew.-% |
|---|---|---|---|
| I | Deionized water | Aqua | 61.50 |
| | Glycerine | Glycerin | 5.00 |
| | Butylene Glycol | Butylene Glycol | 2.00 |
| | EDTA, 4 Na | Tetrasodium EDTA | 0.10 |
| | Phenonip | Phenoxyethanol (and) Parabens | 0.80 |
| II | Cosmedia® SP | Sodium Polyacrylate | 1.50 |
| III | Eumulgin® BA 25 | Beheneth-25 3.00 | 3.00 |
| | Lanette® O | Cetearyl Alcohol | 4.00 |
| | Cetiol® SN | Cetearyl Isononanoate | 3.00 |
| | Cetiol® SB45 | Butyrospermum Parkii | 5.00 |
| | Cetiol® CC | Dicaprylyl Carbonate | 0.50 |
| | CETIOL® 4 All | Dipropylheptylcarbonate | 0.50 |
| | KF 96, 100cs | Dimethicone | 1.00 |
| | Generol® R | Brassica Campestris (Rapeseed) Sterols | 0.50 |
| IV | Cosmedia® Gel CC | Dicaprylyl Carbonate (and) Stearalkonium | 5.00 |
| | | Hectorite (and) Propylene Carbonate | |
| V | SUNPMMA-S | Polymethylmethacrylate | 2.00 |
| | Deionized water | Aqua | 4.00 |
| VI | Coviox® T70C | Tocopherol | 0.10 |
| | Perfume Pétale 139012E | | 0.50 |
| Viskosität, Brook. RVT Helipath TE, speed4, 800 000mPas pH:7 | | | |

Happy Deo Mist

| Bestandteil | INCI | Gew.-% |
|---|---|---|
| IPM | Isopropyl Palmitate | 6.50 |
| CETIOL® 4 All | Dipropylheptylcarbonate | 6.50 |
| Cosmedia® Gel CC | Caprylic/Capric Triglyceride (and) Stearalkonium Hectorite (and) Propylene Carbonate | 2.00 |
| Locron P | Aluminium Clorohydrate | 5.00 |
| Perfume Deo Talco | Perfume | 0.30 |
| Propellant | | 77.7 |
| Ratio propellant/other (3.34:1); Valve: RK 150P RA 634/5/8 (Vapor Phase); Button: V04.776 | | |

Hair gloss Gelatine für sehr geschädigtes und stumpfes Haar

| Handelsname | INCI | Gew.-% |
|---|---|---|
| Cetiol® CC | Dicaprylyl Carbonate | 33,6 |
| Undecane | | 10 |

(fortgesetzt)

| Handelsname | INCI | Gew.-% |
|---|---|---|
| CETIOL® 4 All | Dipropylheptylcarbonate | 43,6 |
| Wacker HDK H 20 (Wacker) | Silica Dimethyl Silylate | 12,5 |
| Parfum | | 0,3 |
| pH Wert | | N.A. |
| Viskosität bei 20°C, Helipath, spindel E, 5rpm (mPas) | | 500000 |
| Herstellung: Vermischen der Zutaten in der angegebenen Reihenfolge. | | |

Tiefen Haar Pflege Creme mit Wildmango Butter

| Phase | Handelsname | INCI | Gew.-% |
|---|---|---|---|
| I. | Strukture XL (National Starch) | Hydroxypropyl Starch Phospat | 5 |
| | Wasser, entionisiert | | 86,3 |
| II. | Dehyquart L 80 | Dicocoylethyl Hydroxyethylmonium Methosulfat (und) Propylene Glycol | 2,6 |
| | Dipropylheptylcarbonate | | 1 |
| | Irwinol® LS 9319 | Octyldodecanol (und) Irvingia gabonensis (und) Hydrogenated Coco Glycerides | 0,1 |
| | Lamesoft®TM Benz | Glycol distearate (and) Coco Glucoside (and) Glyceryl Oleate (and) Glyceryl Stearate | 4 |
| | Gluadin ®WLM | Hydrolyzed Wheat Protein | 0,5 |
| | Nutrilan®MILK | Hydrolyzed Milk Protein | 0,5 |
| III. | Konservierungsmittel | | q.s. |
| | Parfum | | q.s. |
| >> | pH Wert | | 3,8 |
| >> | Viskosität (mPas), Brookfield RVT, 20°C, Spindel 4, 10 rpm | | 10000 |

Herstellung: 1.Auflösen von Struktur XL in Wasser und Rühren bis zur vollständigen Homogenität, 2. Anpassen des pH Wertes auf 3,5 - 4,0 mit Zitronensäure, verdünnt, 3. Hinzufügen der restlichen Zutaten in der aufgeführten Reihenfolge während des Rührens (Irwinol muss vor der Hinzugabe geschmolzen werden)

Hair Cream gloss & Conditioning

| Phase | Handelsname | INCI | Gew.-% |
|---|---|---|---|
| I. | Dehyquart® F 75 | Distearoylethyl Hydroxyethylmonium Methosulfat (und) Cetearyl Alcohol | 2 |
| | Lanette®O | Cetearyl Alcohol | 3 |
| | Dipropylheptylcarbonate | | 1 |
| | Dehyquart® L 80 | Dicocoylethyl Hydroxyethylmonium Methosulfat (und) Propylene Glycol | 2 |
| | Cacao Butter (Nederland) | Theobroma cacao Seed Butter | 0,5 |
| | Structure XL (National Starch) | Hydroxypropyl Starch Phosphat | 5 |
| II. | Wasser, entionisiert | | 86,02 |

(fortgesetzt)

| Phase | Handelsname | INCI | Gew.-% |
|---|---|---|---|
| | Glycerin | | 0,48 |
| III. | Konservierungsmittel | | q.s. |
| | Parfum | | q.s. |
| >> | pH Wert | | 4,3 |
| >> | Viskosität (mPas) Brookfield RVT, 20°C Spindel 5, 10 rpm | | 47600 |

Herstellung: 1. Schmelzen der Zutaten von Phase I bei 80-85°C, 2. Hinzufügen des Wassers bei derselben Temperatur während des Rührens. Diese Temperatur halten und für 30 Minuten rühren, 3. Abkühlen unter langsamen Rühren, 4. Anpassen des pH Wertes, wenn nötig mit Zitronensäure, 5. Hinzufügen des Konservierungsmittels und des Parfums bei unter 40°C.

Cream to Powder Foundation

| | Komponente INCI Bezeichung (Handelsname, Hersteller) | Gew.% |
|---|---|---|
| A | Diisostearyl malate (Corum 5015, Corum) | 7,7 |
| | Titanium dioxide (HD-0748, Lubrizol) | 12,6 |
| | Iron oxides (HD-3170, Lubrizol) | 1,25 |
| | Iron oxides (HD-3511, Lubrizol) | 0,2 |
| B | Dimethicone (and) dimethicone crosspolymer (DC 9041 Sillcone Elastomer Blend, Dow Corning) | 8 |
| | Dipropylheptylcarbonate | 5 |
| | Ethylhexyl Isononanoate (Corum 5021, Corum) | 6 |
| | Pentaerythryl tetraisostearate (Corum 5041, Corum) | 7 |
| | Polyisobutane (and) $C_{15-19}$ alkane (and) $C_{12-14}$ isoparaffin (Permethyl 284C, Presperse) | 3 |
| | Cholesteryl hydroxystearate (Corum 5-89, Corum) | 0,5 |
| | Carnauba Wachs | 4 |
| | Cetyl Palmitate (Corum 5000, Corum) | 0,6 |
| | Myristyl myristate (Corum 5028, Corum) | 0,6 |
| | Tribehenine (Corum 5094, Corum) | 0,5 |
| | Mikrocristallines Wachs | 1,5 |
| | Methyl methacrylate crosspolymer (Glanzpearl GMX 0610, ISP) | 5,5 |
| | Boron nilinde (Soltouch CC6059, Momantive) | 1 |
| | Talk (und) methicone (Corum 5901, Corum) | 11,67 |
| | Mica (und) methicone (und) Parffinium liquidum (mineral) oil (Sericite SL012P, Nikko)10 | |
| | Lauroyl lysine (Corum 5106, Corum) | 6 |
| C | Pentaerythryl tetraisostearate (und) Paraffinium liquidum (mineral) oil (und) disteardimonium hectorite (und) propylene carbonate (und) palmitoyl tripeptide (Corum 9913, Corum) | 3 |
| | Pentaerythryl tetraisostearate (und) Paraffinium liquidum (mineral) oil (und) disteardimonium hectorite (und) propylene carbonate (und) palmitoyl oligopeptide (Corum 8616, Corum) | 3 |

(fortgesetzt)

| | Komponente INCI Bezeichung (Handelsname, Hersteller) | Gew.% |
|---|---|---|
| | Phenoxyethenol (und) methylparaben (und) butylparaben (und) ethylparaben (und) propylparaben (Paragon MEPB, Molmyre) | 1 |
| | Parfum | 0,06 |
| Herstellung: Vormischung von A herstellen, B zu A geben und auf 90 °C erhitzen, gut mischen, Hitzezufuhr einstellen und C zugeben. | | |

Makeup foundation

| Komponente INCI Bezeichung (Handelsname, Hersteller) | Gew.-% |
|---|---|
| Wasser | 78,2 |
| Glycerin | 1,89 |
| Triethanolamine 99% | 0,75 |
| Methylparaben (Nipagin M. Nipa) | 0,19 |
| Xanthan gum (Keltrol SP, CP Kelco) | 0,28 |
| Titanium dioxid (Softex Titanium dioxide, C47-7756, Sun Chemical) | 8,75 |
| Brown iron oxid (SunChroma Brown iron oxide, C33-315 Sun Chroma, Sun Chemical) | 1,08 |
| Red iron oxide (SunChroma red iron oxide, C33-124 Sun Chroma, Sun Chemical) | 0,17 |
| Phenyl trimethicone (DC 556 cosmetic Fluid, Dow Corning) | 3,41 |
| Dipropylheptylcarbonate | 1 |
| Stearic acid, 94% | 1,33 |
| Cetyl stearyl alcohol (Lanette O, BASF Personal Care and Nutrition) | 2,84 |
| Propylparaben (Nipasol, M. Nipa) | 0,1 |
| " | |

Skin Corrector"

| Komponente INCI Bezeichung (Handelsname, Hersteller) | Gew.-% |
|---|---|
| Synthetisches Wachs (Cirebelle 108, Cirebella) | 2,9 |
| Dipropylheptylcarbonate | 19,51 |
| Synthetisches Wachs (Cirebelle 303, Cirebella) | 2,33 |
| Titanium dioxid Wachs dispersion (CWD-8906, Sun Chemical) | 65,12 |
| Yellow iron oxide wax dispersion (CWD-8942, Sun Chemical) | 4,18 |
| Red iron oxide wax dispersion (CWD-8801, Sun Chemical) | 1,91 |
| Black iron oxide wax dispersion (CWD-9921, Sun Chemical) | 0,05 |
| Alumina (und) titanium dioxid (SpectraFlex Focus White C88-1001, Sun Chemical) | 4 |

Matte Antiaging Foundation

| Phase | Komponente INCI Bezeichung (Handelsname, Hersteller) | Gew.-% |
|---|---|---|
| A | Cyclopentasiloxane (und) dimethicone copolyol (DC 5225C, Dow Corning) | 12 |

(fortgesetzt)

| Phase | Komponente INCI Bezeichung (Handelsname, Hersteller) | Gew.-% |
|---|---|---|
| | Dipropylheptylcarbonate | 10 |
| | Titanium dioxid (BTD-401, Kobo) | 9 |
| | Yellow iron oxide (BYO-12, Kobo) | 0,8 |
| | Red iron oxide (BRO-12, Kobo) | 0,07 |
| | Black iron oxide (BRO-12, Kobo) | 0,034 |
| | Methylmethacrylate crosspolymer (Sepimat SB, Seppic) | 1 |
| | Methylmethacrylate crosspolymer (Sepimat HB V, Seppic) | 1 |
| | Phenoxyethanol (und) methylparaben (und) ethylparaben (und) propylparaben (und) butylparaben (Sepicide HB, Seppic) | 17,296 |
| | Parfum | 0,3 |
| B | Dipalmitoyl hydroxyproline (Sepilift DPHP, Seppic) | 0,5 |
| | Palmitoyl praline (und) magnesium palmitoyl-glutamate (und) sodium palmitoyl sarcosinate (Sepifeel One, Seppic) | 05, |
| | Undecylenoyl phenylalanine (Sepiwhite MSH, Seppic) | 0,1 |
| C | Sodium hydroxide, 48% Lösung | 0,2 |
| | Glycerin | 5 |
| | Sodium chloride | 2 |
| | Imidazolidinyl urea (Sepicide CI, Seppic) | 0,2 |
| D | Wasser | 40 |

Herstellung: Phase A wird hergestellt durch Dispersion der Puder in den Silikonen und den n-Undecan/n-Tridecan unter Rühren, in Kugelmühle zerkleinern. Phasen B, C und D werden separat hergestellt und jeweils auf 80 °C erhitzt. Phase D wird zu Phase C hinzugefügt, dieses Gemisch wird dann zu B gegeben. Homogenisierung für einige Minuten, Abkühlen unter mäßigem Rühren. Diese Mischung wird dann unter heftigem Rühren zu Phase A gegeben und die entstehende Emulsion wird homogenisiert.

Super Matte Foundation

| Phase | Komponente INCI Bezeichung (Handelsname, Hersteller) | Gew.-% |
|---|---|---|
| A | Wasser | 54,72 |
| | Disodium EDTA | 0,1 |
| | Wasser (und) montmorillonite (und) cetyl alcohol (und) glycerin (und) pentylene glycol (und) sucrose stearate (und) aderoglucan (Matte Lite Concentrate 269, MMP) | 20 |
| B | Sucrose distearate (Sistearna SP30-C, MMP/Sisterna) | 0,75 |
| C | Titanium dioxide | 6 |
| | Red iron oxide | 0,5 |
| | Yellow iron oxide | 1,25 |
| | Black iron oxide | 0,13 |
| | Nylon 12 | 0,25 |
| D | Dimethicone (und) dimethicone/vinyl dimethicone crosspolymer (Clearocast 200, MMP) | 5 |
| | Dipropylheptylcarbonate | 1 |
| | Isododecane (und) Isononyl Isononanoate (Clearocast 550, MMP) | 10 |

(fortgesetzt)

| Phase | Komponente INCI Bezeichung (Handelsname, Hersteller) | Gew.-% |
|---|---|---|
| E | Konservierungsmittel | 0,3 |

"Chocolate Mousse"

| Phase | Komponente INCI Bezeichung (Handelsname, Hersteller) | Gew.-% |
|---|---|---|
| A | CI 77891 dimethicone (SAT-T-47051, US cosmetics) | 0,528 |
| | CI 77491 dimethicone (SAT-R-33128, US cosmetics) | 0,456 |
| | CI 77492 dimethicone (SAT-Y-33873, US cosmetics) | 0,732 |
| | CI 77499 dimethicone (SAT-B-33134, US cosmetics) | 0,284 |
| B | Dimethicone/vinyl dimethicone crosspolymer (und) silica (Dow Corning 9701 Cosmetic Powder, Dow Corning) | 23 |
| | Zink oxid (und) dimethicone (Z-cote HP-1, BASF) | 6 |
| C | Wasser | 4 |
| | Wasser (und) propylene glycol (und) Helianthus annuus (Sonnnenblume) seed oil (und) Theobroma cacao extract (und) sclarolum gum (Cocoa Phytolat, Alban Muller) | 1 |
| | Dimethicone (Dow Corning 200 Fluid, 5cSt, Dow Corning) | 22,75 |
| | Dipropylheptylcarbonate | 22,75 |
| | Cyclopentasiloxane (und) dimethiconol (Dow Corning 1501 Fluid, Dow Corning) | 5 |
| | Cyclopentasiloxane (und) dimethicone crosspolymer (und) dimethiconol (Dow Corning 9546 Silicone Elastomer Blend, Dow Corning) | 5 |
| | Theobroma cacao seed butter (Cocoa Butter, Alban Muller) | 1 |
| | Cocos nucifera (Kokosnuss) Öl (und) Gardenia tahitensis Blüten Extrakt (Monoi de Tahip Butter frangaced, Pacific Sud Cosmétique) | 1 |
| | Tocopherol acetate (di-alpha-Tocopherol Acetate, DSM) | 0,5 |
| | Polysilicone-15 (Parsol-SLX, DSM) | 5 |
| D | Parfum (Chocolat Creme 0310585, Expressions Perfumees) | 1 |

Herstellung: A wird vermahlen, und B wird zugefügt und gerührt. Phase C wird vorgelegt und unter Rühren und auf 45 °C erhitzt. Dann wird AB in kleinen Portionen zugefügt und zum Schluss wird D zugegeben.

Fresh Foundation Emulsion Mousse

| Phase | Komponente INCI Bezeichung (Handelsname, Hersteller) | Gew.-% |
|---|---|---|
| A | Ceteareth-25 (und) PEG-2 stearate (und) Paraffinium liquidum (mineral) oil (und) hydrogenated coconut oil (und) cetyl alcohol (und) sodium stearate (Base O/W 097, LCW) | 5 |
| | Caprylic/capric triglyceride | 15 |
| | Dipropylheptylcarbonate | 1 |
| B | Wasser | 59 |
| | Konservierungsmittel | 0,3 |
| | Glycerin | 2 |
| | Algas extract (und) sorbitol (Fucosorb, LCW) | 2 |
| C | CI 77499 (und) silica (Unipure Black LC 989 EM, LCW) | 0,1 |

(fortgesetzt)

| Phase | Komponente INCI Bezeichung (Handelsname, Hersteller) | Gew.-% |
|---|---|---|
| | CI 77492 (und) silica (Unipure Yellow LC 162 EM, LCW) | 0,75 |
| | CI 77491 (und) silica (Unipure Red LC 361 EM, LCW) | 0,18 |
| | CI 77891 (und) silica (Unipure white LC 981 EM, LCW) | 2,95 |
| | Mica (Submica FL, LCW) | 1 |
| | Methylmethacrylate crosspolymer (und) silica (Covabeed PMMA 2MUSL, LCW) | 1 |
| | Mica (Mica 8 R2041, LCW) | 4 |
| | Talk (Talc LCW, LCW) | 4 |
| | Sodium plyacrylate (Covacryl MV50, LCW) | 1 |
| | Silica dimethyl silyata (Covasilic 15, LCW) | 0,5 |
| Herstellung: A und B werden getrennt hergestellt und jeweils auf 70°C erhitzt. B wird zu A unter Rühren zugefügt. Phase C wurde separate hergestellt und mit einem Pulvermischer gut durchmischt und danach unter Rühren zur Mischung aus A und B gegeben. | | |

Mattifying Fluid Foundation

| Phase | Komponente INCI Bezeichung (Handelsname, Hersteller) | Gew.-% |
|---|---|---|
| A | Wasser | 20 |
| | Butylene glycol | 4 |
| | PEG-400 | 4 |
| | Dimethicone copolyol PEG-7 phosphate (Pecostil PS 100, Pheonix) | 1 |
| B | Titanium dioxide | 10 |
| | Talk | 2 |
| | Iron oxide yellow | 0,9 |
| | Iron oxide red | 0,3 |
| | Iron oxide black | 0,05 |
| | Titanium dioxide (CI 77891) (und) iron oxide (CI 77491) (und) mica (und) triethoxy caprylylsilane | 3 |
| C | $C_{20-22}$ alkyl phosphate (und) $C_{20-22}$ alcohols (Sensanov WR, Seppic) | 1 |
| | $C_{14-22}$ alcohol (und) $C_{12-20}$ alkylglucoside (Montanov L, Seppic) | 2 |
| | Isostearyl isostearat | 8 |
| | Undecane (and) Tridecane | 1 |
| | Dipropylheptylcarbonate | 9 |
| | Vegetable oil (und) palm alcohol (und) lecithin (und) vegetable stearyl esters (Sensactive Veg. Chemyunion) | 2 |
| D | Wasser | q.s. to 100 |
| | Xanthan gum | 0,15 |
| E | Tromethamine | q.s. |
| | Tetrasodium EDTA | 0,05 |
| | Polymethyl methacrylate (Micropearl M100, Seppic) | 3 |

(fortgesetzt)

| Phase | Komponente INCI Bezeichung (Handelsname, Hersteller) | Gew.-% |
|---|---|---|
| F | Sodium acrylate/acryloyldimethyllaurate copolymer (und) isohexadecanle (und) polysorbane (Simulgel EG, Seppic) | 0,5 |
| G | Phenoxyethanol (und) methylparaben (und) ethylparaben (und) propylparaben (und) butylparaben (Chemynoi, Chemyunion) | 0,1 |
| | Parfum | qs |

Maximum shine Lippenstifte

| Phase | Komponente INCI Bezeichung (Handelsname, Hersteller) | Gew.-% |
|---|---|---|
| A | D&C Red No. 6 Barium Lake (Red 6 lake, Emerald Hilton Davis) | 0,7 |
| | Iron oxides (CC33-5136 Russet Iron Oxide, Sun Chemical) | 2,5 |
| | Iron oxides (CC33-135 Black iron oxide, Sun Chemical) | 0,25 |
| | Ricinus communis (castor) seed oil (Crystal O, CasChem) | 3 |
| B | Ricinus communis (castor) seed oil (Crystal O, CasChem) | 35,75 |
| | Copernica cerifera (carnauba) Wachs (Carnauba No. 1, Strahl & Pitsch) | 1,1 |
| | Euphorbia cerifera (candelilla) Wachs (Candelilla Pure, Strahl & Pitsch) | 5 |
| | Ozokerite (Ozokerite Wax 1700, Frank B, Ross Company) | 2,5 |
| | Dipropylheptylcarbonate | 1 |
| | Mikrocrystallines Wachs (Microwax SP 19, Strahl & Pitsch) | 2 |
| | Diisostearyl malata (Schercamol DISM Ester, Lubrizol) | 24 |
| | Trisostearyl citrate (Schercomol TISC Ester, Lubrizol) | 16,85 |
| | Methylparaben | 0,2 |
| | Propylparaben | 0,1 |
| C | Mica (und) iron oxides (Colorona Copper, EMD chemicals) | 5 |
| D | Ascorbyl palmitate | 0,05 |

Ultra lasting lip color

| Phase | Komponente INCI Bezeichung (Handelsname, Hersteller) | Gew.-% |
|---|---|---|
| A | D&C Red No. 7 Calcium Lake (31-DA-3707, Emerald Hilton Davis) | 0,6 |
| | D&C Red no. 6 Barium Lake (31-DA-3006, Emerald Hilton Davis) | 1,5 |
| | Iron oxides (CC33-5138 Russet iron oxide, Sun Chemical) | 4,5 |
| | Titanium dioxide | 0,5 |
| | Ricinus communis (castor) seed oil (Crystal O, CasChem) | 8,3 |
| B | Ricinus communis (castor) seed oil (Crystal O, CasChem) | 21,65 |
| | Copernica cerifera (carnauba) Wachs (Carnauba No. 1, Strahl & Pitsch) | 1,5 |
| | Euphorbia cerifera (candelilla) Wachs (Candelilla Pure, Strahl & Pitsch) | 6 |
| | Ozokerite (Ozokerite Wax 1700, Frank B. Ross Company) | 2,5 |
| | Mikrocrystallines Wachs (Microwax SP 19, Strahl & Pitsch) | 3,5 |
| | Triisostearyl polyglyceryl-3 dimer diinoleate (Schercemol PTTD Ester, Lubrizol) | 10 |

(fortgesetzt)

| Phase | Komponente INCI Bezeichung (Handelsname, Hersteller) | Gew.-% |
|---|---|---|
| | Trisostearyl citrate (Schercomol TISC Ester, Lubrizol) | 22 |
| | Isopropyl Isostearate (Schercamol 316 Ester, Lubrizol) | 4 |
| | Dipropylheptylcarbonate | 1 |
| | Methylparaben | 0,2 |
| | Propylparaben | 0,1 |
| C | Mica (und) Titanium dioxide (Flamenco Red, Engelhard) | 12 |
| D | Ascorbyl palmitate (Ascorbyl palmitate, DSM) | 0,05 |

Long lasting Shine Lip Gloss

| Phase | Komponente INCI Bezeichung (Handelsname, Hersteller) | Gew.-% |
|---|---|---|
| A | Octyldodecanol | 47,93 |
| B | Ethylcellulose (Ethocel 100FP, Amerchol) | 5 |
| C | Undecane (and) Tridecane | 10 |
| | Dipropylheptylcarbonate | 8 |
| | bis-Biglyceryl polyacladipate-2 | 15 |
| | Octyl methoxycinnamate | 7,5 |
| | Polyethylene | 2 |
| D | Red No. 7 Aluminium lake (Sun Chemical) | 0,58 |
| | Iron oxide (Cl 77491, Sun Chemical) | 0,52 |
| | Iron oxide (Cl 77492, Sun Chemical) | 0,48 |
| | Iron oxide (Cl 77499, Sun Chemical) | 0,09 |
| E | Mica (und) titanium dioxide (Prepearis Shining Salin, Prespense) | 1,9 |
| | Dimethicone crosspolymer/silica (DC 7901 Powder, Dow Corning) | 1 |

High-shine Lippenstift

| | Komponente | Gew.-% |
|---|---|---|
| A | Hydrogenated Polydecene (und) ethylene propylene/styrene copolymer (und) butylene/ ethylene/styrene copolymer (Versagel MG 750, Penreco) | 25,89 |
| | Petrolatum (und) ethylene/propylene/styrene copolymer (und) butylene/ethylene/styrene copolymer (Versagel P100, Penreco) | 10,38 |
| | Euphorbia cerifera (candelilla) wachs (Candelilla wax refined, Ross) | 8,8 |
| | Cera alba (Bienenwachs) (White Bleached Beeswax, Ross) | 5,18 |
| | Lanolin Wachs | 4,14 |
| | Lanolin Öl | 4,14 |
| | Octyldodecanol (Eutanol® G, BASF Personal Care and Nutrition) | 4,14 |
| | Dipropylheptylcarbonate | 3,63 |
| | Copernicia cerifera (carnauba) Wachs (Carnauba wax, Ross) | 3,11 |
| | Octyldodecyl stearoyl stearate (Ceraphyl 647, ISP) | 3,11 |

(fortgesetzt)

| | Komponente | Gew.-% |
|---|---|---|
| | Tridecyl trimellitate (Liponate TDTM, Lipo) | 3,11 |
| | Propylparaben | 0,1 |
| | Butylated hydroxytoluene | 0,1 |
| B | Butyrospermum parkii (shea butter) (Shea Butter, Ultra refined, Biochemica) | 5,18 |
| | Jojoba esters (Floraesters 30, Floratech) | 2,07 |
| | Theobroma grandiflorum seed butter (Cupuacu Butter, Beraca/Ross) | 2,07 |
| C | Iron oxides (und) synthetic fluorphlogopite (Sunshine Super Russet, Sun Chemical) | 8,29 |
| | Iron oxides (Soft-Tex Russet Iron Oxide, Sun Chemical) | 5,18 |
| | Red No. 7 Lake | 1,04 |
| | Yellow No. 6 Lake | 0,38 |

Eleganter Lippenstift

| Komponente INCI Bezeichnung (Hersteller) | Gew.-% |
|---|---|
| Dipentaerythrityl hexacaprylate/hexacaprate (und) tridecyl trimellitate (und) tri-decyl stearate (und) neopentyl glycol dicaprylate/dicaprate (Lipovol MOS-350, Lipo) | 72,2 |
| Synthetisches Bienenwachs (Lipobee 102, Lipo) | 8 |
| Copernicia cerifera (carnauba) Wachs | 4 |
| Euphorbia cerifera (candelilla) wachs | 4 |
| Dipropylheptylcarbonate | 1 |
| Propylparaben | 0,2 |
| Mica (und) boron nitride (Lipomic 501 BN, Lipo) | 1 |
| Tocopherol (Vitamin E Acetate, Ruger Chemical) | 0,5 |
| Titanium dioxide (CI 77891) (und) dipentaerythrityl hexacaprylate/hexacaprate (und) tridecyl trimellitate (und) tridecyl stearate (und) neopentyl glycol dicaprylate/dicaprate (Titanium dioxide, 60% in Lipovol MOS-350, Lipo) | 2,8 |
| Iron oxide (CI 77489) (und) dipentaerythrityl hexacaprylate/hexacaprate (und) tridecyl trimellitate (und) tridecyl stearate (und) neopentyl glycol dicaprylate/dicaprate (iron oxide Red, 60% in Lipovol MOS-350, Lipo) | 4,7 |
| Iron oxide (CI 77489) (und) dipentaerythrityl hexacaprylate/hexacaprate (und) tridecyl trimellitate (und) tridecyl stearate (und) neopentyl glycol dicaprylate/dicaprate (iron oxide Yellow, 60% in Lipovol MOS-350, Lipo) | 0,8 |
| Iron oxide (CI 77489) (und) dipentaerythrityl hexacaprylate/hexacaprate (und) tridecyl trimellitate (und) tridecyl stearate (und) neopentyl glycol dicaprylate/dicaprate (iron oxide Black, 60% in Lipovol MOS-350, Lipo) | 0,8 |
| Herstellung: Alle Bestandteile werden unter Rühren auf 80-85 °C erhitzt. Der Ansatz wird auf 70-75°C abgekühlt und in eine Gießform gefüllt. | |

Lip Gloss

Herstellung: Phasen A und B werden in getrennten Gefäßen jeweils homogenisiert, dann wird Phase B zu Phase A gefügt und gemischt, bis das Produkt homogen vorliegt.

Creamy Sheer Lip Colour

| | Komponente INCI | Gew.-% |
|---|---|---|
| A | Sesamum indicum (Sesam) Samen Öl | 21,28 |
| | Octyldodecanol (Jarcol I-20, Jarchem) | 15,47 |
| | Euphorbia cerifera (candelilla) wachs (SP75, Strahl & Pitsch) | 12 |
| | Isohexyl Caprate | 10,07 |
| | Bis-Diglyceryl polyaryladipate-2 (Softisan 649, Sasol) | 9,95 |
| | Polyisobutene (Permethyl 104A, Presperse) | 9,67 |
| | Dipropylheptylcarbonate | 2,89 |
| | Polyethylene (Performalene PL Polyethylene, New Phrase) | 2,5 |
| | Mikrocrystallines Wachs (Be Square 195, New Phrase) | 2,5 |
| B | Synthetic wax and titanium dioxide (und) Isopropyl Titanium triisostearate (SW65U, Kobo) | 4,24 |
| | Synthetic wax and iron oxide (und) Isopropyl Titanium triisostearate (SW60ER, Kobo) | 1,41 |
| | Synthetic wax and D&C Red No. 6 Barium Lake (und) Isopropyl Titanium triisostearate (SW40R6E, Kobo) | 1,41 |
| | Synthetic wax and Blue No.1 Lake (und) Isopropyl Titanium triisostearate (SW30B1A, Kobo) | 0,71 |
| | Mica (und) titanium dioxide (KTZ Classic White, Kobo) | 0,96 |
| C | Vitis vinifera (grape) seed oil | 4,84 |
| | Vitamin E acetata | 0,1 |

Lip volume booster

| | INCI Bezeichnung (Handelsname, Hersteller) | Gew.-% |
|---|---|---|
| A | Ricinus communis (castor) oil | 39,7 |
| | Hydrogenated coco-glycerides (Softisan 100, Sasol) | 8 |
| | Candelilla cera (und) Paraffin (Cenilla G, Barlocher) | 9 |
| | Bis-Diglyceryl polyacyladipate-1 (Softisan 645, Sasol) | 8 |
| | Cera alba (Cerabell White, Barlocher) | 3 |
| | Cera carnauba Wachs (Cernauba T1, Barlocher) | 2 |
| | Tricaprylin (Trivant OC-G, Trivant Chemical) | 20 |
| | Tocopheryl acetate (D-alpha-Tocopheryl Acetate, DSM/Roche) | 0,5 |
| | Dipropylheptylcarbonate | 1 |
| | Propylparaben (Nipasol M. Nipa) | 0,1 |
| | BHT (Butylhydroxytoluol) | 0,05 |
| B | Ricinus communis (castor) oil (und) Cl 15850 (und) BHT (COD 8001, Sun Chemical) | 0,8 |
| | Calcium aluminum borosilicate (und) Cl 77891 (und) silica (und) tin oxide (Fionastar Noble Sparks, Merck) | 1 |
| | Mica (und) Cl 77891 (und) Cl 77491 (Timiron MP29 Sun Gold Sparkle, Merck) | 2 |
| | Mica (und) Cl 77891 (Timiron MP149 Diamond Cluster, Merck) | 2 |
| C | Glycerin (und) palmitoyl tripeptide-3 (Syn-Coll, Pentapharm) | 2,5 |

(fortgesetzt)

| | INCI Bezeichnung (Handelsname, Hersteller) | Gew.-% |
|---|---|---|
| | Alkyl methacrylate crosspolymer (Poly-Pore E200, Amcol HBS) | 0,35 |
| Herstellung: alle Bestandteile der Phase A auf 80°C erhitzen, Phase B zu A hinzufügen, Phase C mischen und zu AB zugeben kurz vor der Homogenisierung. | | |

Plumping effect Lippenstift

| | Komponenten INCI Bezeichnung (Handelsname, Hersteller) | Gew.-% |
|---|---|---|
| A | D&C Red No. 7 Ca Lake (Cl 15850:1) (HD-3007, Lubrizol) | 0,4 |
| | Red iron oxide (Cl 77491) (HD-3511, Lubrizol) | 0,4 |
| | Titanium dioxide Cl 77891 (HD-0748, Lubrizol) | 1,2 |
| | Paraffinum liquidum (mineral) oil (und) disteardimonium hectorite (und) propylene carbonate (Bentone Gel MO V, Elementis Specialities) | 1,5 |
| | Diisostearyl maleate (Corum 5015, Corum) | 10,5 |
| | Mica (und) titanium dioxide (und) tin oxide (Prestige Sparkling blue, Eckart) | 1,5 |
| B | Pentaerythrityl tetraisostearate (Corum 5041, Corum) | 15 |
| | Cholesteryl hydroxystearate (Corum 5069, corum) | 1 |
| | Hydrogenated polydecene (Nexbase 2006FG, Fortum) | 15 |
| | Undecane (and) Tridecane | 5 |
| | Dipropylheptylcarbonate | 5 |
| | Isostearyl isostearate (corum 5088, Corum) | 19,9 |
| | Phenyltrimethicone (DC 556, Dow Corning) | 2 |
| | PEG-8 Bienenwachs (Corum 2680, Corum) | 2 |
| | Ceresin | 9 |
| | Euphorbia cerifera (candelilla) wachs | 2 |
| | Cera alba (Bienenwachs) | 2 |
| | Copernicia cerifera (carnauba) Wachs | 3 |
| | Jojoba ester (Floraester-70, Floratech) | 1 |
| | Pentaerythrityl tetraisostearate (und) Paraffinum liquidum (mineral) oil (und) disteardimonium hectorite (und) propyl carbonate (und) palmitoyl oligopeptide (Corum 8813, Corum) | 2 |
| C | Vanilyl butyl ether (Corum 9235, Corum) | 0,4 |
| | Flavor | 0,2 |

Herstellung: Phase A wird zur Homogenität gemischt, die Phase B wird hinzugefügt und auf 90°C erhitzt und gut gemischt. Die Hitzezufuhr wird gestoppt und Phase C hinzugefügt, danach gut gemischt.

Lippenstift

| | Komponenten INCI Bezeichnung (Handelsname, Hersteller) | Gew.-% |
|---|---|---|
| A | Propylene glycol dicaprylate/dicaprate (und) bis-diglyceryl polyacyladipate-1 (und) bis-diglyceryl polyadipate-2 (Softisan Gel, Sasol) | 9 |
| | Stearalkonium hectorite (und) propylene carbonate (Softisan 649, Sasol) | 6 |
| | Hydrogenated coco-glycerides (Softisan 100, Sasol) | 22,5 |

(fortgesetzt)

| | Komponenten INCI Bezeichnung (Handelsname, Hersteller) | Gew.-% |
|---|---|---|
| | Hydrogenated palm oil (Softisan 154, Sasol) | 3 |
| | $C_{12-10}$ alkyl octanoate (Cosmacol EDI, Sasol) | 5 |
| | Petrolsturm (Merkur Vaseline773, Merkur) | 11 |
| | Cera alba (Bienenwachs) | 7 |
| | Cyclomethicone (Belsil CM 040, Wacker Chemie) | 3 |
| | Dipropylheptylcarbonate | 2 |
| | Undecane (and) Tridecane | 2 |
| | Ricinus communis (castor) oil | 6 |
| | Paraffin | 14 |
| | Antioxidans | qs |
| B | Titanium dioxide (Cl 77891) (Timiron Splendid Red, Merck) | 3,5 |
| | Mica (und) Titanium dioxide (Cl 77891) (Timiron Starlusler MP-115, Merck) | 2,5 |
| | Mica (und) Titanium dioxide (Cl 77891) (Timiron Super Silver Fine, Merck) | 3 |
| C | Parfum | qs |
| | Tocopheryl acetate | 0,5 |

Volumizing Mascara

| | Komponenten INCI Bezeichnung (Handelsname, Hersteller) | Gew.-% |
|---|---|---|
| A | Cera alba (Bienenwachs) (White Beeswax SP422P, Strahl & Pitsch) | 2,76 |
| | Glyceryl stearate | 2,3 |
| | Copernicia cerifera (carnauba) Wachs (Carnauba wax SP63, Strahl & Pitsch) | 1,98 |
| | Stearic acid | 3 |
| | Ethylcellulose (Ethocel Standard 100FP Premium, Amerchol) | 3 |
| | Dipropylheptylcarbonate | 1 |
| | Octyldodecanol | 7 |
| | Premised Chitosan PCA, 2,5% (Kytamer PCA Polymer, Amerchol) | 15 |
| B | Iron oxide (Black Iron Oxide, Sun Chemical) | 6 |
| | Wasser | 55,45 |
| C | Triethanolamine 99% | 1,5 |
| D | Propylene glycol (und) diazolidinyl ures (und) methylparaben (und) propylparaben (NiPaguard, Clariant) | 1 |

Lidschatten Stift (Eye shadow stick)

| | Komponenten INCI Bezeichnung (Handelsname, Hersteller) | Gew.-% |
|---|---|---|
| A | Dipropylheptylcarbonate | 1 |
| | Ocryldodacyl neopentanoate (Elefac I-205, Alzo) | qs. to 100 |
| | Calcium sodium borosilicate (und) silica (und) titanium dioxide (Reflecks MultiDimensions Varying Violet G58000000, BASF) | 14 |

(fortgesetzt)

| | Komponenten INCI Bezeichnung (Handelsname, Hersteller) | Gew.-% |
|---|---|---|
| | Mica (und) Titanium dioxide (und) iron oxides (Gemtone Moonstone G004, BASF) | 1 |
| B | Butylene glycol | 3,13 |
| | Acetylated glycol stearate (Unitwax, Guardian, ISP) | 22 |
| | Silica (Cab-o-Sil M5, Cabot) | 1,5 |
| C | Simmondsia chinensis (jojoba) seed oil (Lipovol J. Lipo) | 7,5 |
| | Stearic acid (Emersol 132, BASF Personal Care and Nutrition) | 3,6 |
| | Konservierungsmittel | qs |
| | Antioxidans | qs |
| Phase B wird geschmolzen und gemischt, und dann zur vorgemischten Phase A gegeben. AB werden unter Rühren auf 80-05°C erhitzt und dann wird langsam Phase C zugegeben. | | |

Metallic Silver shine Mascara

| | Komponenten INCI Bezeichnung (Handelsname, Hersteller) | Gew.-% |
|---|---|---|
| A | Wasser | 56,25 |
| | Xanthan gum (Keltrol T, CP Kelco) | 0,15 |
| | Magnesium aluminium silicate (Veegum HV, RT Vandervilt) | 0,55 |
| | Carbon black (und) wasser (MBD 201 20% Dispersion, Geotech) | 5 |
| B | Disodium EDTA (Edeta BD, BASF) | 0,05 |
| | Methylparaben (Methyl-4-hydroxybenzoate H 5501, Sigma Aldrich) | 0,3 |
| | Triethanolamine | 0,5 |
| | Propylene glycol | 2 |
| C | Aluminium powder (und) silica (Visionaire Bright Silver sea, Eckart) | 3 |
| D | Dipropylheptylcarbonate | 5 |
| | Cyclopentasiloxane (und) PEG/PPG-18/18 dimethicone (Dow Corning 5225C Formulation Aid, Dow Corning) | 1 |
| | Cera alba (Bienenwachs) (Ewacers 12, Wagner) | 10 |
| | Stearic acid (Kortacid 1695, Akzo Nobel) | 2 |
| | Glyceryl stearate (Imwitor 960K, Sasol Wax) | 2 |
| | Stearyl alcohol (Lanette 18, BASF Personal Care and Nutrition) | 2 |
| | Polyisobutene (Permethyl-104A, Chesham Chemicals) | 1 |
| | Phenoxyethanol (und) methylparaben (und) butylparaben (und) ethylparaben (und) propylparaben (und) isobutylparaben (Phenonip, Clariant) | 1,2 |
| | PVP polyvinylpyrrolidone (Luviskol K30 Powder, BASF) | 4 |
| | Carnauba Wachs (Ewacera 34, Wagner) | 4 |

Herstellung: A wird gemischt, B wird gemischt und zu A zugefügt, C wird zu AB gegeben und auf 75°C erhitzt. D wird separat vorgemischt und auf 75°C erhitzt und zu ABC gegeben, während des Abkühlens wird gerührt.

Creamy Shadow Stick

| | Komponenten INCI Bezeichnung (Handelsname, Hersteller) | Gew.-% |
|---|---|---|
| A | Undecane (and) Tridecane | 20,0 |
| | Dipropylheptylcarbonate | 18,5 |
| | PPG-3 myristyl ether (Varonic APM, Evonik, Degussa) | 7,0 |
| | Polyglyceryl-4- isostearate (and) cetyl PEG-PPG-10/1 dimethicone (and) hexyl laurate (Abil WE 09, Evonik, Degussa) | 1,0 |
| | Dimethicone, 10 mPas | 2,5 |
| | Cera alba (bees wax) | 4,5 |
| | Copernicia cerifera (carnauba) wax | 4,0 |
| | Ethylene/VA copolymer (A-C copolymer 400, Honeywell) | 2,5 |
| | Ozokerite | 5,8 |
| | C16-C36 acid triglyceride | 2,0 |
| | Isobutylparaben (and) isopropylparaben (and) butylparaben (Liquipar Oil, From Nature with Love) | 0,5 |
| B | Nylon-12 (egolon 12-10, Evonik, Degussa | 2,0 |
| | Titanium dioxide | 5,0 |
| | Chromium oxide green | 10,00 |
| | Cl 77491 (and) aluminium powder (and) silica | 5,0 |
| | Cl 77891 (and) Cl 77288 (and)mica | 10,0 |

Wasserfeste Mascara

| | Komponenten INCI Bezeichnung (Handelsname, Hersteller) | Gew.-% |
|---|---|---|
| A | Wasser | 32,84 |
| | Magnesium aluminium silicate (Veegum HV, RT Vanderbilt) | 0,10 |
| | Xanthan gum (Keltrol 1000, Kelco Corp.) | 0,20 |
| | Methylparaben | 0,25 |
| | Trisodium EDTA (Protacide Na3 EDTA, Protameen) | 0,01 |
| | Premixed sodium acrylates/acrylnitrogens copolymer (Hydrilien 9, 2,5% gel. Lio - siehe unten) | 15,00 |
| B | Glycerin (and) polyester 5 (Lipo PE Base G-55, Lipo) | 12,00 |
| | Talc (Rose Talc, Prespersa) | 3,00 |
| | Iron oxide (Cl 77499) (Iron Oxide Black, Ultra Chemical) | 9,00 |
| C | Triethanolamine, 99% | 1,00 |
| D | Synthetic bees wax (Lipobee 102, Lipo) | 10,00 |
| | Copernicia cerifera (carnauba) wax | 4,50 |
| | Undecane (and) Tridecane | 5,50 |
| | Dipropylheptylcarbonate | 1,00 |
| | Stearic acid (Lipo Stearic Acid, Lipo) | 3,00 |
| | Sorbitan sesquioleate (Liposorb SQO, Lipo) | 1,30 |
| | Propylparaben | 0,20 |
| E | Water (aqua) | 1,00 |

(fortgesetzt)

| | Komponenten INCI Bezeichnung (Handelsname, Hersteller) | Gew.-% |
|---|---|---|
| | Imidazolidinyl urea (Leposerve IU, Lipo) | 0,10 |

Herstellung: Die Bestandteile der Phase A werden gemischt und auf 70 °C erhitzt, Phase A wird in einer Kolloidmühle gemahlen, die Bestandteile der Phase B werden in der angegebenen Reihenfolge zugefügt unter beständigem Mahlen, bis das Pigment gleichmäßig dispergiert ist, danach wird Phase C zugefügt. Die Bestandteile von C werden gemischt und bei 80-84°C gemischt (und dabei Entlüftet). Phase D wird zum Ansatz gegeben und emulgiert, der Ansatz wird auf 35 °C abgekühlt und die vorgemischte Phase E wird zugefügt. Bei 30°C wird der Ansatz in geeignete Gefäße abgefüllt.

Sodium acrylates/acrylnitrogens copolymer Premix: 97,50 Gew.- % Wasser und 2,5 Gew.- % Sodium acrylates/acrylnitrogens copolymer werden gemischt und auf 78-80°C erhitzt unter Rühren. Es wird für 15 bis 20 Minuten geführt bis die Lösung klar und homogen ist. Danach wird auf Raumtemperatur abgekühlt.

Sebum resistenter Lidschatten

| | Komponenten INCI Bezeichnung (Handelsname, Hersteller) | Gew.-% |
|---|---|---|
| A | Stearyl dimethicone (DC 2503 Cosmetic Wax, Dow Corning) | 10,00 |
| | C30-C45 alkyl methicone (and) C30-C45 olefin (DC AMS-C30 Cosmetic Wax, Dow Corning) | 5,00 |
| | Isododecane (and) arcylates/polytrimethylsiloxymethacrylate (DC FA 4002 ID Silicaone Acrylate, Dow Corning) | 10,00 |
| | Cyclopentasiloxane (and) triemthylsiloxymethacrylate (DC 749 Fluid, Dow Corning) | 51,00 |
| B | Cyclopentasiloxane (and) dimethicone crosspolymere (DC 9040 Silcone Elastomer Blend, Dow Corning) | 5,00 |
| | Dipropylheptylcarbonate | 1 |
| | Undecane (and) Tridecane | 2 |
| C | Silica silylate (DC VM-2270 Aerogel Fine Particles, Dow Corning) | 1,00 |
| | Mica | 15,00 |
| Herstellung: Die Bestandteile der Phase A werden gemischt und auf 80 °C erhitzt, unter Rühren wird abgekühlt. Die Phase B wird gemischt und unter Rühren zur Phase A gegeben, C wird unter langsamem Rühren zugegeben. | | |

Lippenstift

| | Inhaltsstoff | INCI Bezeichnung | Gew.-% |
|---|---|---|---|
| I | Cerilla | Candellila Wax | 9.00 |
| | Cerewax | Microcristallina Wax | 3.50 |
| | Cerozo | Ozokerite Wax | 2.50 |
| | Cerauba | Carnauba Wax | 2.50 |
| | Cetiol MM | Myristyl Mysristate | 5.00 |
| | Eutanol® G | Octyldodecanol | 10.00 |
| | Myritol® 331 | Cocoglycerides | 5.00 |
| | CETIOL® 4 All | Dipropylheptylcarbonate | 4.00 |
| | CETIOL®Ultimate | Undecane (and) Tridecane | 4.00 |
| | Cetiol® J600 | Oleyl Erucate | 1.00 |
| | Nipasol M | Propyl Paraben | 0.50 |
| | Lamesoft® TGI | Polyglyceryl-3 Diisostearat | 3.00 |

(fortgesetzt)

| | Inhaltsstoff | INCI Bezeichnung | Gew.-% |
|---|---|---|---|
| | Cetiol® Sensoft | Propylheptyl Caprylate | 6.00 |
| II | COD 8008 | CI 77981 | 2.70 |
| | COD 8001 | CI 15850 | 12.90 |
| | COD 8010 | CI 15985 | 2.80 |
| | COD 8002 | CI 15850 | 1.40 |
| | COD 8007 | CI 42090 | 0.20 |
| | Ricinus Oil Codex | Ricinus Communis | 20.60 |
| III | | Fragance Astral 112 445 B | 0.40 |
| | Irwinol® LS 9319 | Octyldodecanol & Irvingia Gabonesis & Hydrogenated Coco-Glycerides | 2.50 |
| | Coviox® T70C | Tocopherol | 0.50 |

Lippenpflege

| | Inhaltsstoff | INCI Bezeichnung | Gew.-% |
|---|---|---|---|
| I | Polybutene M100 | Polybutene | 53.50 |
| | Cetiol® Sensoft | Propylheptyl Caprylate | 5.00 |
| | CETIOL® 4 All | Dipropylheptylcarbonate | 5.00 |
| | CETIOL® Ultimate | Undecane (and) Tridecane | 5.00 |
| | Isopropyl palmitate | Isopropyl Palmitate | 20.00 |
| | Cosmedia® DC | Hydrogenated Dimer Dilinoleyl Dimethylcarbonate Copolymer | 5.00 |
| | Red 28 Lake | Red 28 Lake | 0.05 |
| | | Perfume Fruits Rouges | 0.30 |
| | Coviox® T70C | Tocopherol | 0.10 |
| | Nipasol M | Propylparaben | 0.50 |
| II | Aerosil R972 | Silica Dimethyl Silylate | 5.30 |
| III | Ronastar Noble Spars | Calcium Aluminium Borosilicate (and) Silica (and) Titanium Dioxide | 0.25 |
| Viskosität (mPa.s): Brookfield RVT, Spindle 5, Speed 10 11 000 | | | |

O/W Flüssig Foundation

| | Inhaltsstoff | INCI Bezeichnung | Gew.-% |
|---|---|---|---|
| I | Deionized Water | Aqua | 50.50 |
| | Butylene Glycol | Butylene Glycol | 2.00 |
| | Glycerine | Glycerin | 3.00 |
| II | Veegum Ultra | Magnesium Aluminium Silicate | 1.00 |
| | Keltrol T | Xanthan Gum | 0.40 |
| III | Lameform® TGI | Polyglyceryl-3 Diisostearat | 1.50 |
| | Eumulgin® B2 | Ceteareth-20 | 3.50 |
| | Cutina® PES | Pentaerythrityl Distearate | 1.00 |

(fortgesetzt)

| | Inhaltsstoff | INCI Bezeichnung | Gew.-% |
|---|---|---|---|
| | Cegesoft® SH | Shorea Stenoptera | 1.00 |
| | CETIOL® 4 All | Dipropylheptylcarbonate | 4.00 |
| | CETIOL® Ultimate | Undecane (and) Tridecane | 2.00 |
| | Myritol® 331 | Cocoglycerides | 4.00 |
| | Fitoderm® | Squalane | 3.00 |
| | Cetiol® Sensoft | Propylheptyl Caprylate | 5.00 |
| | Phenonip | Phenoxyethanol (and) Parabens | 0.80 |
| IV | Sun Croma C47-051 | Titanium Dioxide | 6.00 |
| | SunPuro Yellow Iron Oxide C33-9001 | Iron Oxide CI 77492 | 1.30 |
| | SunPuro Red Iron Oxide C33-8001 | Iron Oxide CI 77491 | 0.60 |
| | SunPuro Black Iron Oxide C33-7001 | Iron Oxide CI 77499 | 0.20 |
| V | Cosmedia® Gel CC | Dicaprylyl Carbonate (and) Stearalkonium Hectorite (and) PropyleneCarbonate | 3.00 |
| VI | Micropearl M100 | Polymethylmethacrylate | 2.00 |
| VII | Mirasil CDPDM | Cyclomethicone (and) Diphenyldimethicone | 3.00 |
| VIII | Perfume Caresse | | 0.20 |
| Viskosität (mPa.s): Brk. RVT spindle 5, speed 10 5 500, pH 7.3 | | | |

Emulsion - applicable also for aerosol mousses

| SWOP emulsions - | | | 9 | 10 | 11 | 12 |
|---|---|---|---|---|---|---|
| Phase | Ingredients | INCI | % by weight | % by weight | % by weight | % by weight |
| A | Water, demin. | Aqua | 71.44 | 71.44 | 71.44 | 71.44 |
| | D-Panthenol USP | Panthenol | 1.00 | 1.00 | 1.00 | 1.00 |
| | Rheocare® HSP-1180 | Polyacrylamid omethylpropa ne Sulfonic Acid | 0.63 | 0.63 | 0.63 | 0.63 |
| | Potassium Hydroxide (20% solution) | Potassium Hydroxide | 0.13 | 0.13 | 0.13 | 0.13 |
| | Glycerin | Glycerin | 3.00 | 3.00 | 3.00 | 3.00 |
| | Cosmedia® SP | Sodium Polyacrylate | 0.80 | 0.80 | 0.80 | 0.80 |
| | Rheocare® XG (Europe only) | Xanthan Gum | 0.10 | 0.10 | 0.10 | 0.10 |
| B | Plantapon® LGC SORB | Sodium Lauryl Glucose Carboxylate (and) Lauryl Glucoside | 1,5 | 1,5 | 1,5 | 1,5 |
| C | Dehymuls® PGPH | Polyglyceryl-2 Dipolyhydrox ystearate | 4.00 | 4.00 | 4.00 | 4.00 |

(fortgesetzt)

| SWOP emulsions - | | | 9 | 10 | 11 | 12 |
|---|---|---|---|---|---|---|
| Phase | Ingredients | INCI | % by weight | % by weight | % by weight | % by weight |
| | | Cetyl Phosphate | 0.50 | | | |
| | | Stearic Acid / Pamitic Acid | | 0.70 | | |
| | Lanette E | Sodium Cetearyl Sulfate | | | | |
| | Eumulgin® Prisma | Disodium Cetearyl Sulfosuccina te | | | 0.30 | |
| | Eumulgin® SG | Sodium Stearoyl Glutamate | | | | 0.30 |
| | Cutina® HVG | Hydrogenate d Vegetable Glycerides | 0.50 | 0.50 | 0.50 | 0.50 |
| | Lanette® 22 | Behenyl Alcohol | 0.50 | 0.50 | 0.50 | 0.50 |
| | Cetiol® C 5 | Coco-Caprylate | 6.00 | 6.00 | 6.00 | 6.00 |
| | CETIOL® 4 All | Dipropylhept ylcarbonate | 6,0 | 12,0 | 4,0 | 10 |
| | Myritol® 331 | Cocoglycerid es | 1,5 | 1,5 | 1,5 | 1,5 |
| D | Cetiol® Ultimate | Undecane, Tridecane | 2.00 | 2.00 | 2.00 | 2.00 |
| E | Protectol® PE | Phenoxyetha nol | 1.00 | 1.00 | 1.00 | 1.00 |
| | Sensiva SC 10 | Capryl Glycol, Ethylhexylgly cerin | 0.50 | 0.50 | 0.50 | 0.50 |
| | (Schülke) | | | | | |
| **F** | **Perfume** | Parfum | 0.60 | 0.60 | 0.60 | 0.60 |
| | **SunPMMA** | Polymethyl Methacrylat e | 1 | 2 | 2 | 1 |
| | Timica® Gold Sparkle 212P* | | 0.10 | 0.25 | 0.5 | 0.20 |
| | Manufacturing Process 1.) Phase A: Disperse first D-Panthenol USP, Rheocare®HSP-1180 in water and adjust the pH at 5. Add Glycerin, Cosmedia® SP and Rheocare® XG in water and stir until a uniform gel is formed. Then add the Plantapon® LGC SORB (Phase B).2.) Heat Phase A+B up to 75°C-80°C3.) Heat Phase C (oil phase) separately to 80°C-85°C, mix homogeneously and add into the gel-surfactant mixture (A+B) while stirring.4.) At 70°C emulsion temperature add phase D 4.) At 55°C add Phase E. IMPORTANT: complete the evaporated water and homogenize with a suitable dispersion unit (e.g. Ultra Turrax). 5) Stir while cooling to 35°C and add phase F. Stop stirring at 30°C.<br>Filling up in aerosol can : 47g + 3g Propellant (2,7 Bar Propan/Butan) in 35*125mm, with PAM, Shoulder: Ogival 1 | | | | | |

*can also be replaced by the following pigments and mixtures thereof

Calcium Sodium Borosilicate (and) Titanium Dioxide
Calcium Sodium Borosilicate (and) Iron Oxides
Calcium Sodium Borosilicate (and) Titanium Dioxide (and) Iron Oxides
Calcium Sodium Borosilicate (and) Silica (and) Titanium Dioxide
Calcium Sodium Borosilicate (and) Titanium Dioxide (and) Ferric Ferrocyanide
Calcium Sodium Borosilicate (and) Titanium Dioxide (and) Carmine
Calcium Aluminium Borosilicate (and) Silica (and) Titanium Dioxide (and) Tin Oxide
Mica (and) Bismuth Oxychloride (and) Iron Oxides
Mica (and) Iron Oxides
Mica (and) Iron Oxides (and) Titanium Dioxide

Mica (and) Titanium Dioxide (and) Iron Oxides
Mica (and) Titanium Dioxide (and) Iron Oxides (and) Ferric Ferrocyanide
Mica (and) Titanium Dioxide (and) Iron Oxides (and) Chromium Oxide Greens
Mica (and) Titanium Dioxide (and) Iron Oxides (and) Carmine
Mica (and) Iron Oxides (and) Titanium Dioxide (and) Silica
Mica (and) Iron Oxides (and) Titanium Dioxide (and) Silica (and) Tin Oxide
Iron Oxides, Hydrogenated Lecithine, Aluminum Hydroxide
Iron Oxides (and) Mica
Iron Oxides (and) Disodium Stearoyl Glutamate (and) Aluminum Hydroxide
Iron Oxides (and) Triethoxycaprylylsilane
Silica (and) Iron Oxides
Synthetic Fluorphlogopite (and) Titanium Dioxid
Synthetic Fluorphlogopite (and) Lauroyl Lysine

AP/DEO formulations could be applied as

1. Stick based on anhydrous formulations
2. Aerosol spray based on dispersions

1. Stick based on anhydrous formulations

| AP/DEO Stick | | | | | |
|---|---|---|---|---|---|
| Phase | INCI | % by weight | % by weight | % by weight | % by weight |
| A | Stearyl Alcohol | 15.3 | 15 | 15 | 15 |
| | Hydrogenated Castor Oil | 3.8 | 3 | 3 | 3 |
| | Dipropylheptyl Carbonate | 60.9 | 30 | 20 | 40 |
| | Cyclomethicone | | 20 | 20 | 10 |
| | Caprylic / Capric Triglyceride | | | 3 | |
| | PPG-14 Butyl Ether | | 2 | 10 | 5 |
| | C12-15 Alkyl Benzoate | | | 2 | 1 |
| | Dimethicone | | 2 | 2 | |
| | Isopropylmyristate | | 3 | | 5 |
| | Polyethylene | | | 2 | 3 |
| | Silicate | | 2 | | 3 |
| | Starch-Derivate as e.g.Sodium Starch Octenylsuccinate | | 3 | 3 | |
| B | Aluminium Zirconium Tetrachlorohydrex Gly | 20.0 | | 20.0 | |
| | Aluminum Sesquichlorohydrate | | 20.0 | | 15.0 |
| C | Perfume | q.s | q.s. | q.s. | q.s. |
| | Deo actives as Ethanol, Benzylalcohol, Benzyl Benzoate, Farnesol , Piroctone Olamine, Ethylhexylglycerin, 2-Butyloctanoic acid, | q.s. | q.s. | q.s. | q.s. |
| Manufacturing Process | q) Melt phase A at 85°C and stir homogeneously. 2) Cool phase A to 75°C while stirring and add phase B. Stir until phase B is homogeneously dispersed. 3) Cool mixed phases A & B to 65°C while stirring and fill into stick containers. | | | | |

2.) AP/DEO Aerosol Spray (dispersion)

| Phase | INCI | % by weight | % by weight | % by weight | % by weight |
|---|---|---|---|---|---|
| A | Isopropyl Palmitate | 4.00 | | | |
| | Propylheptyl Caprylate | 4.00 | | | |
| | Isopropyl Myristate | | 5.00 | | |
| | Dicaprylyl Ether | | 5.00 | | |
| | Dicaprylyl Carbonate | | | | |
| | Cyclomethicone | | 1.00 | | |
| | Octyldodecanol | | | | |
| | Dipropylheptyl Carbonate | 4.00 | 5.00 | | |
| | Caprylic/Capric Triglyceride, Stearalkonium Hectorite, Propylene Carbonate 4.00 4.00 | | 4.00 | | |
| | Dicaprylyl Carbonate, Stearalkonium Hectorite, Propylene Carbonate | 4.00 | | | |
| | Aluminum Chlorohydrate | 5.00 | 6.00 | 6.00 | |
| | Triethyl Citrate | | 2.00 | | |
| | Perfume | 0.30 | 0.3 | | |
| B | Propellant: Butane/Propane | Ad.100 | Ad.100 | Ad.100 | Ad.100 |

Pressed Powder Foundation:

| | | | | |
|---|---|---|---|---|
| A | Talc FM SSA *(K.S. Pearl)* | Talc (and) Dimethicone | 35.3 | Filler |
| | Mearlmica® Treated SVA | Mica, Lauroyl Lysine | 25.00 | Performance mineral |
| | Pearl-Glo® SF PG1099 | Bismuth Oxychloride | 6.0 | Effect pigment |
| | SA-SB-300 *(Miyoshi Kasei)* | Silica | 3.5 | Skin feel modifier |
| | Z-Cote® HP1 | Zinc Oxide (nano), Triethoxycaprylylsilane | 2.0 | Broad spectrum UV filter |
| | STR-100A-LP *(Sakai Chemical Industry)* | Titanium Dioxide (nano), Hydrated Silica, Dimethicone/ Methicone Copolymer, Aluminum Hydroxide | 3.0 | UV-B filter |
| | Timica® Terra White MN4501 | Mica, Titanium Dioxide | 5.0 | Effect pigment |
| | Timica® Terra Brown MN4509 | Mica, Iron Oxides, Titanium Dioxide | 1.4 | Effect pigment |
| | Timica® Terra Yellow MN4502 | Mica, Iron Oxides, Titanium Dioxide | 0.4 | Effect pigment |
| B | Uvinul® A Plus B | Ethylhexyl Methoxycinnamate, Diethylamino Hydroxybenzoyl Hexyl Benzoate | 7.0 | Broad spectrum UV filter |
| | Cetiol® 4 All | Dipropylheptyl Carbonate | 4.4 | Emollient |

(fortgesetzt)

| | | | | |
|---|---|---|---|---|
| | Euxyl PE 9010 *(Schülke)* | Phenoxyethanol, Ethylhexylglycerin | 1.0 | Preservative |
| C | Flamenco® Silk Blue 630M | Mica, Titanium Dioxide | 6.0 | Effect pigment |

BB Cream SPF 25

| PHASE | BESTANDTEILE | INCI | % BY WEIGHT | |
|---|---|---|---|---|
| A | Water, demin. | Aqua | 40.3 | |
| | Rheocare® XG *(Europe only)* | Xanthan Gum | 0.2 | Rheology modifier |
| | Euxyl® PE 9010 *(Schülke)* | Phenoxyethanol, Ethylhexylglycerin | 1.0 | Preservative |
| B | SunCROMA Titanium Dioxide C47-5566 *(Sun Chemical)* | CI 77891 | 5.25 | Colorant |
| | SunCROMA Yellow Iron Oxide C33-8073 *(Sun Chemical)* | CI 77492 | 0.4 | Colorant |
| | SunCROMA Red Iron Oxide C33-8075 *(Sun Chemical)* | CI 77491 | 0.15 | Colorant |
| | SunCROMA Black Iron Oxide C33-5198 *(Sun Chemical)* | CI 77499 | 0.04 | Colorant |
| | Butylene Glycol | Butylene Glycol | 4.0 | Humectant |
| | Glycerin | Glycerin | 2.0 | Humectant |
| C | Emulgade® Sucro Plus | Sucrose Polystearate, Cetyl Palmitate | 5.0 | Emulsifier (O/W) |
| | Eumulgin® Prisma | Disodium Cetearyl Sulfosuccinate | 2.0 | Emulsifier (O/W) |
| | Cosmedia® Gel CC | Dicaprylyl Carbonate, Stearalkonium Hectorite, Propylene Carbonate | 3.5 | Rheology modifier |
| | Cetiol® 4 All | Dipropylheptyl Carbonate | 15.0 | Emollient |
| | Uvinul® MC 80 | Ethylhexyl Methoxycinnamate | 5.5 | UV-B filter |
| | Uvinul® A Plus Granular | Diethylamino Hydroxybenzoyl Hexyl Benzoate | 4.0 | UV-A filter |
| | Tinosorb® S | Bis-Ehtylhexyloxyphenol | 1.5 | Broad spectrum UV filter |
| | | Methoxyphenyl Triazine | | |
| D | Tinosorb® M | Methylene Bis-Benzotriazolyl Tetramethylbutylphe nol (nano), Aqua, Decyl Glucoside, Propylene Glycol, Xanthan Gum | 4.0 | Broad spectrum UV filter |
| E | Mearlmica® SV | Mica | 3.5 | Filler |
| | Flamenco® Silk Gold 230M | Mica, Titanium Dioxide | 2.0 | Effect pigment |

(fortgesetzt)

| PHASE | BESTANDTEILE | INCI | % BY WEIGHT | |
|---|---|---|---|---|
| F | Perfume Just Delicious RS32785 (Technicoflor) | Parfum | 0.2 | Fragrance |
| G | Citric Acid (25% solution) | Citric Acid | 0.46 | pH Adjustment |
| Specifications | | | | |
| pH Wert (20°C) | | | 6.26 | |
| Viskosität (Brookfield; DV-/+; spindle 4; 10 rpm; 20°C) | | | 7 840 mPas | |

Soufflée Cream

| PHASE | BESTANDTEILE | INCI | | |
|---|---|---|---|---|
| A | Water, demin. | Aqua | 68.54 | |
| | Glycerin | Glycerin | 3.00 | Humectant |
| | Edeta® BD | Disodium EDTA | 0.10 | Complexing agent |
| | Plantapon® LGC SORB | Sodium Lauryl Glucose Carboxylate (and) Lauryl Glucoside | 0.75 | Surfactant |
| | Tinocare® GL | Sclerotium Gum | 1.50 | Rheology modifier |
| | Rheocare® XG (Europe only) | Xanthan Gum | 0.30 | Rheology modifier |
| B | Eumulgin® SG | Sodium Stearoyl Glutamate | 1.00 | Emulsifier (O/W) |
| | Cutina® PES | Pentaerythrityl Distearate | 1.00 | Consistency agent |
| | Lanette® O | Cetearyl Alcohol | 1.00 | Consistency agent |
| | Vitamin E-Acetate Care | Tocopheryl Acetate | 0.50 | Antioxidant |
| | Cetiol® CC | Dicaprylyl Carbonate | 3.00 | Emollient |
| | Cetiol® 4 All | Dipropylheptyl Carbonate | 3.00 | Emollient |
| | Myritol® 312 | Caprylic/Capric Triglyceride | 3.00 | Emollient |
| C | Cetiol® Ultimate* | Undecane, Tridecane | 4.00 | Emollient |
| D | Microcare PM 2 (THOR) | Phenoxyethanol, Methylparaben, Ethylparaben | 1.00 | Preservative |
| | Perfume** | Parfum | q.s. | Fragrance |
| | Tinovis® GTC UP | Acrylates/Beheneth-25 Methacrylate Copolymer | 2.25 | Rheology modifier |
| E | Biophytex™ LS 9832 | Aqua, Butylene Glycol, Panthenol, Escin, Glycerin, Ruscus Aculeatus Root Extract, Ammonium Glycyrrhizate, Centella Asiatica Extract, Hydrolyzed Yeast Protein, Calendula Officinalis Flower Extract | 3.00 | Active ingredient |

(fortgesetzt)

| PHASE | BESTANDTEILE | INCI | | |
|---|---|---|---|---|
| | Puricolor® Yellow AYE23 (0.1% solution) | CI 19140 | 0.21 | Colorant |
| | Puricolor® Blue ABL9 FDA (0.1% solution) | CI 42090 | 0.35 | Colorant |
| | Flamenco® Silk Green 830M | Mica, Titanium Dioxide | 1.50 | Effect pigment |
| | Timica® Terra White MN4501 | Mica, Titanium Dioxide | 1.00 | Effect pigment |
| F | Sodium Hydroxide (20% solution) | Sodium Hydroxide | q.s. | pH Adjustment |
| | | | | |

| SPECIFICATIONS | | | MANUFACTURING PROCESS |
|---|---|---|---|
| pH Wert (23°C) | 6.5 | | Heat the components listed under A to 70 - 75°C and stir until uniform (anchor stirrer; 700 rpm). Heat components listed under B to 70 - 75°C. Add phase B to phase A while stirring (1100 rpm). Allow the emulsion to cool while stirring in such a way that it remains in continual motion. Once below 60°C, add Cetiol® Ultimate and homogenize by means of a suitable dispersion unit (e.g., Ultra Turrax; 1 minute, 7000 rpm). Once below 40°C, add phase D and stir until homogenous (700 rpm). At room temperature, while stirring, add phase E and adjust pH value to 6.8 with phase F. |
| Viskosität (Brookfield; DV-I+; spindle TD, helipath; 4 rpm; 23°C) | 105 000 mPas | | |

Anhang

1) Abil® EM 90
INCI: Cetyl Dimethicone Copolyol
Hersteller: Tego Cosmetics (Evonik)

2) Allianz® OPT
INCI: Acrylates/C12-22 Alkyl Methacrylate Copolymer
Hersteller: Rohm und Haas

3) Amphisol® K
INCI: Potassium Cetyl Phosphate
Hersteller: Hoffmann La Roche

4) Antaron® V 220
INCI: PVP/Eicosene Copolymer
Hersteller: GAF General Aniline Firm Corp. (IPS-Global)

5) Antaron® V 216
INCI: PVP/Hexadecene Copolymer

Hersteller: GAF General Aniline Firm Corp. (IPS-Global)

6) Arlacel® 83
INCI: Sorbitan Sesquioleate
Hersteller: Croda (ICI Surfacants)

7) Arlacel® P 135
INCI: PEG-30 Dipolyhydroxystearate
Hersteller: Croda (ICI Surfacants)

8) Bentone® 38
INCI: Quaternium-18 Hectorite
Hersteller: Elementis (Elementis Specialties)

9) Carbopol® 980
INCI: Carbomer
Hersteller: Goodrich

10) Carbopol® 2984
INCI: Carbomer
Hersteller: Lubrizol, Inc.

11) Carbopol® ETD 2001
INCI: Carbomer
Hersteller: Lubrizol, Inc.

12) Carbopol® Ultrez 10
INCI: Carbomer
Hersteller: Lubrizol, Inc.

13) Cegesoft® C 17
INCI: Myristyl Lactate
Hersteller: BASF Personal Care and Nutrition GmbH, Grünau

14) Cegesoft® PFO
INCI: Passiflora Incarnata (EU)
Hersteller: BASF Personal Care and Nutrition GmbH

15) Cegesoft® PS 6
INCI: Olus
Hersteller: BASF Personal Care and Nutrition GmbH

16) Ceraphyl® 45
INCI: Diethylhexyl Malate
Hersteller: International Specialty Products

17) Cetiol® 868
INCI: Ethylhexyl Stearate
Hersteller: BASF Personal Care and Nutrition GmbH

18) Cetiol® A
INCI: Hexyl Laurate
Hersteller: BASF Personal Care and Nutrition GmbH

19) Cetiol® B
INCI: Dibutyl Adipate
Hersteller: BASF Personal Care and Nutrition GmbH

20) Cetiol® CC
INCI: Dicaprylyl Carbonate
Hersteller: BASF Personal Care and Nutrition GmbH

21) Cetiol® J 600
INCI: Oleyl Erucate
Hersteller: BASF Personal Care and Nutrition GmbH

22) Cetiol® LC
INCI: Coco-Caprylate/Caprate
Hersteller: BASF Personal Care and Nutrition GmbH

23) Cetiol® OE
INCI: Dicaprylyl Ether
Hersteller: BASF Personal Care and Nutrition GmbH

24) Cetiol® PGL
INCI: Hexyldecanol, Hexyldecyl Laurate
Hersteller: BASF Personal Care and Nutrition GmbH

25) Cetiol® S
INCI: Diethylhexylcyclohexane
Hersteller: BASF Personal Care and Nutrition GmbH

26) Cetiol® SB 45
INCI: Shea Butter Butyrospermum Parkii (Linne)
Hersteller: BASF Personal Care and Nutrition GmbH

27) Cetiol® SN
INCI: Cetearyl Isononanoate
Hersteller: BASF Personal Care and Nutrition GmbH

28) Copherol® F 1300 C
INCI: Tocopherol
Hersteller: BASF Personal Care and Nutrition GmbH

29) Copherol 1250 C
INCI: Tocopheryl Acetate
Hersteller: BASF Personal Care and Nutrition GmbH

30) Cosmedia® DC
INCI: Hydrogenated Dimer Dilinoleyl / Dimethylcarbonate Copolymer
Hersteller: BASF Personal Care and Nutrition GmbH

31) Cosmedia® SP
INCI: Sodium Polyacrylate
Hersteller: BASF Personal Care and Nutrition GmbH

32) Cutina® E 24
INCI: PEG-20 Glyceryl Stearate
Hersteller: BASF Personal Care and Nutrition GmbH

33) Cutina® HR
INCI: Hydrogenated Castor Oil
Hersteller: BASF Personal Care and Nutrition GmbH

34) Cutina® MD
INCI: Glyceryl Stearate

Hersteller: BASF Personal Care and Nutrition GmbH

35) Cuitina® PES
INCI: Pentaerythrityl Distearate
Hersteller: BASF Personal Care and Nutrition GmbH

36) Dehymuls® FCE
INCI: Dicocoyl Pentaerythrityl Distearyl Citrate
Hersteller: BASF Personal Care and Nutrition GmbH

37) Dehymuls® HRE 7
INCI: PEG-7 Hydrogenated Castor Oil
Hersteller: BASF Personal Care and Nutrition GmbH

38) Dehymuls® PGPH
INCI: Polyglyceryl-2 Dipolyhydroxystearate
Hersteller: BASF Personal Care and Nutrition GmbH

39) Dow Corning® 244 Fluid
INCI: Cyclomethicone
Hersteller: Dow Corning

40) Dow Corning® 246 Fluid
INCI: Cyclopentasiloxane
Hersteller: Dow Corning

41) Dow Corning® 2502
INCI: Cetyl Dimethicone
Hersteller: Dow Corning

42) Dry®Flo Plus
INCI: Aluminium Starch Octenylsuccinate
Hersteller: National Starch

43) Elfacos®ST 37
INCI: PEG-22 Dodecyl Glycol Copolymer
Hersteller: Akzo-Nobel

44) Elfacos®ST 9
INCI: PEG-45 Dodecyl Glycol Copolymer
Hersteller: Akzo-Nobel

45) Emery® 1780
INCI: Lanolin Alcohol
Hersteller: BASF Personal Care and Nutrition Corporation (Emery)

46) Emulgade® CM
INCI: Cetearyl Isononanoate and Ceteareth-20 and Cetearyl Alcohol and Glyceryl Stearate and Glycerin and Ceteareth-12 and Cetyl Palmitate
Hersteller: BASF Personal Care and Nutrition GmbH

47) Emulgade®PL 68/50
INCI: Cetearyl Glucoside, Cetearyl Alcohol
Hersteller: BASF Personal Care and Nutrition GmbH

48) Emulgade® SE - PF
INCI: Glyceryl Stearate (and) Ceteareth-20 (and) Ceteareth-12 (and) Cetearyl Alcohol (and) Cetyl Palmitate
Hersteller: BASF Personal Care and Nutrition GmbH

49) Emulgade® SUCRO
INCI: Sucrose Polystearate (and) Hydrogenated Polyisobutene
Hersteller: BASF Personal Care and Nutrition GmbH

50) Eumulgin® B1
INCI: Ceteareth-12
Hersteller: BASF Personal Care and Nutrition Deutschland G mbH

51) Eumulgin® B 2
INCI: Ceteareth- 20
Hersteller: BASF Personal Care and Nutrition GmbH

52) Eumulgin® HRE 40
INCI: PEG-40 Hydrogenated Castor Oil
Hersteller: BASF Personal Care and Nutrition GmbH

53) Eumulgin® SG
INCI: Sodium Stearoyl Glutamate
Hersteller: BASF Personal Care and Nutrition GmbH

54) Eumulgin® VL 75
INCI: Lauryl Glucoside (and) Polyglyceryl-2 Dipolyhydroxystearate (and) Glycerin
Hersteller: BASF Personal Care and Nutrition GmbH

55) Eusolex® OCR
INCI: Octocrylene
Hersteller: Merck

56) Eusolex® T 2000
INCI: Titanium Dioxide, Alumina, Simethicone
Hersteller: Merck

57) Eusolex® T AQUA
INCI: Water and Titanium Dioxide and Alumina and Sodium Metaphosphate and Phenoxyethanol and Sodium Methylparaben Hersteller: Merck

58) Eutanol® G
INCI: Octyldodecanol
Hersteller: BASF Personal Care and Nutrition GmbH

59) Eutanol®G 16
INCI: Hexyldecanol
Hersteller: BASF Personal Care and Nutrition GmbH

60) Eutanol®G 16 S
INCI: Hexyldecyl Stearate
Hersteller: BASF Personal Care and Nutrition GmbH

61) Finsolv® TN
INCI: C 12/15 Alkyl Benzoate
Hersteller: Findex (Nordmann/Rassmann)

62) Generol® R
INCI: Brassica Campestris (Rapseed) Sterols
Hersteller: BASF Personal Care and Nutrition GmbH

63) Glucate® DO
INCI: Methyl Glucose Dioleate

Hersteller: NRC Nordmann/Rassmann

64) Hispagel® 200
INCI: Glycerin, Glyceryl Polyacrylate
Hersteller: BASF Personal Care and Nutrition GmbH

65) Hostaphat® KL 340 N
INCI: Trilaureth-4 Phosphate
Hersteller: Clariant

66) Hydagen® C.A.T.
INCI Triethyl Citrate
Hersteller: BASF Personal Care and Nutrition GmbH

67) Hydagen® DCMF
INCI : Chitosan
Hersteller: BASF Personal Care and Nutrition GmbH

68) Insect Repellent® 3535
INCI : Ethyl Butylacetylaminopropionate
Hersteller: EMD Chemicals Inc

69) Isolan® PDI
INCI: Diisostearoyl Polyglyceryl-3 Diisostearat
Hersteller: Evonik AG

70) Keltrol® T
INCI: Xanthan Gum
Hersteller: CP Kelco

71) Lameform® TGI
INCI: Polyglyceryl-3 Diisostearat
Hersteller: BASF Personal Care and Nutrition GmbH

72) Lanette® 14
INCI: Myristyl Alcohol
Hersteller: BASF Personal Care and Nutrition GmbH

73) Lanette 18
INCI: Stearyl Alcohol
Hersteller: BASF Personal Care and Nutrition GmbH

74) Lanette® 22
INCI: Behenyl Alcohol
Hersteller: BASF Personal Care and Nutrition GmbH

75) Lanette® E
INCI: Sodium Cetearyl Sulfate
Hersteller: BASF Personal Care and Nutrition GmbH

76) Lanette® O
INCI: Cetearyl Alcohol
Hersteller: BASF Personal Care and Nutrition GmbH

77) Locron® L
INCI: Aluminium Chlorhydrate
Hersteller: Clariant

78) Lucentite® SAN
INCI: Quaternium-18 Hectoritr
Hersteller: Co-Op Chemical Co., Ltd.

79) Monomuls® 90-O 18
INCI: Glyceryl Oleate
Hersteller: BASF Personal Care and Nutrition GmbH

80) Myrj® 51
INCI: PEG-30-Sterate
Hersteller: Croda

81) Myritol® 312
INCI: Caprylic/Capric Triglyceride
Hersteller: BASF Personal Care and Nutrition GmbH

82) Myritol® 331
INCI: Cocoglycerides
Hersteller: BASF Personal Care and Nutrition GmbH

83) Myritol® PC
INCI: Propylene Glycol Dicaprylate/Dicaprate
Hersteller: BASF Personal Care and Nutrition GmbH

84) Neo Heliopan® 303
INCI: Octocrylene
Hersteller: Symrise

85) Neo Heliopan® AP
INCI: Disodium Phenyl Dibenzimidazole Tetrasulfonate
Hersteller: Symrise

86) Neo Heliopa® AV
INCI: Ethylhexyl Methoxycinnamate
Hersteller: Symrise

87) Neo Heliopan® BB
INCI: Benzophenone-3
Hersteller: Symrise

88) Neo Heliopan® E 1000
INCI: Isoamyl-p-Methoxycinnamate Hersteller: Symrise

88) Neo Heliopan® Hydro
INCI: Phenylbenzimidazole Sulfonic Acid
Hersteller: Symrise

89) Neo Heliopan® MBC
INCI: 4-Methylbenzylidene Camphor
Hersteller: Symrise

90) Neo Heliopan® OS
INCI: Ethylhexyl Salicylate
Hersteller: Symrise

91) Novata® AB
INCI: Cocoglycerides
Hersteller: BASF Personal Care and Nutrition GmbH

92) Parsol® 1789
INCI: Butyl Methoxydibenzoylmethane
Hersteller: Hoffmann-La Roche (Givaudan)

93) Pemulen® TR-2 Polymer
INCI: Acrylates / C10-30 Alkylacrylate Crosspolymer
Hersteller: Lubrizol, Inc.

94) Photonyl® LS
INCI: Arginine, Disodium Adenosine Triphosphate, Mannitol, Pyridoxine HCL, Phenylalanine, Tyrosine
Hersteller: Laboratoires Serobiologiques (BASF Personal Care and Nutrition)

95) Prisorine® 3505
INCI: Isostearic Acid
Hersteller: Croda

96) Prisorine® 3758
INCI: Hydrogenated Polyisobutene
Hersteller: Croda

98) Rezal 36G
INCI: Aluminum Zirconium Tetrachlorohydrex GLY
Hersteller: Reheis, Inc

99)SFE® 839
INCI: Cyclopentasiloxane and Dimethicone/Vinyl Dimethicone Crosspolymer
Hersteller: Momentive

100) Silikonöl Wacker AK® 350
INCI: Dimethicone
Hersteller: Wacker

101) Tego® Care 450
INCI: Polyglyceryl-3 Methylglucose Distearate
Hersteller: Tego Cosmetics (Evonik)

102) Tego® Care CG 90
INCI: Cetearyl Glucoside
Hersteller: Evonik

103) Tegosoft® DEC
INCI: Diethylhexyl Carbonate
Hersteller: Evonik

104) Tinosorb® S
INCI: Bis-Ethylhexyloxyphenol Methoxyphenyl Triazine
Hersteller: BASF

105) Tinosorb® M
INCI: Methylene Bis-Benzotriazolyl Tetramethylbutylphenol
Herstelller: BASF

106) Tween® 60
INCI: Polysorbate 60
Hersteller: Croda (ICI Surfactants)

107) Uvasorb® HEB
INCI: Diethylhexyl Butamido Triazone

Hersteller: 3V Inc.

108) Unirep® U-18
INCI: Dimethyl Phthalate and Diethyl Toluamide and Ethyl Hexanediol
Hersteller: Induchem AG

109) Uvinul® T 150
INCI: Ethylhexyl Triazone
Hersteller: BASF

110) Uvinul® A plus
INCI: Diethylamino Hydroxybenzoyl Hexyl Benzoate
Hersteller: BASF

111) Veegum® Ultra
INCI: Magnesium Aluminium Silicate
Hersteller: R. T. Vanderbilt Company, Inc

112) Veegum® Plus
INCI: Magnesium Aluminum Silicate and Cellulose Gum
Hersteller: R. T. Vanderbilt Company, Inc

113) Z-Cote® HP 1
INCI: Zinc Oxide and Triethoxy-caprylylsilane
Hersteller: BASF

114) Zinc Oxide NDM
INCI: Zinc Oxide
Hersteler: Symrise

**Patentansprüche**

1. Verwendung von Dialkylcarbonaten der Formel (I)

$$R^1\text{-O-CO-OR}^2 \qquad (I)$$

worin $R^1$ ein 2-Propyl-1-heptyl-Rest oder ein 2-Ethyl-1-butyl-Rest ist und $R^2$ für lineare oder verzweigte oder cyclische Kohlenwaserstoffreste mit 1 bis 22 Kohlenstoffatomen steht, als Dispergiermittel für Feststoffe.

2. Verwendung nach Anspruch 1 in kosmetischen und/oder pharmazeutischen Zusammensetzungen

3. Verwendung nach Anspruch 1 und/oder Anspruch 2, **dadurch gekennzeichnet, dass** $R^1$ und $R^2$ identisch sind.

4. Verwendung nach Anspruch 3, **dadurch gekennzeichnet, dass** das Dialkylcarbonat Di-(2-Propyl-1-heptyl)carbonat oder Di-(2-Ethyl-1-butyl)carbonat ist.

5. Verwendung nach Anspruch 4, **dadurch gekennzeichnet, dass** $R^1$ und $R^2$ ein 2-Propyl-1-heptyl-Rest ist.

6. Verwendung nach einem der Ansprüche 1 bis 5, **dadurch gekennzeichnet, dass** die Feststoffe Pigmente sind.

7. Verwendung nach Anspruch 6, **dadurch gekennzeichnet, dass** die Pigmente anorganische oder organische Pigmente sind.

8. Verwendung nach Anspruch 7, **dadurch gekennzeichnet, dass** das Pigment ein pigmentärer Lichtfilter, vorzugsweise Titandioxid und/oder ein oder mehrere Eisenoxid oder Zinkoxid-Pigmente, ist.

**9.** Verwendung nach Anspruch 8, **dadurch gekennzeichnet, dass** das Pigment ein organischer pigmentärer Lichtfilter, vorzugsweise Methylen-bis-benzotriazolyltetramethylbutylphenol ist.

**10.** Verwendung nach einem der Ansprüche 1 bis 5, **dadurch gekennzeichnet, dass** die Feststoffe Aluminiumsalze, Puderrohstoffe, Füllstoffe und Feststoffe zur Modifikation der Sensorik sind.

**11.** Kosmetische und/oder pharmazeutische Zusammensetzung, **dadurch gekennzeichnet, dass** sie 0,1 bis 95 Gew.-% von mindestens einem Dialkylcarbonat der allgemeinen Formel (I)

$$R^1\text{-O-CO-OR}^2 \qquad (I)$$

worin $R^1$ ein 2-Propyl-1-heptyl-Rest oder ein 2-Ethyl-1-Butyl-Rest ist und $R^2$ für lineare oder verzweigte oder cyclische Kohlenwaserstoffreste mit 1 bis 22 Kohlenstoffatomen steht,
als Dispergiermittel enthält.

**12.** Zusammensetzung nach Anspruch 11, **dadurch gekennzeichnet, dass** $R^1$ und $R^2$ identisch sind.

**13.** Zusammensetzung nach Anspruch 12, **dadurch gekennzeichnet, dass** $R^1$ und $R^2$ ein 2-Propyl-1-heptyl-Rest ist.

**14.** Zusammensetzung nach einem der Ansprüche 11 bis 13, **dadurch gekennzeichnet, dass** mindestens ein organisches oder anorganisches Pigment und/oder ein oder mehrere Aluminiumsalz(e), Puderrohstoffe, Füllstoffe und Feststoffe zur Modifikation der Sensorik enthalten ist/sind.

**15.** Zusammensetzung nach Anspruch 14, **dadurch gekennzeichnet, dass** das mindestens eine Pigment ein pigmentärer anorganischer Lichtfilter, vorzugsweiseTitandioxid und/oder Zinkoxid oder ein organischer pigmentärer Lichtfilter, vorzugsweise Methylen-bis-benzotriazolyltetramethylbutylphenol ist.

**16.** Zusammensetzung nach Anspruch 14, **dadurch gekennzeichnet, dass** mindestens ein Pigment ein farbgebendes Pigment, vorzugsweise ein anorganisches Metalloxid und besonders bevorzugt Eisenoxid ist.

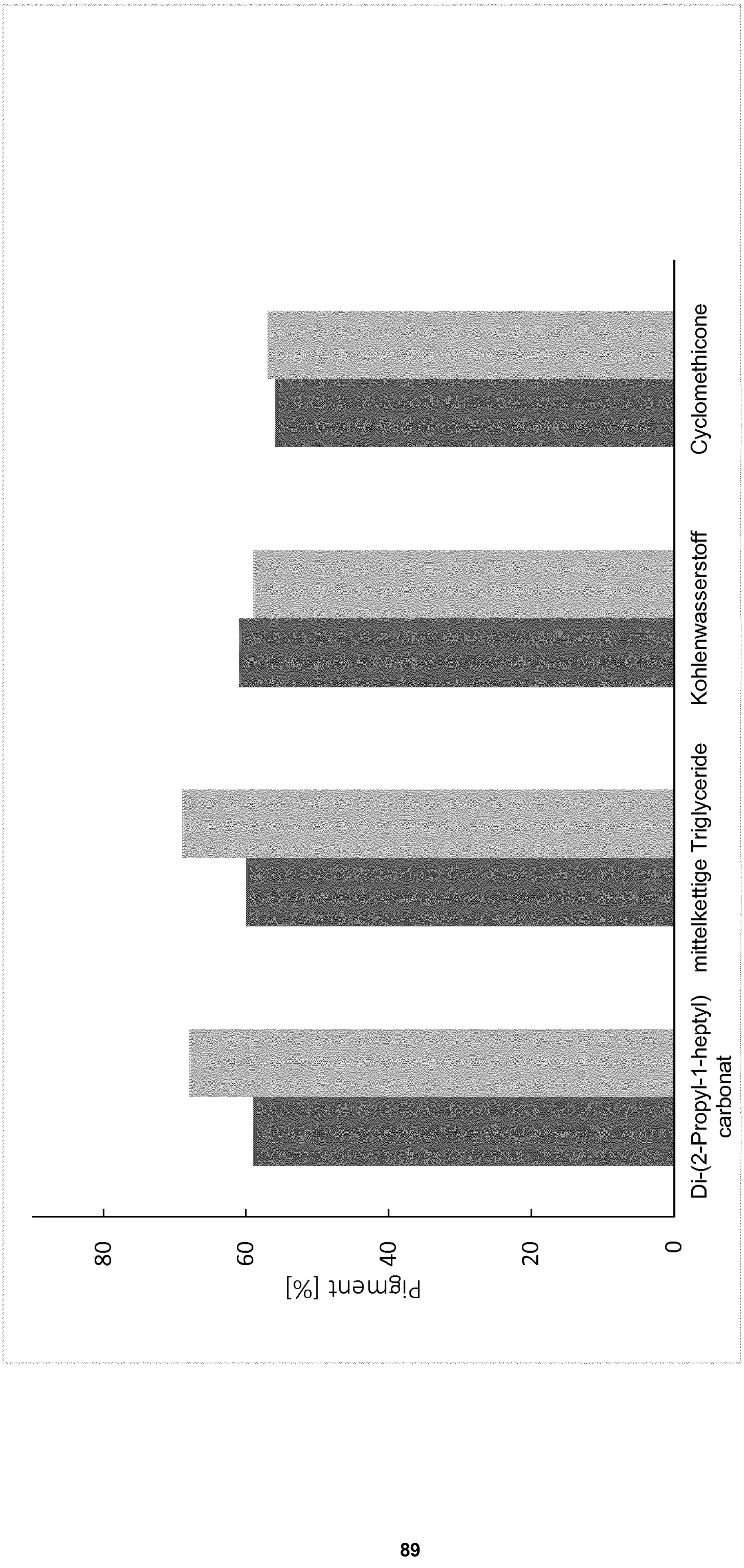
Pigment [%]
0
20
40
60
80
Di-(2-Propyl-1-heptyl) carbonat
mittelkettige Triglyceride
Kohlenwasserstoff
Cyclomethicone

Abb. 1

Europäisches Patentamt
European Patent Office
Office européen des brevets

# EUROPÄISCHER RECHERCHENBERICHT

Nummer der Anmeldung
EP 16 19 1899

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 930 311 A1 (COGNIS IP MAN GMBH [DE]) 11. Juni 2008 (2008-06-11)<br>* Absätze [0006] - [0011], [0019]; Ansprüche *<br>----- | 1-14 | INV.<br>A61K8/37<br>C07C69/96 |
| A | WO 2010/046061 A2 (COGNIS IP MAN GMBH [DE]; KAWA ROLF [DE]; JACKWERTH BETTINA [DE]) 29. April 2010 (2010-04-29)<br>* Seite 2 - Seiten 7,11; Ansprüche *<br>----- | 1-14 | |
| A | WO 97/47282 A1 (HENKEL KGAA [DE]; LOEHL THORSTEN [DE]; GASSENMEIER THOMAS [DE]; KAHRE) 18. Dezember 1997 (1997-12-18)<br>* Ansprüche; Tabellen 1-3 *<br>----- | 1-14 | |
| A | WO 2005/063189 A1 (BEIERSDORF AG [DE]; ENGFELDT LINDA [DE]; WARNKE KATJA [DE]; MIERTSCH H) 14. Juli 2005 (2005-07-14)<br>* Seite 8 - Seite 10; Ansprüche 1,3; Beispiele *<br>----- | 1-14 | RECHERCHIERTE SACHGEBIETE (IPC) |
| A | EP 1 277 460 A2 (BEIERSDORF AG [DE]) 22. Januar 2003 (2003-01-22)<br>* Beispiele 3.3,5.3 *<br>----- | 1-14 | A61K<br>C07C |

Der vorliegende Recherchenbericht wurde für alle Patentansprüche erstellt

2

| Recherchenort | Abschlußdatum der Recherche | Prüfer |
|---|---|---|
| München | 15. Februar 2017 | Uhl, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 16 19 1899

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-02-2017

| Im Recherchenbericht angeführtes Patentdokument | Datum der Veröffentlichung | Mitglied(er) der Patentfamilie | Datum der Veröffentlichung |
|---|---|---|---|
| EP 1930311 A1 | 11-06-2008 | CN 101547889 A | 30-09-2009 |
| | | CN 104592031 A | 06-05-2015 |
| | | EP 1930311 A1 | 11-06-2008 |
| | | EP 2094641 A1 | 02-09-2009 |
| | | ES 2503723 T3 | 07-10-2014 |
| | | JP 5399260 B2 | 29-01-2014 |
| | | JP 2010511645 A | 15-04-2010 |
| | | KR 20090096443 A | 10-09-2009 |
| | | US 2010331565 A1 | 30-12-2010 |
| | | WO 2008067946 A1 | 12-06-2008 |
| WO 2010046061 A2 | 29-04-2010 | CN 102292066 A | 21-12-2011 |
| | | DE 102008052520 A1 | 22-04-2010 |
| | | EP 2391335 A2 | 07-12-2011 |
| | | JP 2012506393 A | 15-03-2012 |
| | | KR 20110073532 A | 29-06-2011 |
| | | US 2011243863 A1 | 06-10-2011 |
| | | WO 2010046061 A2 | 29-04-2010 |
| WO 9747282 A1 | 18-12-1997 | AU 3171997 A | 07-01-1998 |
| | | DE 29724204 U1 | 21-09-2000 |
| | | EP 0935454 A1 | 18-08-1999 |
| | | ES 2162304 T3 | 16-12-2001 |
| | | ID 17407 A | 24-12-1997 |
| | | JP 2000512285 A | 19-09-2000 |
| | | US 6482418 B1 | 19-11-2002 |
| | | WO 9747282 A1 | 18-12-1997 |
| WO 2005063189 A1 | 14-07-2005 | DE 10361526 A1 | 28-07-2005 |
| | | EP 1701694 A1 | 20-09-2006 |
| | | WO 2005063189 A1 | 14-07-2005 |
| EP 1277460 A2 | 22-01-2003 | DE 10135024 A1 | 24-04-2003 |
| | | EP 1277460 A2 | 22-01-2003 |
| | | EP 1829583 A2 | 05-09-2007 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9747282 A
- EP 1510199 A
- WO 9747583 A
- EP 1083247 A
- WO 2008067946 A
- EP 0693471 B1
- EP 0818450 A1
- EP 0694521 B1
- DE 19712033 A1
- EP 1371359 A2
- EP 766661 B1
- WO 07048757 A


### In der Beschreibung aufgeführte Nicht-Patentliteratur


- **M. DIERKER.** *Lipid Technology,* 2004, vol. 16 (6), 130-134
- **VON P.FINKEL.** *SÖFW-Journal,* August 1996, vol. 122, 543-548
- *Parf.Kosm.,* Marz 1999, vol. 80, 10-16
- **H. P. FIEDLER.** Lexikon der Hilfsstoffe für Pharmazie, Kosmetik und angrenzende Gebiete. 1996
- **KIRK-OTHMER.** Encyclopedia of Chemical Technology. 1979, vol. 8, 913